# EUROPÄISCHE PATENTSCHRIFT

(11) **EP 1 833 781 B1**
(45) Veröffentlichungstag und Bekanntmachung des Hinweises auf die Patenterteilung: **18.05.2011**
(21) Anmeldenummer: 05823028.5
(22) Anmeldetag: 13.12.2005
(51) Int. Cl.: C07C 209/52, C07C 209/62, C07C 231/18, C07C 211/35, C07C 211/40, C07C 211/42, C07C 233/65

(54) **VERFAHREN ZUR HERSTELLUNG VON OPTISCH AKTIVEN CYCLISCHEN AMINEN**
METHOD FOR THE PRODUCTION OF OPTICALLY ACTIVE CYCLIC AMINES
PROCEDE DE PRODUCTION D'AMINES CYCLIQUES OPTIQUEMENT ACTIVES

(30) Priorität: 30.12.2004 DE 102004063443
(43) Veröffentlichungstag der Anmeldung: 19.09.2007
(73) Patentinhaber: Bayer CropScience AG, 40789 Monheim (DE)
(72) Erfinder: DIETRICH, Hansjörg, 65719 Hofheim (DE); FORD, Mark, James, 61389 Schmitten-Oberreifenberg (DE); MÜLLER, Thomas, 60322 Frankfurt (DE); LASSALETTA SIMON, José Maria, E-41009 Sevilla (ES); ROS LAO, Abel, E-41009 Sevilla (ES); MAGRIZ TASCON, Antonio, E-41740 Lebrija (ES)
(86) Internationale Anmeldenummer: PCT/EP2005/013371
(87) Internationale Veröffentlichungsnummer: WO 2006/072374

(56) Entgegenhaltungen:
- EP-A- 0 916 637
- WO-A-2004/069814
- NACHTSHEIM C M ET AL: "ASYMMETRISCHE REDUKTIVE AMINIERUNG VON CYCLOALKANONEN, 7. MITT. DIE ASYMMETRISCHE SYNTHESE VON CIS-1R,2R- UND CIS-1S,2S-2-ARYLCYCLOHEXANAMIN ASYMMETRIC REDUCTIVE AMINATION OF CYCLOALKANONES, VII: ASYMMETRIC SYNTHESIS OF CIS-1R,2R- AND CIS-1S,2S-2-ARYLCYCLOHEXANAMINES" ARCHIV DER PHARMAZIE, VCH VERLAGSGESELLSCHAFT MBH, WEINHEIM, DE, Bd. 322, Nr. 4, 1. April 1989 (1989-04-01), Seiten 187-197, XP000614042 ISSN: 0365-6233
- MASSEY, EDDIE H. ET AL: "Optically active amines. VI. Terpenes. 2. The stereochemistry and absolute configurations of the thujylamines and some related compounds" JOURNAL OF ORGANIC CHEMISTRY, 31(3), 684 -90 CODEN: JOCEAH; ISSN: 0022-3263, 1966, XP002366802
- WIEHL, WOLFGANG ET AL: "Asymmetric reductive amination of cycloalkanones. 5. Synthesis and absolute configuration of 2-substituted cyclopentanamines" CHEMISCHE BERICHTE, 119(8), 2668 -77 CODEN: CHBEAM; ISSN: 0009-2940, 1986, XP002366803
- EGUCHI T ET AL: "Rational De Novo Design of NADH Mimic for Stereoselective Reduction Based on Molecular Orbital Calculation" TETRAHEDRON, ELSEVIER SCIENCE PUBLISHERS, AMSTERDAM, NL, Bd. 54, Nr. 5-6, 29. Januar 1998 (1998-01-29), Seiten 705-714, XP004106597 ISSN: 0040-4020
- NOYORI R ET AL: "ASYMMETRIC CATALYSIS BY ARCHITECTURAL AND FUNCTIONAL MOLECULAR ENGINEERING: PRACTICAL CHEMO- AND STEREOSELECTIVE HYDROGENATION OF KETONES" ANGEWANDTE CHEMIE. INTERNATIONAL EDITION, WILEY VCH VERLAG, WEINHEIM, DE, Bd. 40, Nr. 1, Januar 2001 (2001-01), Seiten 41-73, XP000998801 ISSN: 1433-7851

## Beschreibung

Die Erfindung betrifft das technische Gebiet der Verfahren zur Herstellung von optisch aktiven Zwischenprodukten, die für Synthesen von Wirkstoffen, beispielsweise Wirkstoffe für Pflanzenschutzmittel oder Arzneimittel, eingesetzt werden können.

Optisch aktive Verbindungen werden häufig als Zwischenprodukte für die Herstellung optisch aktiver Wirkstoffe benötigt. Dies trifft bei enantioselektiven Herstellungsverfahren zu, bei denen die gewünschte räumliche Struktur an bestimmten Chiralitätszentren auf der Stufe eines Zwischenprodukts eingeführt und über ein oder mehrere Verfahrensschritte bis zum Wirkstoff erhalten bleibt. Geeignete Zwischenprodukte sind optisch aktive Naturstoffe oder Verbindungen, bei denen ein Chiralitätszentrum mit Hilfe enantioselektiver Reaktionen aus achiralen Verbindungen erzeugt wird, oder racemische Verbindungen, bei welchen selektiv nur eines der enthaltenen Enantiomere enantioselektiv umgesetzt und weiter verwendet wird.

Aminoverbindungen haben eine große Bedeutung als Zwischenprodukte für Pflanzenschutzmittelwirkstoffe und Arzneimittelwirkstoffe. Zahlreiche Verfahren zur Herstellung von optisch aktiven Aminen sind bekannt, siehe beispielsweise Angew. Chem. Int. Ed. 2004 (116) 806-843 und dort zitierte Literatur. Neben der klassischen Racematspaltung über diastereomere Salze mit optisch aktiven Säuren betreffen einige die Verwendung optisch aktiver Ausgangsmaterialien und enantioselektive Umsetzungen bis zur gewünschten Verbindung. Beispielsweise beschreibt C. M. Nachtsheim et al. [Archiv der Pharmazie, Bd. 322 (4), S. 187-197 (1989)] die Herstellung optisch aktiver cyclischer 2-Aryl- oder 2-Alkyl-cydoalkanamine durch Umsetzung entsprechender razemischer Cyclohexanone mit R(+)- bzw. S(-)-1-Phenylethyl-amin zum Imin, Hydrierung der Iminogruppe zum Amin mit Raney-Ni und Entfernung des N-Substituenten durch Hydrogenolyse. Andere Verfahren basieren auf enzymatischen Reaktionen, beispielsweise Transaminierungen unter Verwendung von Enzymen oder Mikroorganismen. Wieder andere Verfahren nutzen den Einsatz von synthetischen optisch aktiven Reagenzien und deren Eigenschaften bei Reaktionen, enantioselektiv Chiralitätszentren zu erzeugen.

Die bekannten Verfahren haben meist den Nachteil, dass sie nur sehr spezifisch auf bestimmte Substrate angewandt werden können. Mit strukturell anderen Substraten sind dann die Resultate hinsichtlich Parametern wie chemischer Ausbeute, enantiomerem Überschuss, Selektivität, Reinheit, Reaktionsdauer und Zugänglichkeit von Ausgangsmaterialien und Hilfsstoffen oft unbefriedigend. Es besteht deshalb im Einzelfall und auch generell Bedarf für die Bereitstellung alternativer Verfahren zur Herstellung von optisch aktiven Aminen.

Es wurde nun gefunden, dass bestimmte optisch aktive cyclische Amine mit einem zweiten, benachbarten Chiralitätszentrum in besonders effektiver Weise enantioselektiv aus Verbindungen hergestellt werden können, die bezüglich des benachbarten Chiralitätszentrums racemisch vorliegen.

Gegenstand der Erfindung ist ein Verfahren zur Herstellung von optisch aktiven cyclischen Aminen der Formel (I) und deren Salzen, 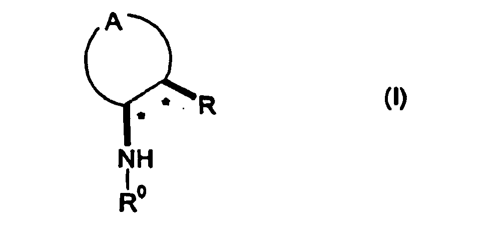 worin
- A: gemeinsam mit den jeweils mit einem Stern (*) bezeichneten C-Atomen einen carbocyclischen oder heterocyclischen gesättigten oder ungesättigten, nicht- aromatischen Ring mit 3 bis 30 Ringatomen, vorzugsweise mit 4 bis 9 Ringatomen, insbesondere mit 5 bis 7 Ringatomen, der zusätzlich zu den Resten R und NH-R⁰ weiter substituiert sein kann, bedeutet,
- R⁰: unabhängig von R einen Rest (C₁-C₆)Alkyl, (C₂-C₆)Alkenyl oder (C₃-C₆)Alkinyl, wobei jeder der drei letztgenannten Reste unsubstituiert oder durch einen oder mehrere Reste aus der Gruppe Halogen, Cyano, (C₁-C₆)Alkoxy, (C₁-C₆)Haloalkoxy, (C₁-C₆)Alkylthio, (C₁-C₆)Alkanoyloxy, (C₁-C₆)Haloalkanoyloxy, Aryl, Aryloxy, Aroyl, Aroyloxy und Heterocyclyl, wobei jeder der letztgenannten 5 Reste unsubstituiert oder substituiert ist, substituiert ist,
oder (C₃-C₉)Cycloalkyl, (C₄-C₉)Cycloalkenyl, Aryl oder Heterocyclyl, wobei jeder der letztgenannten 4 Reste unsubstituiert oder substituiert ist, bedeutet,
- R: unabhängig von R⁰
(C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, Cyano, (C₁-C₆)Alkoxy, (C₁- C₆)Haloalkoxy, (C₁-C₆)Alkylthio, Aryl, das unsubstituiert oder substituiert ist, und Heteroaryl, das unsubstituiert oder substituiert ist, substituiert ist, oder
Aryl, das unsubstituiert oder substituiert ist, oder
Heteroaryl, das unsubstituiert oder substituiert ist,
bedeutet oder
- R⁰ und A: einen Ring B1 bilden und dabei gemeinsam mit der NH-Gruppe und dem an der NH-Gruppe gebundenen, mit einem Stern (*) bezeichneten C-Atom einen heterocyclischen Ring mit 4 bis 30 Ringatomen, vorzugsweise mit 4 bis 9 Ringatomen, insbesondere mit 5 bis 7 Ringatomen, der gegebenenfalls zusätzlich weiter substituiert ist und der gegebenenfalls 1 oder 2 weitere Heteroatome aus der Gruppe N, O und S enthält, bedeuten oder
- R und A: einen Ring B2 bilden und dabei gemeinsam mit dem an R gebundenen, mit einem Stern (*) bezeichneten C-Atom einen carbocyclischen oder heterocyclischen Ring mit 3 bis 30 Ringatomen, vorzugsweise mit 4 bis 9 Ringatomen, insbesondere mit 5 bis 7 Ringatomen, der gegebenenfalls zusätzlich weiter substituiert ist und der im Falle eines heterocyclischen Rings 1 oder 2 oder 3 Heteroatome aus der Gruppe N, O und S enthält, bedeuten oder
- R⁰ und R: einen Ring B3 bilden und dabei gemeinsam mit der NH-Gruppe und den jeweils mit einem Stern (*) bezeichneten C-Atomen einen heterocyclischen Ring mit 4 bis 30 Ringatomen, vorzugsweise mit 4 bis 9 Ringatomen, insbesondere mit 5 bis 7 Ringatomen, der gegebenenfalls zusätzlich weiter substituiert ist und der gegebenenfalls 1 oder 2 weitere Heteroatome aus der Gruppe N, O und S enthält, bedeuten oder
- R⁰ und R: und gegebenenfalls A zwei oder mehrere der genannten Ringe B1, B2 und B3 zugleich bilden,
wobei R und die Gruppe NH-R⁰ an den beiden jeweils mit einem Stern (*) markierten Ring-C-Atomen in cis-Stellung zueinander ausgerichtet sind und die stereochemische Konfiguration an diesen C-Atomen von der racemischen Konfiguration verschieden ist,
dadurch gekennzeichnet, dass man ein Imin der Formel (II) oder ein Salz davon, 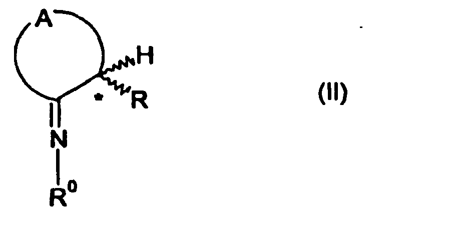 wobei A, R⁰ und R wie in Formel (I) definiert sind und die Verbindung der Formel (II) bezüglich des mit einem Stern (*) markierten Ring-C-Atoms als racemisches Gemisch oder als ein Gemisch mit einem beliebigen anderen Isomerenverhältnis der betreffenden Stereoisomere vorliegt,
in Gegenwart von Wasserstoff oder einem Wasserstoff-Donor und einem nicht enzymatischen Katalysator, der einen katalytisch wirksamen optisch aktiven Komplex eines oder mehrerer Übergangsmetalle aus der Gruppe Ruthenium, Rhodium, Palladium, Iridium, Osmium Platin, Eisen, Nickel und Samarium, vorzugsweise eines oder mehrerer Übergangsmetalle aus der Gruppe Ruthenium, Rhodium, Palladium und Iridium, insbesondere Ruthenium und Iridium, mit organischen Liganden enthält, zur Verbindung der Formel (I) oder dessen Salz umsetzt.

Die absolute Konfiguration des Produkts hängt im Einzelfall von der Struktur des optisch aktiven Katalysators aus der genannten Gruppe von Katalysatoren ab.

Verbindungen der Formel (I) sind insbesondere die Verbindungen der Formeln (I-A) und (I-B), 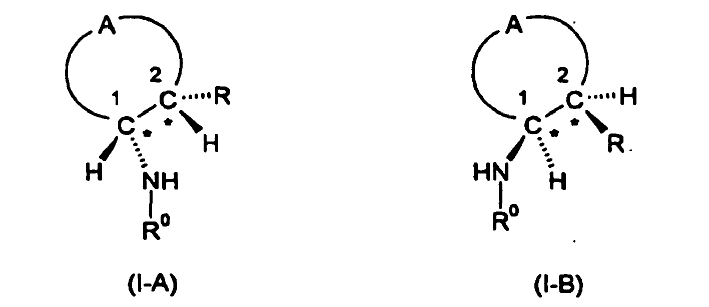 worin A, R⁰ und R die genannten Bedeutungen haben. Nach der stereochemischen Nomenklatur von Cahn-Ingold-Prelog hat die Verbindung (I-A) je nach Rangfolge der Substituenten am C-Atom 1 die stereochemische Bezeichnung (1S-cis) oder (1R-cis). Die Verbindung (I-B) hat entsprechend die stereochemische Bezeichnung (1 R-cis) bzw. (1 S-cis).

Vorzugweise werden die Verbindungen (I) mit cis-Konfiguration an den beiden jeweils mit Stern (*) bezeichneten Chiralitätszentren im Vergleich zur trans-Konfiguration mit einer Selektivität von 60 bis 100%, vorzugsweise 80 bis 100%, insbesondere 90 bis 100%, ganz besonders 95 bis 100% erhalten, wobei die jeweilige cis-Verbindung (I-A) bzw. (I-B) mit einer Enantioselektivität von jeweils mehr als 20 % ee oder besser mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffenden cis-Verbindungen (I-A) und (I-B) erhalten wird.

Der Enantiomerenüberschuss gemessen in % ee bedeutet dabei die Differenz der prozentualen Mengen beider Enantiomere bezogen auf das Gemisch der Enantiomere.

Die jeweilige Verbindung der Formel (II) besteht in der Regel aus einem Gemisch der Verbindungen (II-A1), (II-A2), (II-B1) und (II-B2), 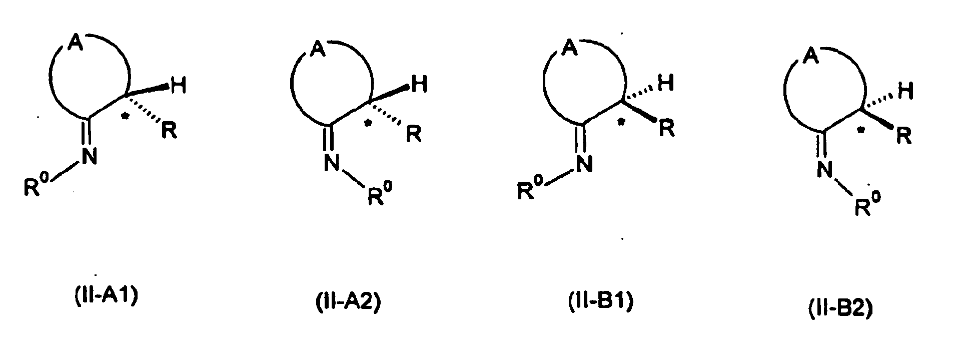 wobei die Verbindungen (II-A1) und (II-A2) zusammen kurz als Verbindungen (II-A) bzw. die Verbindungen (II-B1) und (II-B2) zusammen kurz als Verbindungen (II-B) bezeichnet werden. Die Verbindungen (II-A) und (II-B) können bezüglich der Konfiguration am C-Atom 2 im Allgemeinen in jedem Verhältnis eingesetzt werden. In der Regel liegen sie diesbezüglich vorzugsweise im Verhältnis 1:1, d. h. in einem racemischen Gemisch vor.

Die Verbindungen (II-A1) und (II-A2) bzw. (II-B1) und (II-B2) stehen unter den Reaktionsbedingungen meist miteinander im Gleichgewicht.

Wenn die Verbindungen (I) oder (II) durch Wasserstoffverschiebung Tautomere bilden können, welche strukturell formal nicht durch die Formel (I) bzw. (II) erfasst würden, so sind diese Tautomere gleichwohl von der Definition der erfindungsgemäßen Verbindungen der Formel (I) oder (II) umfasst.

Die Verbindungen der allgemeinen Formel (I) (bzw. entsprechend andere erfindungsgemäße Verbindungen) können je nach Art und Verknüpfung der Substituenten weitere Chiralitätszentren als die in Formel (I) mit einem Stern (*) markierten C-Atome enthalten und entsprechend als Stereoisomere vorliegen. Die durch ihre spezifische Raumform definierten möglichen Stereoisomere, wie Enantiomere, Diastereomere, Z- und E-Isomere sind alle von der Formel (I) umfaßt. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfaßt, jedoch nicht mit ihrer spezifischen Stereoform angegeben sind und deren Gemische.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Die Verbindungen der Formel (I) können Salze bilden, beispielsweise durch Anlagerung einer geeigneten anorganischen oder organischen Säure, wie beispielsweise Mineralsäuren, wie beispielsweise HCl, HBr, H₂SO₄ oder HNO₃, oder organische Säuren, wie Ameisensäure, Essigsäure, Propionsäure, Oxalsäure oder Sulfonsäuren, an eine basische Gruppe, wie z.B. die enthaltene Aminogruppe oder anderer Aminogruppen, Alkylamino, Dialkylamino, Piperidino-, Morpholino- oder Pyridino. Diese Salze enthalten dann die konjugierte Base der Säure als Anion.

Salzbildung kann durch Einwirkung einer Base auf solche Verbindungen der Formel (I) erfolgen, die ein acides Wasserstoffatom tragen. Geeignete Basen sind beispielsweise organische Amine sowie Ammonium-, Alkali- oder Erdalkalimetallhydroxide, -carbonate und -hydrogencarbonate, insbesondere Natrium- und Kaliumhydroxid, -carbonat und -hydrogencarbonat. Diese Salze sind Verbindungen, in denen der acide Wasserstoff durch ein Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

Die vorstehend und weiter unter verwendeten Bezeichnungen sind dem Fachmann geläufig und haben insbesondere die im Folgenden erläuterten Bedeutungen:
Ein anorganischer Rest ist ein Rest ohne Kohlenstoffatome, vorzugsweise Halogen, OH und dessen anorganische Salze bei denen das H durch ein Kation, beispielsweise Alkalimetall- und Erdalkalimetallsalze ersetzt wird, NH₂ und dessen Ammoniumsalze mit (anorganischen) Säuren, beispielsweise Mineralsäuren, N₃ (Azid), N₂⁺A⁻ (Diazonium-Rest, wobei A⁻ ein Anion darstellt), NO, NHOH, NHNH₂, NO₂, S(O)OH (Sulfinsäurerest), S(O)₂OH (oder auch kurz SO₃H, Sulfonsäurerest), -O-SO₂H (Sulfit), -O-SO₃H (Sulfat), -P(O)(OH)₂ (Phosphonsäurerest), -O-P(OH)₃, (Phosphatrest) und die hydratisierten oder dehydratisierten Formen der letztgenannten 6 Säurereste sowie deren (anorganischen) Salze; der Begriff "anorganischer Rest" umfasst auch den Wasserstoffrest (das Wasserstoffatom), wobei dieser in den Definitionen oft bereits Bestandteil des unsubstituierten Grundkörpers eines organischen Restes ist (Beispiel "unsubstituiertes Phenyl");
der Begriff "anorganischer Rest" umfasst hier vorzugsweise nicht Pseudohalogen-Gruppen wie CN, SCN, organische Metallkomplexe, Carbonat oder COOH, die wegen des Gehalts an C-Atomen besser den organischen Resten zugeordnet werden.

Ein organischer Rest ist im Gegensatz zu einem anorganischen Rest ein Rest mit Kohlenstoffatomen, wobei dieser Rest auch über Heteroatome gebunden sein kann. Vorzugsweise ist er ein gegebenenfalls substituierter Kohlenwasserstoffrest oder ein gegebenenfalls substituierter heterocyclischer Rest. Er umfasst aber auch vorzugsweise Acylreste, d. h. Reste organischer Säuren, welche durch Entfernen einer OH Gruppe entstehen. Acylreste umfassen auch Sulfonsäureester-, Phosphonsäureester-, Phosphinsäureestergruppen, jeweils mit organischen Alkoholkomponenten (und leiten sich dann von mehrbasigen Säuren ab), oder Alkylsulfonyl oder Alkylsulfinyl, welche von Sulfonsäuren oder Sulfinsäuren abgeleitet sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für die Kohlenwasserstoffoxyreste. Wenn nicht näher definiert weisen die Kohlenwasserstoff- und Kohlenwasserstoffoxyreste in den obigen Definitionen vorzugsweise 1 bis 20 C-Atome, weiter bevorzugt 1 bis 16 C-Atome, insbesondere 1 bis 12 C-Atome auf.

Die Kohlenwasserstoffreste und die spezielleren Reste Alkyl, Alkoxy, Haloalkyl, Haloalkoxy, Alkylamino und Alkylthio sowie die entsprechenden ungesättigten und/oder substituierten Reste können im Kohlenstoffgerüst jeweils geradkettig oder verzweigt sein.

Der Ausdruck "(C₁-C₄)Alkyl" bedeutet eine Kurzschreibweise für offenkettiges Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste. z.B. mit 1 bis 6 C-Atomen, insbesondere 1 bis 4 C-Atomen, bzw. bei ungesättigten Gruppen mit 2 bis 6 C-Atomen, insbesondere 2 - 4 C-Atomen bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. (C₂-C₆)-Alkynyl bedeutet beispielsweise Ethinyl, Propargyl, 1-Methyl-2-propinyl, 2-Methyl-2-propinyl, 2-Butinyl, 2-Pentinyl oder 2-Hexinyl, vorzugsweise Propargyl, But-2-in-1-yl, But-3-in-1-yl oder 1-Methyl-but-3-in-1-yl.

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl. Im Falle von substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Adamantan-1-yl und Adamantan-2-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend. Insbesondere umfasst substituiertes Cycloalkenyl auch entsprechende kondensierte polycyclische Verbindungen, beispielsweise benzokondensierte Verbindungen wie Tetrahydronaphthalin-1-yl oder -2-yl, Fluorenyl (Biphenylmethyl) oder Suberyl (Di-[b,f]-benzocyclohepta-2,6-dien-1-yl).

Die Bezeichnung "Halogen" bedeutet beispielsweise Fluor, Chlor, Brom oder lod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CH₂ClCH₃, CH₂FCH₃, CH₂Cl, CH₂F; Perhaloalkyl wie CCl₃ oder CF₃ oder CF₃CF₂; Polyhaloalkyl wie CHF₂, CH₂F, CH₂FCHCl, CHCl₂, CF₂CF₂H, CH₂CF₃, CH₂ClCH₃, CH₂FCH₃, Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System mit vorzugsweise 6 bis 14, insbesondere 6 bis 12 C-Atomen, beispielsweise Phenyl, Naphthyl, Tetrahydronaphthyl, Indenyl, Indanyl, Pentalenyl, Fluorenyl, Biphenylyl und ähnliches, vorzugsweise Phenyl.

Ein heterocyclischer Rest oder Ring (Heterocyclyl) enthält mindestens einen heterocyclischen Ring, der gesättigt, ungesättigt oder heteroaromatisch ist und der im generell substituierten Fall mit anderen carbocyclischen oder heterocyclischen Ringen annelliert sein kann; wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen und noch mindestens ein Kohlenstoffatom im Ring vorhanden sein muß z.B. ein Rest von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Tetrazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin, 4H-Chinolizin, Piperidin, Morpholin, Piperazin, Oxetan, Oxiran, Pyrrolidin, Oxazolin, Tetrahydrofuran, Tetrahydropyran, 1,3-Dioxolan, 1,3- und 1,4-Dioxan, Isoxazolidin oder Thiazolidin.

"Heteroaryl" bedeutet von den vorstehend unter "Heterocyclyl" genannten Gruppen jeweils die vollständig ungesättigten aromatischen heterocyclischen Verbindungen, z. B. Pyridin, Pyrimidin, (1,2,4)-Oxadiazol, (1,3,4)-Oxadiazol, Pyrrol, Furan, Thiophen, Oxazol, Thiazol, Imidazol, Pyrazol, Isoxazol, 1,2,4-Triazol, Tetrazol, Pyrazin oder Pyridazin.

Weiterhin bevorzugt ist Heterocyclyl ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidinyl oder Piperidinyl.
Weiterhin bevorzugt ist er ein_partiell oder vollständig hydrierter heterocyclischer Rest mit 2 Heteroatomen aus der Gruppe N, O und S, beispielsweise Oxazolinyl, Thiazolinyl, Piperazinyl, 1,3-Dioxolanyl, 1,3- und 1,4-Dioxanyl, Oxazolinyl, Isoxazolinyl, Oxazolidinyl, Isoxazolidinyl und Morpholinyl.
Handelt es sich es sich um einen teilweise oder vollständig gesättigten Stickstoff-Hetererocyclus, so kann dieser sowohl über Kohlenstoff als auch über den Stickstoff mit dem Rest des Moleküls der jeweiligen Verbindung verknüpft sein.

Vorzugsweise ist Heterocyclyl ein aliphatischer, gesättigter oder ungesättigter, insbesondere gesättigter, Heterocyclylrest mit 3 bis 7, insbesondere 3 bis 6 Ringatomen oder ein heteroaromatischer Rest mit 5 oder 6 Ringatomen. Vorzugsweise enthält Heterocyclyl Heteroringatome aus der Gruppe N, O und S.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidinyl, Piperidinyl, insbesondere Oxiranyl, 2-Oxetanyl, 3-Oxetanyl oder Oxolanyl, oder ein heterocyclischer Rest mit zwei oder drei Heteroatomen, beispielsweise Pyrimidinyl, Pyridazinyl, Pyrazinyl, Triazinyl, Thienyl, Thiazolyl, Thiadiazolyl, Oxazolyl, Isoxazolyl, Pyrazolyl, Triazolyl, Piperazinyl, Dioxolanyl, Dioxanyl, Oxazolinyl, Isoxazolinyl, Oxazolidinyl, Isoxazolidinyl oder Morpholinyl.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Im Falle von mehreren gegebenenfalls substituierten Grundkörpern bedeutet die Definition "wobei jeder der letztgenannten Reste (= Grundkörper) unsubstituiert oder substituiert ist", dass jeder der Reste (= Grundkörper) unabhängig von den anderen Resten unsubstituiert oder substituiert ist.

Als Substituenten für einen substituierten heterocyclischen Rest kommen die weiter unten genannten Substituenten in Frage, zusätzlich auch Oxo. Die Oxogruppe als Substituent bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂-(auch kurz SO₂) im heterocyclischen Ring.

Substituierte Reste, wie ein substituierter Alkyl-, Alkenyl-, Alkinyl-, Cycloalkyl-, Cycloalkenyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkanoyl, Alkoxycarbonyloxy, Alkanoyloxy, Aryloxycarbonyl, Aryloxycarbonyloxy, Carbamoyl, Mono- und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, Alkylsulfonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und Dialkylaminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Arylcarbonyloxy, Alkanoyloxy, Alkenyl-carbonyloxy, Alkinyl-carbonyloxy, Alkoxycarbonyloxy, Alkenyloxy-carbonyloxy, Alkinyloxy-carbonyloxy, Aryloxycarbonyloxy, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkylaminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie gesättigte N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Fluorphenyl, 2-, 3-und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl -und(C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist,

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren, Phosphinsäuren.

Acyl bedeutet beispielsweise Alkanoyl wie Formyl oder [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl, N-Alkyl- und N,N-Dialkylcarbamoyl und andere Reste von organischen Säuren. Dabei können die Reste jeweils im Alkyl- oder Phenylteil noch weiter substituiert sein, beispielsweise im Alkylteil durch ein oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Alkanoyl, wie Formyl und Acetyl, Aroyl wie Phenylcarbonyl, und andere Reste von gesättigten oder ungesättigten organischen Säuren.

"Aroyl" bedeutet einen wie vorstehend definierter Arylrest, der über eine CarbonylGruppe gebunden ist, z.B. die Benzoyl-Gruppe.

Wenn ein allgemeiner Rest mit "Wasserstoff" definiert ist, bedeutet dies ein Wasserstoffatom.

Mit "yl-Position" eines Restes ist dessen Bindungstelle bezeichnet.

Aus Gründen der besseren Herstellbarkeit, insbesondere bezüglich der Ausbeute, Raumzeitausbeute, Enantioselektivität des Verfahrens und Einfachheit der Verfahrensführung, aber auch aus Gründen der Verwendbarkeit sind erfindungsgemäße Verfahren zur Herstellung von Aminoverbindungen der Formel (I) von näherem Interesse, worin
- A: gemeinsam mit den jeweils mit einem Stern (*) bezeichneten C-Atomen einen carbocyclischen oder heterocyclischen gesättigten oder ungesättigten, nicht- aromatischen Ring mit 4 bis 9 Ringatomen, insbesondere mit 5 bis 7 Ringatomen, der zusätzlich zu den Resten R und NH-R⁰ unsubstituiert oder durch einen oder mehrere Reste aus der Gruppe Halogen, Hydroxy, Amino, Cyano, Nitro, (C₁-C₄)Alkyl, (C₃-C₉)Cycloalkyl, (C₂-C₄)Alkenyl, (C₅- C₉)Cycloalkenyl, (C₂-C₄)Alkinyl, (C₁-C₄)Alkoxy, (C₁-C₄)Alkoxycarbonyl, (C₁- C₄)Alkylthio, (C₁-C₄)Alkylsulfonyl, Aryl und Heterocyclyl, wobei die letztgenannten 11 Reste unabhängig voneinander unsubstituiert oder durch einen oder mehrere Reste aus der Gruppe Halogen, Cyano, (C₃- C₉)Cycloalkyl, (C₄-C₉)Cycloalkenyl, (C₁-C₄)Alkoxy, (C₁-C₄)Alkylthio und im Falle cyclischer Reste auch (C₁-C₄)Alkyl und (C₁-C₄)Haloalkyl substituiert sind, weiter substituiert ist und gegebenenfalls durch einen annellierten carbocyclische oder heterocyclischen Ring mit 3 bis 30 Ringatomen substituiert ist, der im Falle eines heterocyclischen Rings 1 bis 3 Ringatome aus der Gruppe N, O und S enthält und der gegebenenfalls weiter substituiert ist durch einen oder mehrere Reste aus der Gruppe Halogen, Cyano, (C₁- C₄)Alkyl, (C₃-C₉)Cycloalkyl, (C₂-C₄)Alkenyl, (C₅-C₉)Cycloalkenyl, (C₂- C₄)Alkinyl, (C₁-C₄)Alkoxy und (C₁-C₄)Alkylthio, wobei jeder der letztgenannten 7 Reste unabhängig voneinander unsubstituiert oder durch einen oder mehrere Reste aus der Gruppe Halogen, Cyano, (C₃-C₉)Cycloalkyl, (C₄- C₉)Cycloalkenyl, (C₁-C₄)Alkoxy, (C₁-C₄)Alkylthio und im Falle cyclischer Reste auch (C₁-C₄)Alkyl und (C₁-C₄)Haloalkyl substituiert ist, bedeutet,
- R⁰: unabhängig von R einen Rest (C₁-C₆)Alkyl, (C₂-C₆)Alkenyl oder (C₃-C₆)Alkinyl, wobei jeder der drei letztgenannten Reste unsubstituiert oder durch einen oder mehrere Reste aus der Gruppe Halogen, Cyano, (C₁- C₄)Alkoxy, (C₁-C₄)Haloalkoxy, (C₁-C₄)Alkylthio, (C₁-C₄)Alkanoyloxy, (C₁-C₄)Haloalkanoyloxy, Phenyl, das unsubstituiert oder substituiert ist, und Heterocycl, das unsubstituiert oder substituiert ist, substituiert ist,
oder (C₃-C₉)Cycloalkyl, das unsubstituiert oder substituiert ist, vorzugsweise unsubstituiert oder durch einen oder mehrere Reste aus der Gruppe Halogen, Cyano, (C₁-C₄)Alkyl, (C₁-C₄)Haloalkyl, (C₁-C₄)Alkoxy, (C₁- C₄)Haloalkoxy, (C₁-C₄)Alkylthio und Phenyl, das unsubstituiert oder substituiert ist, substituiert ist,
oder (C₄-C₉)Cycloalkenyl, das unsubstituiert oder substituiert ist, vorzugsweise unsubstituiert oder durch einen oder mehrere Reste aus der Gruppe Halogen, Cyano, (C₁-C₄)Alkyl, (C₁-C₄)Haloalkyl, (C₁- C₄)Alkoxy, (C₁-C₄)Haloalkoxy, (C₁-C₄)Alkylthio und Phenyl, das unsubstituiert oder substituiert ist, substituiert ist und gegebenenfalls an einer oder mehreren Doppelbindungen im Ring mit einem weiter unsubstituierten oder substituierten aromatischen Ring kondensiert, vorzugsweise benzokondensiert ist,
oder Phenyl, das unsubstituiert oder substituiert ist, vorzugsweise unsubstituiert oder durch einen oder mehrere Reste aus der Gruppe Halogen, Cyano, (C₁-C₄)Alkyl, (C₁-C₄)Haloalkyl, (C₁-C₄)Alkoxy, (C₁- C₄)Haloalkoxy, (C₁-C₄)Alkylthio und Phenyl, das unsubstituiert oder substituiert ist, substituiert ist und gegebenenfalls an einer oder mehreren Doppelbindungen im Ring mit einem weiter unsubstituierten oder substituierten aromatischen Ring kondensiert, vorzugsweise benzokondensiert ist,
oder Heterocyclyl, das unsubstituiert oder substituiert ist, vorzugsweise unsubstituiert oder durch einen oder mehrere Reste aus der Gruppe Halogen, Cyano, (C₁-C₄)Alkyl, (C₁-C₄)Haloalkyl, (C₁-C₄)Alkoxy, (C₁- C₄)Haloalkoxy, (C₁-C₄)Alkylthio und Phenyl, das unsubstituiert oder substituiert ist, substituiert ist und gegebenenfalls im Fall eines ungesättigten oder heteroaromatischen Heterocyclyls an einer oder mehreren Doppelbindungen im Ring mit einem weiter unsubstituierten oder substituierten aromatischen Ring kondensiert, vorzugsweise benzokondensiert ist,
bedeutet, oder
insbesondere R⁰ einen abspaltbaren Rest aus der Gruppe
i) CH₂C₆H₅ (Benzyl)
ii) CH₂CH=CH₂ (Allyl)
iii) C(CH₃)₃ (t-Butyl)
iv) C(C₆H₅)₃ (Trityl)
v) CH₃ (Methyl)
vi) (CH₂)₃OCOCH₃ (3-Acetoxypropyl)
vii) CH₂C₆H₃-2,4-(OCH₃)₂ (2,4-Dimethoxybenzyl)
viii) C₆H₃-2,4-(NO₂)₂ (2,4-Dinitrophenyl)
ix) CH₂C₆H₄-4-OCH₃ (4-Methoxybenzyl)
x) CH₂C₆H₄-2-OH (2-Hydroxybenzyl)
xi) CH(C₆H₅)₂ (Diphenylmethyl)
xii) CH(C₆H₄-4-OCH₃)₂ (Bis-(4-methoxyphenyl)methyl)
xiii) C(C₆H₅)₂(C₆H₄-4-OCH₃) (4-Methoxyphenyl)diphenylmethyl)
xiv) 5-Dibenzosuberyl (Dibenzo-[b,f]-cyclohepta-2,6-dien-1-yl)
xv) 9-Phenylfluoren-9-yl (1-Phenyl-diphenylen-methyl)
bedeutet,
wobei jeder der genannten Reste Phenyl oder Heterocylyl, der unsubstituiert oder substituiert ist (d.h. gegebenenfalls substituiert ist ohne Angabe der Substituenten), vorzugsweise unsubstituiert oder durch einen oder mehrere Reste aus der Gruppe Halogen, Cyano, Nitro, (C₁-C₄)Alkyl, (C₂-C₄)Alkenyl, (C₂-C₄)Alkinyl, (C₁-C₄)Haloalkyl, (C₁-C₄)Alkoxy-(C₁-C₄)alkyl, (C₁-C₄)Alkoxy (C₁-C₄)Haloalkoxy, (C₁-C₄)Alkoxy-(C₁-C₄)alkyloxy, (C₁-C₄)Alkylthio, (C₁- C₄)Alkylsulfinyl, (C₁-C₄)Alkylsulfonyl, (C₁-C₄)Alkanoyl, [(C₁-C₄)Alkoxy]- carbonyl, ((C₁-C₄)Alkylamino und Di[(C₁-C₄)alkyl]-amino substituiert ist, insbesondere jeder der genannten Reste Phenyl oder Heterocyclyl der unsubstituiert oder substituiert ist, vorzugsweise unsubstituiert oder durch einen oder mehrere Reste aus der Gruppe Halogen, (C₁-C₄)Alkyl, (C₁- C₄)Alkoxy, (C₁-C₄)Haloalkyl, (C₁-C₄)Haloalkoxy und Nitro substituiert ist,
- R: unabhängig von R⁰
(C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, Cyano, (C₁-C₄)Alkoxy, (C₁- C₄)Haloalkoxy, (C₁-C₄)Alkylthio, Aryl, das gegebenenfalls substituiert ist, und Heteroaryl, das gegebenenfalls substituiert ist, substituiert ist, oder
Aryl, das gegebenenfalls substituiert ist, oder
Heteroaryl, das gegebenenfalls substituiert ist,
bedeutet,
wobei die gegebenenfalls substituierten Reste unabhängig voneinander vorzugsweise unsubstituiert oder durch einen oder mehrere Reste aus der Gruppe Halogen, Hydroxy, Amino, Cyano, Nitro, (C₁-C₄)Alkyl, (C₃- C₉)Cycloalkyl, (C₂-C₄)Alkenyl, (C₅-C₉)Cycloalkenyl, (C₂-C₄)Alkinyl, (C₁- C₄)Alkoxy, (C₁-C₅)Alkanoyl, [(C₁-C₄)Alkoxy]-carbonyl, (C₁-C₄)Alkylthio, (C₁-C₄)Alkylsulfonyl, (C₁-C₄)Alkylamino, Di-(C₁-C₄)alkylamino, Aryl und Heterocyclyl, wobei die letztgenannten 14 Reste unabhängig voneinander unsubstituiert oder durch einen oder mehrere Reste aus der Gruppe Halogen, Cyano, (C₃-C₉)Cycloalkyl, (C₅-C₉)Cycloalkenyl, (C₁-C₄)Alkoxy, (C₁-C₄)Alkylthio und im Falle cyclischer Reste auch (C₁- C₄)Alkyl und (C₁-C₄)Haloalkyl substituiert sind, substituiert sind, oder
- R⁰ und A: einen Ring B1 bilden und dabei gemeinsam mit der NH-Gruppe und dem an der NH-Gruppe gebundenen, mit einem Stern (*) bezeichneten C-Atom einen heterocyclischen Ring mit 4 bis 30 Ringatomen, vorzugsweise mit 4 bis 9 Ringatomen, insbesondere mit 5 bis 7 Ringatomen, der gegebenenfalls zusätzlich weiter substituiert ist und der gegebenenfalls 1 oder 2 weitere Heteroatome aus der Gruppe N, O und S enthält, bedeuten,
vorzugsweise den genannten heterocyclischen Ring bedeuten, der weiter unsubstituiert oder durch einen oder mehrere Reste aus der Gruppe Halogen, Hydroxy, Amino, Cyano, Nitro, (C₁-C₄)Alkyl, (C₁-C₄)Haloalkyl, (C₃- C₉)Cycloalkyl, (C₂-C₄)Alkenyl, (C₅-C₉)Cycloalkenyl, (C₂-C₄)Alkinyl, (C₁- C₄)Alkoxy, (C₁-C₄)Haloalkoxy, (C₁-C₄)Alkylthio und (C₁-C₄)Alkylsulfonyl substituiert ist, oder
- R und A: einen Ring B2 bilden und dabei gemeinsam mit dem an R gebundenen, mit einem Stern (*) bezeichneten C-Atom einen carbocyclischen oder heterocyclischen Ring mit 3 bis 30 Ringatomen, vorzugsweise mit 3 bis 9 Ringatomen, insbesondere mit 4 bis 7 Ringatomen, der gegebenenfalls zusätzlich weiter substituiert ist und der im Falle eines heterocyclischen Rings 1 oder 2 oder 3 weitere Heteroatome aus der Gruppe N, O und S enthält, bedeuten,
vorzugsweise den genannten carbocyclischen oder heterocyclischen Ring bedeuten, der weiter unsubstituiert oder durch einen oder mehrere Reste aus der Gruppe Halogen, Hydroxy, Amino, Cyano, Nitro, (C₁-C₄)Alkyl, (C₁-C₄)Haloalkyl, (C₃-C₉)Cycloalkyl, (C₂-C₄)Alkenyl, (C₅-C₉)Cycloalkenyl, (C₂- C₄)Alkinyl, (C₁-C₄)Alkoxy, (C₁-C₄)Haloalkoxy, (C₁-C₄)Alkylthio und (C₁- C₄)Alkylsulfonyl substituiert ist,
oder
- R⁰ und R: einen Ring B3 bilden und dabei gemeinsam mit der NH-Gruppe und den jeweils mit einem Stern (*) bezeichneten C-Atomen einen heterocyclischen Ring mit 4 bis 30 Ringatomen, vorzugsweise mit 4 bis 9 Ringatomen, insbesondere mit 5 bis 7 Ringatomen, der gegebenenfalls zusätzlich weiter substituiert ist und der gegebenenfalls 1 oder 2 weitere Heteroatome aus der Gruppe N, O und S enthält, bedeuten,
vorzugsweise den genannten heterocyclischen Ring bedeuten, der weiter unsubstituiert oder durch einen oder mehrere Reste aus der Gruppe Halogen, Hydroxy, Amino, Cyano, Nitro, (C₁-C₄)Alkyl, (C₁-C₄)Haloalkyl, (C₃- C₉)Cycloalkyl, (C₂-C₄)Alkenyl, (C₅-C₉)Cycloalkenyl, (C₂-C₄)Alkinyl, (C₁- C₄)Alkoxy, (C₁-C₄)Haloalkoxy, (C₁-C₄)Alkylthio und (C₁-C₄)Alkylsulfonyl substituiert ist, oder
- R⁰ und R: und gegebenenfalls A zwei oder mehrere der genannten Ringe B1, B2 und B3 zugleich bilden,
wobei R und die Gruppe NH-R⁰ an den beiden jeweils mit einem Stern (*) markierten Ring-C-Atomen in cis-Stellung zueinander ausgerichtet sind und die stereochemische Konfiguration an diesen C-Atomen von der racemischen Konfiguration verschieden ist.

Bevorzugte erfindungsgemäße Verfahren betreffen beispielsweise die Herstellung von monocyclischen Verbindungen der Formel (Ia) 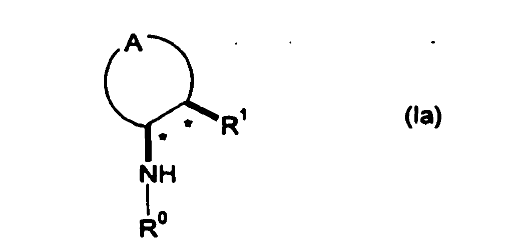 worin
- A: gemeinsam mit den jeweils mit einem Stern (*) bezeichneten C-Atomen einen carbocyclischen oder heterocyclischen, gesättigten oder ungesättigten, nicht- aromatischen Ring mit 3 bis 30 Ringatomen, vorzugsweise mit 4 bis 9 Ringatomen, insbesondere mit 5 bis 7 Ringatomen, und im Falle eines heterocyclischen Rings mit 1 bis 3 Heteroringatomen aus der Gruppe N, O und S, der zusätzlich zu den Resten R¹ und NH-R⁰ unsubstituiert oder durch einen oder mehrere Reste aus der Gruppe Halogen, Hydroxy, Amino, Cyano, Nitro, (C₁-C₄)Alkyl, (C₃-C₉)Cycloalkyl, (C₁-C₄)Alkoxy, (C₁-C₄)Alkoxycarbonyl, (C₁-C₄)Alkylthio, (C₁-C₄)Alkylsulfonyl, Aryl und Heterocyclyl, wobei jeder der letztgenannten 8 Reste unsubstituiert oder durch einen oder mehrere Reste aus der Gruppe Halogen, Cyano, (C₃-C₉)Cycloalkyl, (C₁-C₄)Alkoxy, (C₁- C₄)Alkylthio und im Falle cyclischer Reste auch (C₁-C₄)Alkyl-und (C₁- C₄)Haloalkyl substituiert ist, weiter substituiert ist, bedeutet,
- R⁰: unabhängig von R¹ wie oben in Formel (I) definiert ist, vorzugsweise wie für Formel (I) bevorzugt definiert ist und insbesondere einen Rest (C₁-C₆)Alkyl, (C₂-C₆)Alkenyl oder (C₃-C₆)Alkinyl,
wobei jeder der drei letztgenannten Reste unsubstituiert oder durch einen oder mehrere Reste aus der Gruppe Halogen, Cyano, (C₁- C₄)Alkoxy, (C₁-C₄)Haloalkoxy, (C₁-C₄)Alkylthio, (C₁-C₄)Alkanoyloxy, (C₁-C₄)Haloalkanoyloxy und
Phenyl, das unsubstituiert oder durch einen oder mehrere Reste aus der Gruppe Halogen, Cyano, Nitro, (C₁-C₄)Alkyl, (C₂-C₄)Alkenyl, (C₂- C₄)Alkinyl, (C₁-C₄)Haloalkyl, (C₁-C₄)Alkow-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio, (C₁- C₄)Alkylsulfinyl, (C₁-C₄)Alkylsulfonyl, (C₁-C₄)-Alkanoyl, [(C₁-C₄)-Alkoxy]- carbonyl, (C₁-C₄)Alkylamino und Di-[(C₁-C₄)alkyl]-amino substituiert ist, und
Heterocyclyl, das unsubstituiert oder durch einen oder mehrere Reste aus der Gruppe Halogen, Cyano, Nitro, (C₁-C₄)Alkyl, (C₂-C₄)Alkenyl, (C₂-C₄)Alkinyl, (C₁-C₄)Haloalkyl, (C₁-C₄)Alkoxy-(C₁-C₄)alkyl, (C₁- C₄)Alkoxy, (C₁-C₄)Haloalkoxy, (C₁-C₄)Alkoxy-(C₁-C₄)alkoxy, (C₁- C₄)Alkylthio, (C₁-C₄)Alkylsulfinyl, (C₁-C₄)Alkylsulfonyl, (C₁-C₄)-Alkanoyl, [(C₁-C₄)-Alkoxy]-carbonyl, (C₁-C₄)Alkylamino und Di-[(C₁-C₄)alkyl]- amino substituiert ist,
substituiert ist,
oder (C₃-C₆)Cycloalkyl, Phenyl oder Heterocyclyl, wobei jeder der 3 letztgenannten Reste gegebenenfalls substituiert ist,
oder beispielsweise einen der oben genannten abspaltbaren Reste i) bis xiii) bedeutet,
- R¹: unabhängig von R⁰
(C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, Cyano, (C₁-C₄)Alkoxy, (C₁- C₄)Haloalkoxy und (C₁-C₄)Alkylthio substituiert ist,
oder Aryl oder Heterocyclyl, wobei jeder der 2 letztgenannten Reste unsubstituiert oder substituiert ist, vorzugsweise unsubstituiert oder wie bei R in Formel (I) für gegebenenfalls substituiertes Phenyl oder Heterocylyl substituiert ist, insbesondere durch eine oder mehrere Reste aus der Gruppe Halogen, (C₁-C₄)Alkyl, (C₁-C₄)Haloalkyl und (C₁- C₄)Haloalkoxy substituiert ist, bedeutet,
wobei die Substituenten R¹ und die Aminogruppe an den beiden jeweils mit einem Stern (*) markierten Ring-C-Atomen in cis-Stellung zueinander ausgerichtet sind und die Verbindung (Ia) als stereochemisch reine Verbindung der Formel (Ia-A) oder (Ia-B), 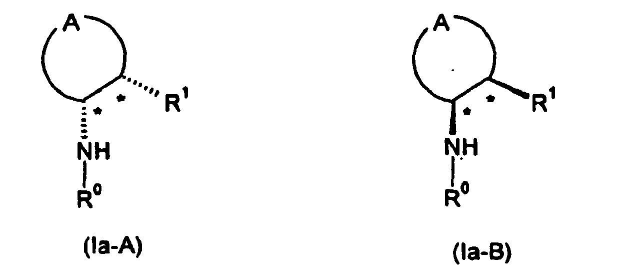 worin die allgemeinen Reste wie in Formel (Ia) definiert sind, oder als Isomerengemisch der Verbindungen der Formeln (Ia-A) und (Ia-B) in einem vom Verhältnis 1:1 verschiedenen Isomerenverhältnis vorliegt.

Bevorzugt sind auch Verfahren zur Herstellung von bicyclischen Aminoverbindungen der Formel (Ib) 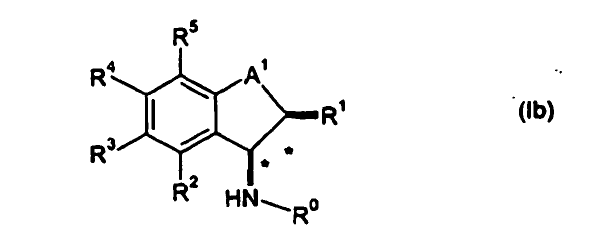 worin
- A¹: eine direkte Bindung oder eine Gruppe der Formel (CR⁶R⁷)ₙ, worin n eine Zahl von 1 bis 6 und R⁶ und R⁷ unabhängig voneinander bzw. im Fall, dass n größer 1 ist, die Reste R⁶ bzw. R⁷ jeweils unabhängig voneinander Wasserstoff, (C₁-C₄)Alkyl, (C₁-C₄)Haloalkyl, Halogen, (C₁-C₄)Alkoxy oder (C₁- C₄)Haloalkoxy bedeuten, wobei einzelne Gruppen CR⁶R⁷ durch Heteroatome aus der Gruppe O und S ersetzt sein können, bedeutet,
- R⁰: wie oben in Formel (I) definiert ist, vorzugsweise wie für Formel (I) bevorzugt definiert ist und insbesondere unabhängig von R¹ einen Rest (C₁-C₆)Alkyl oder (C₂-C₆)Alkenyl,
wobei jeder der beiden letztgenannten Reste unsubstituiert oder durch einen oder mehrere Reste aus der Gruppe Halogen, Cyano, (C₁- C₄)Alkoxy, (C₁-C₄)Haloalkoxy, (C₁-C₄)Alkylthio, (C₁-C₄)Alkanoyloxy, (C₁-C₄)Haloalkanoyloxy und
Aryl, das unsubstituiert oder durch einen oder mehrere Reste aus der Gruppe Halogen, Cyano, Nitro, (C₁-C₄)Alkyl, (C₂-C₄)Alkenyl, (C₂- C₄)Alkinyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio, (C₁- C₄)Alkylsulfinyl, (C₁-C₄)Alkylsulfonyl, (C₁-C₄)-Alkanoyl, [(C₁-C₄)-Alkoxy]- carbonyl, (C₁-C₄)Alkylamino und Di-[(C₁-C₄)alkyl]-amino substituiert ist, und Heterocyclyl, das unsubstituiert oder durch einen oder mehrere Reste aus der Gruppe Halogen, Cyano, Nitro, (C₁-C₄)Alkyl, (C₂- C₄)Alkenyl, (C₂-C₄)Alkinyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁- C₄)Alkylthio, (C₁-C₄)Alkylsulfinyl, (C₁-C₄)Alkylsulfonyl, (C₁-C₄)-Alkanoyl, [(C₁-C₄)-Alkoxy]-carbonyl, (C₁-C₄)Alkylamino und Di-[(C₁-C₄)alkyl]- amino substituiert ist,
oder (C₃-C₆)Cycloalkyl, Phenyl oder Heterocyclyl, wobei jeder der 3 letztgenannten Reste gegebenenfalls substituiert ist,
oder beispielsweise einen der genannten abspaltbaren Reste i) bis xiii)
bedeutet, und
- R¹: unabhängig von R⁰
(C₁-C₆)Alkyl, das unsubstituiert oder durch einen oder mehrere Reste aus der Gruppe Halogen, Cyano, (C₁-C₄)Alkoxy, (C₁-C₄)Haloalkoxy, (C₁- C₄)Alkylthio, Aryl, das gegebenenfalls substituiert ist, und Heterocyclyl, das gegebenenfalls substituiert ist, substituiert ist, oder
Aryl, das gegebenenfalls substituiert ist, oder
Heterocyclyl, das gegebenenfalls substituiert ist,
bedeutet,
wobei die gegebenenfalls substituierten Reste jeweils unabhängig voneinander vorzugsweise unsubstituiert oder durch einen oder mehrere Reste aus der Gruppe Halogen, Hydroxy, Amino, Cyano, Nitro, (C₁- C₄)Alkyl, (C₃-C₉)Cycloalkyl, (C₂-C₄)Alkenyl, (C₅-C₉)Cycloalkenyl, (C₂- C₄)Alkinyl, (C₁-C₄)Alkoxy, (C₁-C₅)Alkanoyl, [(C₁-C₄)Alkoxy]-carbonyl, (C₁- C₄)Alkylthio, (C₁-C₄)Alkylsulfonyl, (C₁-C₄)Alkylamino, Di-(C₁- C₄)alkylamino, Aryl und Heterocyclyl, wobei die letztgenannten 14 Reste unabhängig voneinander unsubstituiert oder durch einen oder mehrere Reste aus der Gruppe Halogen, Cyano, (C₃-C₉)Cycloalkyl, (C₅- C₉)Cycloalkenyl, (C₁-C₄)Alkoxy, (C₁-C₄)Alkylthio und im Falle cyclischer Reste auch (C₁-C₄)Alkyl und (C₁-C₄)Haloalkyl substituiert sind, substituiert sind,
und vorzugsweise
R¹ unabhängig von R⁰
(C₁-C₆)Alkyl, das unsubstituiert oder durch einen oder mehrere Reste aus der Gruppe Halogen, Cyano, (C₁-C₄)Alkoxy, (C₁-C₄)Haloalkoxy und (C₁- C₄)Alkylthio substituiert ist, oder Phenyl oder Heterocyclyl bedeutet und
- R²: H, (C₁-C₄)Alkyl, (C₁-C₃)Haloalkyl, Halogen, (C₁-C₃)Alkoxy, (C₁-C₃)Haloalkoxy oder CN,
- R³: H, (C₁-C₄)Alkyl, (C₁-C₃)Haloalkyl, Halogen, (C₁-C₃)Alkoxy, (C₁-C₃)Haloalkoxy oder CN,
- R⁴: H, (C₁-C₄)Alkyl, (C₁-C₃)Haloalkyl, Halogen, (C₁-C₃)Alkoxy, (C₁-C₃)Hatoalkoxy oder CN und
- R⁵: H, (C₁-C₄)Alkyl, (C₁-C₃)Haloalkyl, Halogen, (C₁-C₃)Alkoxy, (C₁-C₃)Haloalkoxy oder CN
bedeuten,
wobei die Substituenten R¹ und die Aminogruppe an den beiden jeweils mit einem Stern (*) markierten Ring-C-Atomen in cis-Stellung zueinander ausgerichtet sind und die Verbindung als stereochemisch reine Verbindung der Formel (Ib-A) oder (Ib-B), 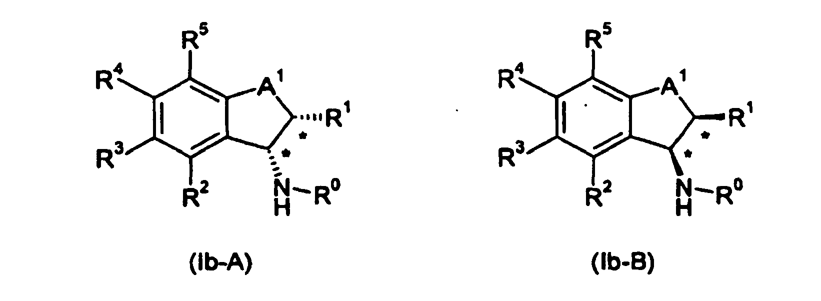 worin die allgemeinen Reste wie in Formel (Ib) definiert sind, oder als Isomerengemisch der Verbindungen der Formeln (Ib-A) und (Ib-B) in einem vom Verhältnis 1:1 verschiedenen Isomerenverhältnis vorliegt.

Bevorzugt sind auch Verfahren zur Herstellung von bicyclischen Aminoverbindungen der Formel (Ic) 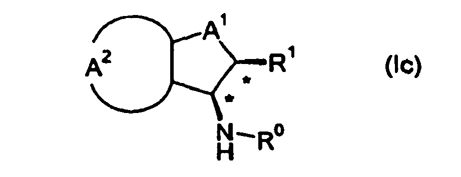 worin
- A¹: eine direkte Bindung oder eine Gruppe der Formel (CR⁶R⁷)ₙ, worin n eine Zahl von 1 bis 6 und R⁶ und R⁷ unabhängig voneinander bzw. im Fall, dass n größer 1 ist, die Reste R⁶ bzw. R⁷ jeweils unabhängig voneinander Wasserstoff, (C₁-C₄)Alkyl, (C₁-C₄)Haloalkyl, Halogen, (C₁-C₄)Alkoxy oder (C₁- C₄)Haloalkoxy bedeuten, wobei einzelne Gruppen CR⁶R⁷ durch Heteroatome aus der Gruppe O und S ersetzt sein können, bedeutet.
- A²: zusammen mit den beiden C-Atomen, die mit dem anderen Ring gemeinsam sind, einen carbocyclischen nichtaromatischen Ring mit 3 bis 9 C-Atomen bilden, der unsubstituiert oder durch einen oder mehrere Reste aus der Gruppe (C₁-C₄)Alkyl, (C₁-C₃)Haloalkyl, Halogen, (C₁-C₃)Alkoxy, (C₁- C₃)Haloalkoxy, Hydroxy, Amino und CN substituiert ist, bedeutet,
- R⁰: wie oben in Formel (I) definiert ist, vorzugsweise wie für Formel (I) bevorzugt definiert ist und insbesondere unabhängig voneinander wie in Formel (Ib) definiert ist,
und
- R¹: unabhängig voneinander wie in Formel (Ib) definiert ist,
wobei die Substituenten R¹ und die Aminogruppe an den beiden jeweils mit einem Stern (*) markierten Ring-C-Atomen in cis-Stellung zueinander ausgerichtet sind und die Verbindung (Ic) als stereochemisch reine Verbindung der Formel (Ic-A) oder (Ic-B), 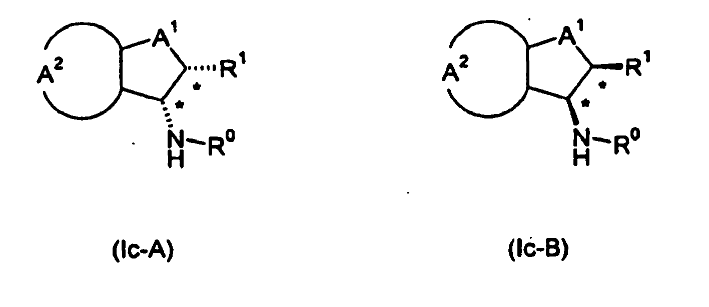 worin die allgemeinen Reste wie in Formel (Ic) definiert sind, oder als Isomerengemisch der Verbindungen der Formeln (Ic-A) und (Ic-B) in einem vom Verhältnis 1:1 verschiedenen Isomerenverhältnis vorliegt.

Bevorzugt sind auch Verfahren zur Herstellung von bicyclischen Aminoverbindungen der Formel (Id) 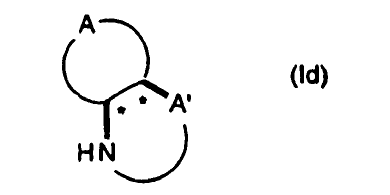 worin
- A: gemeinsam mit den jeweils mit einem Stern (*) bezeichneten C-Atomen einen carbocyclischen oder heterocyclischen, gesättigten oder ungesättigten, nicht- aromatischen Ring mit 4 bis 9 Ringatomen, insbesondere mit 5 bis 7 Ringatomen, und im Falle eines heterocyclischen Rings mit 1 bis 3 Heteroringatomen aus der Gruppe N, O und S, der zusätzlich zu den Resten A' und NH-R⁰ unsubstituiert oder durch einen oder mehrere Reste aus der Gruppe Halogen, Cyano, (C₁-C₄)Alkyl, (C₃-C₉)Cycloalkyl, (C₁-C₄)Alkoxy und (C₁-C₄)Alkylthio, wobei jeder der letztgenannten 4 Reste unsubstituiert oder durch einen oder mehrere Reste aus der Gruppe Halogen, Cyano, (C₃- C₉)Cycloalkyl, (C₁-C₄)Alkoxy, (C₁-C₄)Alkylthio und im Falle cyclischer Reste auch (C₁-C₄)Alkyl und (C₁-C₄)Haloalkyl substituiert ist, weiter substituiert ist, bedeutet und
- A': gemeinsam mit der NH-Gruppe und den jeweils mit einem Stern (*) bezeichneten C-Atomen einen heterocyclischen Ring mit 4 bis 9 Ringatomen, insbesondere mit 5 bis 7 Ringatomen, der gegebenenfalls 1 oder 2 weitere Heteroatome aus der Gruppe N, O und S enthält und der unsubstituiert oder durch einen oder mehrere Reste aus der Gruppe Halogen, Cyano, (C₁- C₄)Alkyl, (C₁-C₄)Haloalkyl, (C₁-C₄)Alkoxy, (C₁-C₄)Haloalkow, (C₁-C₄)Alkylthio und Phenyl, das unsubstituiert oder durch einen oder mehrere Reste aus der Gruppe Halogen, Cyano, Nitro, (C₁-C₄)Alkyl, (C₂-C₄)Alkenyl, (C₂-C₄)Alkinyl, (C₁-C₄)Haloalkyl, (C₁-C₄)Alkoxy-(C₁-C₄)alkyl, (C₁-C₄)Alkoxy, (C₁- C₄)Haloalkoxy, (C₁-C₄)Alkow-(C₁-C₄)alkow, (C₁-C₄)Alkylthio, (C₁- C₄)Alkylsulfinyl, (C₁-C₄)Alkylsulfonyl, (C₁-C₄)-Alkanoyl, [(C₁-C₄)-Alkoxy]- carbonyl, (C₁-C₄)Alkylamino und Di-[(C₁-C₄)alkyl]-amino substituiert ist, substituiert ist, bedeutet,
wobei die Substituenten A' und die Aminogruppe an den beiden jeweils mit einem Stern (*) markierten Ring-C-Atomen in cis-Stellung zueinander ausgerichtet sind und die Verbindung (Id) als stereochemisch reine Verbindung der Formel (Id-A) oder (Id-B), 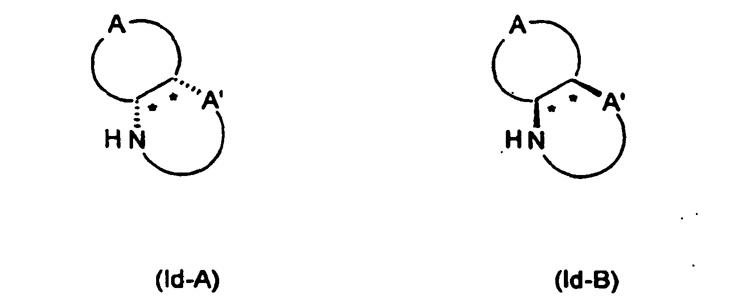 worin die allgemeinen Reste wie in Formel (Id) definiert sind, oder als Isomerengemisch der Verbindungen der Formeln (Id-A) und (Id-B) in einem vom Verhältnis 1:1 verschiedenen Isomerenverhältnis vorliegt.

Bevorzugt sind auch Verfahren zur Herstellung von bicyclischen Aminoverbindungen der Formel (Ie) 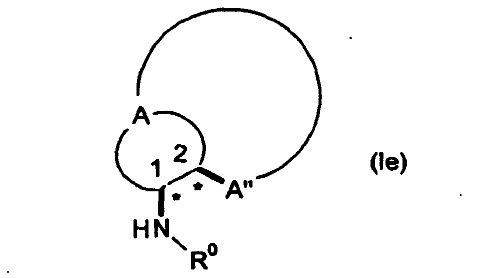 worin
- A: gemeinsam mit den jeweils mit einem Stern (*) bezeichneten C-Atomen einen carbocyclischen oder heterocyclischen, gesättigten oder ungesättigten, nicht- aromatischen Ring mit 4 bis 9 Ringatomen, insbesondere mit 5 bis 7 Ringatomen, und im Falle eines heterocyclischen Rings mit 1 bis 3 Heteroringatomen aus der Gruppe N, O und S, der zusätzlich zu den Resten A" und NH-R⁰ unsubstituiert oder durch einen oder mehrere Reste aus der Gruppe Halogen, Cyano, (C₁-C₄)Alkyl, (C₃-C₉)Cycloalkyl, (C₁-C₄)Alkoxy und (C₁-C₄)Alkylthio, wobei jeder der letztgenannten 4 Reste unsubstituiert oder durch einen oder mehrere Reste aus der Gruppe Halogen, Cyano, (C₃- C₉)Cycloalkyl, (C₁-C₄)Alkoxy, (C₁-C₄)Alkylthio und im Falle cyclischer Reste auch (C₁-C₄)Alkyl und (C₁-C₄)Haloalkyl substituiert ist, weiter substituiert ist, bedeutet und
- A": gemeinsam mit A und dem mit "2" bezeichneten C-Atom (= das mit einem Stern bezeichnete, an A" gebundene C-Atom) einen carbocyclischen oder heterocyclischen Ring mit 4 bis 9 Ringatomen, insbesondere mit 5 bis 7 Ringatomen, der im heterocyclischen Fall 1 oder 2 oder 3 Heteroatome aus der Gruppe N, O und S enthält und der unsubstituiert oder durch einen oder mehrere Reste aus der Gruppe Halogen, Hydroxy, Amino, Cyano, Nitro, (C₁- C₄)Alkyl, (C₁-C₄)Haloalkyl, (C₁-C₄)Alkoxy, (C₁-C₄)Haloalkoxy, (C₁-C₄)Alkylthio und Phenyl, das unsubstituiert oder durch einen oder mehrere Reste aus der Gruppe Halogen, Cyano, Nitro, (C₁-C₄)Alkyl, (C₂-C₄)Alkenyl, (C₂-C₄)Alkinyl, (C₁-C₄)Haloalkyl, (C₁-C₄)Alkoxy-(C₁-C₄)alkyl, (C₁-C₄)Alkoxy, (C₁- C₄)Haloalkoxy, (C₁-C₄)Alkoxy-(C₁-C₄)alkoxy, (C₁-C₄)Alkylthio, (C₁- C₄)Alkylsulfinyl, (C₁-C₄)Alkylsulfonyl, (C₁-C₄)-Alkanoyl, [(C₁-C₄)-Alkoxy]- carbonyl, (C₁-C₄)Alkylamino und Di-[(C₁-C₄)alkyl]-amino substituiert ist, substituiert ist, bedeutet,
wobei R⁰ unabhängig von A" wie in den Formeln (I), (Ia), (Ib) oder (Ic) definiert ist und die Substituenten A" und die Aminogruppe an den beiden jeweils mit einem Stern (*) markierten Ring-C-Atomen in cis-Stellung zueinander ausgerichtet sind und die Verbindung (Ie) als stereochemisch reine Verbindung der Formel (Ie-A) oder (Ie-B), 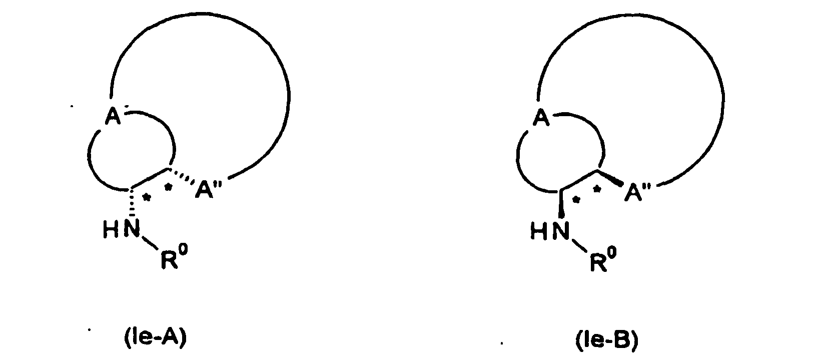 worin die allgemeinen Reste wie in Formel (Ie) definiert sind, oder als Isomerengemisch der Verbindungen der Formeln (Ie-A) und (Ie-B) in einem vom Verhältnis 1:1 verschiedenen Isomerenverhältnis vorliegt.

Die Reduktion der Iminogruppe der Verbindung (II) kann analog Verfahren, wie sie beispielsweise für die Reduktion von Carbonylverbindungen bekannt sind, oder besonders dafür entwickelten Verfahren als asymmetrische katalytische Hydrierung oder katalytische Transferhydrierung in Gegenwart von Wasserstoff oder Wasserstoffdonoren und chiralen Übergangsmetall-Katalysatoren unter Reaktionsbedingungen durchgeführt werden, bei denen ein chemisches Gleichgewicht zwischen Imin (II-A) und Imin (II-B) ermöglicht wird.

Für die Umsetzung kommen chirale Katalysatoren in Frage, die analog für asymmetrische Reduktionen von Ketonen beschrieben sind, beispielsweise beschrieben sind in den nachfolgend genannten Literaturstellen oder in den jeweils dort zitierten Literaturstellen:
Angew. Chem. Int. Ed. 2001 (40) S. 40-73,
Tetrahedron Lett. 1999 (40) 5043-5046,
J. Org. Chem. 1996, 61, 4872-4873
Angew. Chem. Int. Ed. 2004 (116) 806-843, speziell die Seiten 829-830 und dort zitierte Literatur,
Tetrahedron Asymmetry. 2003 (14) 1407-1446,
Org. Lett 1999 (1) 1119-1121,
Tetrahedron 2003 (59) 8291-8327.

Als Katalysatoren für die Reduktionen eignen sich beispielsweise
1. Ruthenium-diphosphane-1,2-diamin-komplexe der Formel (1) und (2) und analoge Komplexe der Formeln (1') bzw. (2') mit anderen Diaminbrücken, 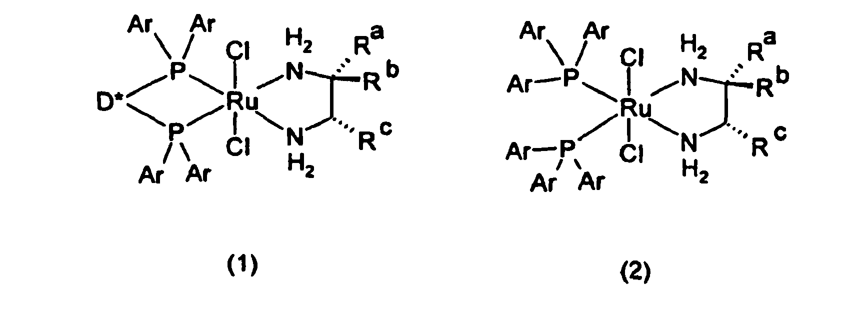 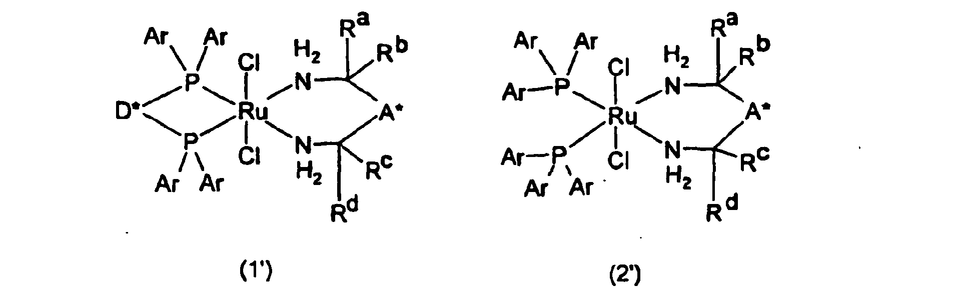 wobei jeweils
   - Ar: einen Arylrest, vorzugsweise Phenylrest, der unsubstituiert oder substituiert ist,
   - D*: eine chirale organische Gruppe,
   - R^{a}, R^{b}, R^{c} und R^{d}: jeweils Wasserstoff oder gegebenenfalls substituierte aliphatische oder aromatische Gruppen, die auch paarweise miteinander verbunden sein können, wobei die 1,2-Diaminbrücke im Fall der Verbindung (1) chiral oder achiral ist und im Fall der Verbindung (2) chiral ist,
   - A*: Alkylen mit 1 bis 4 C-Atomen, vorzugsweise 2 C-Atomen zwischen den Bindungsstellen, wobei Alkylen gegebenenfalls substituiert ist, vorzugsweise unsubstituiert oder durch einen oder mehrere Reste aus der Gruppe Halogen, (C₁-C₄)Alkyl, (C₁-C₄)Alkoxy, (C₁-C₄)Alkylthio und (C₁-C₄)Haloalkyl substituiert ist, oder Cycloalkylen mit 3 bis 6 C-Atomen, das unsubstituiert oder durch einen oder mehrere Reste aus der Gruppe Halogen, (C₁-C₄)Alkyl, (C₁- C₄)Alkoxy, (C₁-C₄)Alkylthio und (C₁-C₄)Haloalkyl, vorzugsweise entsprechend gegebenenfalls substituiertes 1,2-Cycloalkylen, oder einen divalenten heterocyclischen Rest mit 3 bis 6 Ringatomen und 1, 2 oder 3 Heteroringatomen aus der Gruppe O, S und N, wobei der Ring unsubstituiert oder durch einen oder mehrere Reste aus der Gruppe Halogen, (C₁-C₄)Alkyl, (C₁-C₄)Alkoxy, (C₁-C₄)Alkylthio und (C₁- C₄)Haloalkyl substituiert ist,
   bedeuten.
   Die Verbindungen (1) und (2) bzw. (1') und (2') können aus Ruthenium-salzen und den Diphosphanbrücken- und Diaminbrückenverbindungen vor der Hydrierung oder in situ hergestellt werden.
   Beispiele für Diphenylphosphanbrücken Ar₂P-D*-PAr₂ zur Herstellung von Katalysatoren (1) bzw. (1') sind Verbindungen der Formel (1a), (1b) und (1c): 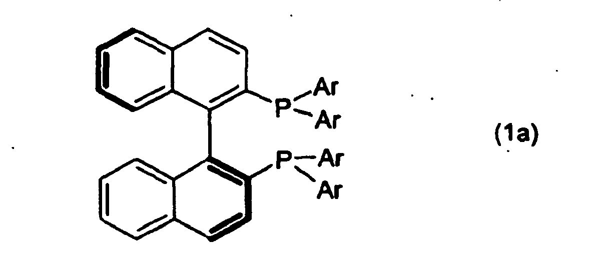 speziell die Verbindungen:
   (S)-BINAP: Ar = Phenyl [1,1'-Binaphthyl-2,2'-bis-(diphenylphosphan)]
   (S)-TolBINAP: Ar = 4-Tolyl [1,1'-Binaphthyl-2,2'-bis-(di-p-totylphosphan)]
   (S)-XyIBINAP: Ar = 3,5-Xylyl [1,1'-Binaphthyl-2,2'-bis-(di-m-xylylphosphan)] und entsprechend
   (R)-BINAP, (R)-ToIBINAP bzw. (R)-XyIBINAP 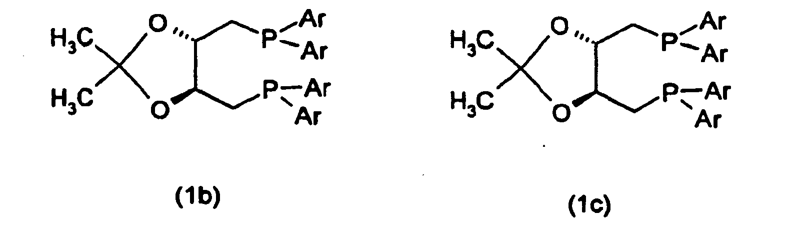 (1b) = (S,S)-DIOP
      (1c) = (S,S)-CHIRAPHOS

   Beispiele für chirale Diaminbrücken für die Herstellung von Katalysatoren (1) und (2) bzw. (1') und (2') sind die folgenden Verbindungen:
   (S,S)-DPEN = (1S,2S)-1,2-Diamino-1,2-diphenylethan,
   (S,S)-1,2-Diaminocyclohexan,
   (S)-DAIPEN = (2S)-1,2-Diamino-1,1-bis-(4-methoxyphenyl)-3-methyl-butan, 3,4-0-Isopropyliden-(3S,4S)-dihydroxy-(2S,5S)-diaminohexan
   und entsprechend die enantiomeren Verbindungen
   (R,R)-DPEN, (R,R)-1,2-Diaminocyclohexan, (R)-DAIPEN und 3,4-O-Isopropyliden-(3R,4R)-dihydroxy-(2R,5R)-diaminohexan.

   Als achirale Diamine zur Herstellung von Verbindungen (1) bzw. (1') kommt beispielsweise 1,2-Diaminoethan in Frage.
2. Entsprechend den unter 1. genannten Ruthenium-komplexen sind auch ähnliche Iridium-komplexe einsetzbar, beispielsweise [Ir((R)-BINAP)(cod)]BF₄ + P[C₆H₄-2-N(CH₃)₂]₂C₆H₅, wobei "cod" den Ligand Cyclooctadien bezeichnet.
3. Entsprechend den unter 1. genannten Ruthenium-komplexen sind auch ähnliche Rhodium-komplexe einsetzbar, beispielsweise (R.S,R,S)-Me-PennPhos-Rh
   oxoProNOP-Rh
4. Metallkomplexe der Formel (3) 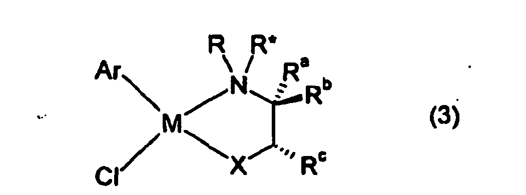 worin
   - M ein: Metall-(II)-Ion aus der Gruppe Ru, Rh und Ir,
   - X: O oder NR*R**,
   - Ar: einen Arylrest, vorzugsweise Phenyl, das unsubstituiert oder substituiert ist, insbesondere Alkylphenyl,
   - R^{a}, R^{b} und R^{c}: gegebenenfalls substituierte aliphatische oder aromatische Gruppen, die auch paarweise miteinander verbunden sein können, wobei die Brücke chiral ist, und
   - R, R*, R** und R***: jeweils unabhängig voneinander eine freies Elektronenpaar, H, Alkyl oder Acyl
   bedeuten,
   insbesondere entsprechende Ruthenium-(II)-1,2-diamin-komplexe,
5. Rhodium-(III) und Iridium-(III)-komplexe der Formeln (4) bzw. (5), welche chiralen bicyclische Liganden enthalten, und deren enantiomeren Formen, 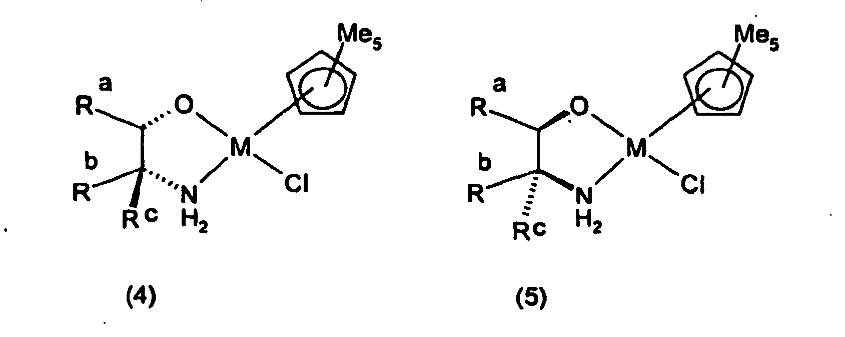 worin M ein Rh oder Ir und R^{a}, R^{b} und R^{c} jeweils organische Reste bedeuten. Katalysatoren aus dieser Gruppe sind teilweise auch kommerziell erhältlich (Katalysatoren ®Cathy von Avecia) wie z. B.
   Rhodium-(III) und Iridium-(III)-komplexe der Formeln (4a) oder (4b), welche chiralen bicyclische Liganden enthalten, und deren enantiomeren Formen,

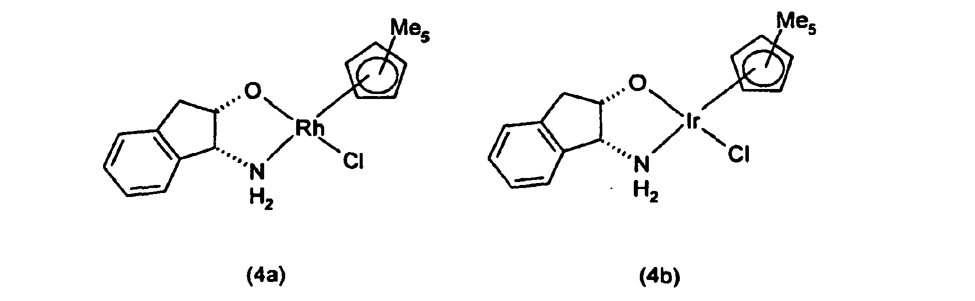

Die asymmetrische Hydrierung kann in der Regel durchgeführt werden, in dem man die Verbindung der Formel (II) in einem geeigneten Lösungsmittel in Gegenwart einer geeigneten Menge des gelösten Katalysators, gegebenenfalls unter Zusatz von Basen, hydriert. Gegebenenfalls kann der Katalysator auch in situ während der Reaktion aus den Komponenten erzeugt werden.

Die Hydrierung kann beispielsweise als asymmetrische Hydrierung unter Verwendung von Wasserstoffgas als Wasserstoffdonor oder unter Bedingungen der asymmetrischen Transferhydrierung mit anderen Wasserstoffdonoren erfolgen.

Das Mengenverhältnis von Verbindung (II) zu Katalysator kann in einem breiten Bereich variiert werden und liegt in der Regel im Bereich von 100000 Mol bis 10 Mol Verbindung (II) pro Mol des Katalysators, vorzugsweise 100000 Mol bis 50 Mol, insbesondere 10000 Mol bis 100 Mol Verbindung (II) pro Mol des Katalysators.

Die geeigneten Temperaturen für die erfindungsgemäße Umsetzung können in Vorversuchen bestimmt werden. Sie liegen in der Regel in einem Temperaturbereich von -80 °C bis zur Siedetemperatur des Gemischs, vorzugsweise bei Temperaturen von 0 °C bis 100 °C, insbesondere 15 °C bis 60 °C, ganz besonders bei Raumtemperatur. Die Umsetzung wird meist bei einem Wasserstoffdruck von 1 bis 100 bar, vorzugsweise 1 bar bis 10 bar durchgeführt.

Geeignete Lösungsmittel für die katalytische Hydrierung sind unter den Reaktionsbedingungen inerte Lösungsmittel, wie sie bei Hydrierungsreaktionen üblicherweise eingesetzt werden, oder deren Gemische, z.B.
- Alkanole wie Methanol, Ethanol, n-Propanol, Isopropanol, n-Butanol, Isobutanol, sec-Butanol, t-Butanol,
- Wasser,
- Carbonsäureester wie Essigsäureethylester,
- Carbonsäuren wie Eisessig,
- aromatische oder aliphatische Kohlenwasserstoffe wie Benzol, Toluol, Xylol und Paraffine, vorzugsweise Toluol, Xylol oder Mesitylen oder auch Gemische wie ®Solvesso (Mineralölgemisch mit aromatischen Anteilen),
- halogenierte aliphatische oder aromatische Kohlenwasserstoffe, z. B. chlorierte Alkane und Alkene, Chlorbenzol, o-Dichlorbenzol,
- Nitrile wie Acetonitril,
- Ether wie Diethylether oder cyclische Ether wie Dioxan oder Tetrahydrofuran,
- Amide wie Dimethylformamid,
Sulfone wie Sulfolan
und Gemische der genannten Lösungsmittel.

Als Variante der Hydrierung kann eine asymmetrische Transferhydrierung mit einem Wasserstoffdonor in Gegenwart der oben beschriebenen Katalysatoren durchgeführt werden. Als Wasserstoffdonor eignen sich Verbindungen, welche selbst unter den Bedingungen oxidiert werden können. Geeignete Wasserstoff-Donoren sind beispielsweise Ameisensäure und deren Salze, wie sie auch für die Durchführung der Leukart-Wallach-Reaktion verwendet werden (siehe beispielsweise Houben-Weyl 11/1, 648-664 und Synthesis 1988, 92 und dort zitierte Literatur). Ein geeigneter Wasserstoff-Donor ist damit Ameisensäure, die gegebenenfalls in Kombination mit einer Base, beispielsweise einer tertiären Aminbase eingesetzt werden kann. Basen sind beispielsweise Ammoniak oder primäre, sekundäre und tertiäre Amine, beispielsweise solche mit Alkyl, Aralkyl und/oder Arylresten, vorzugsweise entsprechende Amine mit (C₁-C₄)Alkylresten. Geeignete Salze sind entsprechende (substituierte) Ammoniumformiate.
Weitere Wasserstoffdonoren sind Isopropanol oder Cyclohexadien (vgl. Adv. Synth. Catal. 2003, Vol. 345, Seiten 67-77, Tetrahedron: Asymmetry 10 (1999) 2045-2061, Eur. J. Inorg. Chem. 2002, 2239-2251), auch jeweils in Kombination mit den in den Referenzen speziell genannten Katalysatoren.
Bevorzugt ist die Verwendung von Katalysatoren der obengenannten Formeln (3), (4) und (5) in Kombination mit Ameisensäure und tertiären Aminen wie Triethylamin. Geeignet sind dabei Mischungsverhältnisse von Ameisensäure:Amin von 10:1 1 bis 1:10 bezogen auf das Gewicht. Das Donor-System kann direkt als Lösungsmittel verwendet werden oder in Mischung mit anderen Lösungsmitteln als Co-Solventien eingesetzt werden.

Geeignete Lösungsmittel für die katalytische Transferhydrierung sind unter den Reaktionsbedingungen inerte Lösungsmittel oder deren Gemische, z.B.
- Alkanole wie Methanol, Ethanol, n-Propanol, Isopropanol, n-Butanol, Isobutanol, sec-Butanol, t-Butanol,
- Wasser,
- Carbonsäureester wie Essigsäureethylester,
- Carbonsäuren wie Eisessig,
- aromatische oder aliphatische Kohlenwasserstoffe wie Benzol, Toluol, Xylol und Paraffine, vorzugsweise Toluol, Xylol oder Mesitylen oder auch Gemische wie ®Solvesso (Mineralölgemisch mit aromatischen Anteilen),
- halogenierte aliphatische oder aromatische Kohlenwasserstoffe, z. B. chlorierte Alkane und Alkene, Chlorbenzol, o-Dichlorbenzol,
- Nitrile wie Acetonitril,
- Ether wie Diethylether oder cyclische Ether wie Dioxan oder Tetrahydrofuran,
- Amide wie Dimethylformamid,
   Sulfone wie Sulfolan
und Gemische der genannten Lösungsmittel.

Die katalytische Hydrierung und die katalytisch Transferhydrierung kann dabei analog den üblichen Bedingungen durchgeführt werden, wie sie in der genannten Literatur für die Reduktion von Ketonen zu optisch aktiven Alkoholen beschrieben ist.
Die Durchführung der Reaktion hinsichtlich Katalysatormenge und Reaktionszeit sollte in Vorversuchen optimiert werden, um eine hohe Konversionsrate, chemische Ausbeute und Enantiomerenüberschuss zu erreichen.

Überraschenderweise kann bei den Hydrierungen eine sehr gute Enantioselektivität des Amins (I) nicht nur hinsichtlich des Chiralitätszentrums, an dem die Aminogruppe gebunden ist, sondern auch hinsichtlich des benachbarten Chiralitätszentrums erreicht werden.

Die Ausgangsmaterialien der Formel (II) sind bekannt oder können analog bekannten Verfahren hergestellt werden. Eine einfache Möglichkeit ist die Herstellung über das entsprechende Keton. Cyclische Ketone sind vielfach kommerziell erhältlich oder durch eine große Zahl dem Fachmann bekannter Reaktionen aus anderen Verbindungen wie Alkoholen, Halogenverbindungen, oder aus Estern oder Carbonsäuren durch Ringschlussreaktionen herstellbar. Bicyclische Ketone sind beispielsweise auch beschrieben in WO 97/031904 und WO 2004/069814.

Eine besondere Möglichkeit zur Herstellung der optisch aktiven Amine (I) und deren Salze besteht auch in der Durchführung eines zweistufigen Verfahrens mit in-situ-Herstellung des Imins aus einem entsprechenden Keton und direkter Reduktion in Gegenwart des Katalysators. Das Imin entsteht dabei durch Reaktion des Ketons mit einer geeigneten Menge eines primären Amins R⁰-NH₂, wobei R⁰ wie in Formel (I) definiert ist, und wird ohne Zwischenisolierung zum Amin reduziert. Das in-situ-Verfahren kann beispielsweise mit einer Menge von 0,1 Mol bis zu einem mehrfachen Überschuss an primärem Amin pro Mol Keton, vorzugsweise 1 bis 10 Mol, insbesondere 1 bis 5 Mol, mehr bevorzugt 1 bis 2 Mol primäres Amin pro Mol Keton durchgeführt werden. Die zweistufige Reaktion lässt sich in der Regel durch Zugabe des optisch aktiven Katalysators kontrollieren. Die Temperaturbedingungen hängen im Wesentlichen von der geeigneten Temperatur für die katalytische Reaktion ab.

Die erhaltenen Verbindungen der Formel (I) eignen sich als optisch aktive Synthone zur Herstellung von optisch aktiven Wirkstoffen. Eine besonders häufige Verwendung der Verbindungen (I) und deren Salze ist die Herstellung der entsprechenden freien Amine (I'), wobei der Unterschied der Formel (I') zur Formel (I) im Ersatz des Restes R⁰ durch Wasserstoff besteht.

Die freien optisch aktiven cis-Amine der Formel (I') und deren Salze können aus geeigneten Verbindungen der Formel (I) nach Standardreaktionen hergestellt werden, beispielsweise durch Abspaltung von Schutzgruppen R⁰ für die eine Abspaltreaktion, vorzugsweise eine schonende Abspaltreaktion bekannt ist. Beispiele für derartige abspaltbare Gruppen sind die weiter oben genannten Gruppen i)-xv). Die Herstellung dieser Gruppen und deren gängigen und bevorzugten Abspaltungsmethoden sind beispielsweise in dem Handbuch "Protective Groups in Organic Synthesis, 3rd ed., T.W. Greene and P.G.M. Wuts; 1999, John Wiley & Sons, Inc. (siehe insbesondere Seiten 573 bis 585) und dort genannter Literatur beschrieben.

Die Versuche werden an folgenden Beispielen näher erläutert, ohne dass die Erfindung auf diese Ausführungsformen beschränkt sein soll. Mengenangaben beziehen sich auf das Gewicht, sofern nichts anderes angegeben ist.

Abkürzungen in den Beispielen:

| | |
|---|---|
| Me = | Methyl |
| Et = | Ethyl |
| n- oder i-Pr oder t-Butyl = | n-Propyl bzw. Isopropyl bzw. t-Butyl |
| Ph = | Phenyl |
| Ts = | p-Tosyl = p-Tolylsulfonyl |
| Allyl = | CH₂-CH=CH₂ |
| Bzl = | Benzyl = CH₂Ph |
| p-MeO-Bzl = | para-Methoxy-benzyl = (p-Methoxy-phenyl)-methyl |

### Beispiel A1

### cis-(S,S)-Benzyl-(2-methyl-1,2,3,4-tetrahydronaphth-1-yl)-amin

### A1a) Herstellung des Katalysators Ru(p-cymol)-(S,S)-TsOPEN

Bis-(Ruthenium-p-Cymol-dichlorid) werden in einem organischen Lösungsmittel vorgelegt und bei Raumtemperatur mit (S,S)-TsDPEN [(S,S)-N-Tosyl-1,2-Diamino-1,2-diphenylethan] umgesetzt. Nach Einengen und Abziehen des Lösungsmittels im Hochvakuum erhält man Ru(*p*-cymene)(S,S)-TsDPEN der Formel:

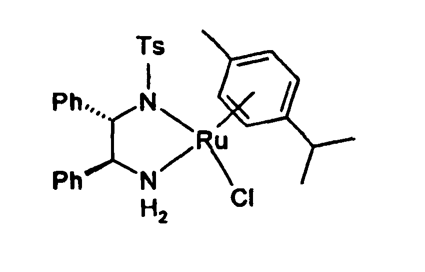

Entsprechend erhält man die Verbindung Ru(*p*-cymene)(R,R)-TsDPEN unter Verwendung von (*R*,*R*)-TsDPEN.

### A1b) (1S,2S)-N-Benzyl-(2-methyl-1,2,3,4-tetrahydronaphth-1-yl)amin

3 mg (4,7 µmol) des Katalysators aus Beispiel A1a werden in 1 ml einer Mischung aus Ameisensäure und Triethylamin (5:2 v/v) gelöst und 20 min gerührt. Dann fügt man eine Lösung von 350 mg (1,141 mmol) racemischem N-Benzyl-(2-methyl-3,4-dihydro-2H-naphth-1-yliden)amin in 1,7 ml Dichlormethan hinzu und rührt 5 Tage bei Zimmertemperatur. Nach Abziehen des Lösungsmittels, Wiederlösen in 5 ml Methanol, Behandlung mit ca. 200 mg Kaliumhydroxid und Versetzen mit 10 ml Wasser bei Zimmertemperatur wird dreimal mit jeweils 15 ml Dichlormethan extrahiert und die organische Phase über Natriumsulfat getrocknet. Nach Abziehen des Lösungsmittels und Säulenchromatographie (Hexan/Essigester 20:1) erhält man 229 mg (65% Ausbeute) (1S,2S)-N-Benzyl-(2-methyl-1,2,3,4-tetrahydronaphth-1-yl)amin als hellgelbes Öl.
[α]²¹D = -31,1° (c 0,8, CHCl₃): ¹H-NMR (400 MHz, C₆D₆) δ =6,98-7,22 (m, 9H), 3,75 (d, 1H, J = 14,0 Hz), 3,71 (d, 1H, J = 14,0 Hz), 3,47 (d, 1H, J = 3.6 Hz), 2,68 (dt, 1H, J = 17,2, 5,6 Hz), 2,55 (dt, 1H, J = 16,8, 8,0 Hz), 1,84 (m, 4H), 1,63 (m, 1H), 1,48 (m, 1H), 0,95 (d, 3H, J = 6,5 Hz), ¹³C-NMR (100 MHz, C₆D₆) δ = 141,8, 140,2, 136,5,129,5,129,2, 128,7, 128,6, 127,2, 127,0, 125,8, 259,3, 52,6, 32,5, 28,0, 26,5, 16,5. Der enantiomere Überschuss (enantiomeric excess, ee%) wurde chromatographisch bestimmt (ee = 98%); HPLC an Chiralpak AD (Daicel),
96:4 n-Hexan:Isopropanol, 30°C, Flussrate: 0,5 ml/min; RT: 7,07 min.

### Beispiel A2

### (1R,2S)-N-Allyl-(2-methylcyclohexyl)amin

Zu einer Mischung von 676 mg (6 mmol) 2-Methylcyclohexanon, 2,74 g (48 mmol) Allylamin und 100 mg Magnesiumsulfat wird eine Lösung von 22.5 mg (35 µmol) des Katalysators aus Beispiel A1a in 3,2 ml einer Mischung aus Ameisensäure und Triethylamin (5:2 v/v) tropfenweise zugegeben und gerührt: Nach 7 Tagen bei Zimmertemperatur wird die Reaktionsmischung mit 20 ml Wasser verdünnt und dreimal mit 15 ml Dichlormethan extrahiert. Die organische Phase wird über Natriumsulfat getrocknet Nach Abziehen des Lösungsmittels und Säulenchromatographie (Kieselgel, Dichlormethan/Methanol 10:1) erhält man 708 mg (77% Ausbeute) (1R,2S)-N-Allyl-(2-methylcyclohexyl)amin.
[α]²¹_{D} = +8,2° (c 0,7, CHCl₃); ¹H-NMR (300 MHz, CDCl₃) δ =5,88 (m, 1H), 5,14 (dd, 1H, J = 1,6, 16,2 Hz), 5,03 (m, 1H), 3,19 (m, 1H), 2,58 (m, 1H), 1,90 (m, 1H), 1,7-1,2 (m, 9H), 0,86 (d, 1H, J = 7,0 Hz),
¹³C-NMR (75 MHz) δ = 137,8, 115,8, 58,8, 49,9, 31,4, 28,4, 24,1, 22,2, 19,6, 13,8. Der enantiomere Überschuss (enantiomeric excess, ee%) wurde chromatographisch bestimmt (ee = 95,9%); HPLC an Chiralpak OJ (Daicel),
99,5:0,5 n-Hexan:Isopropanol, 30°C, Flussrate: 0,5 ml/min; RT: 17,16 min.

### Beispiel A3

### (1R,2R)-N-Benzyl-(2-phenylcyclohexyl)amin

Zu 3,2 ml einer Mischung aus Ameisensäure und Triethylamin (5:2 v/v) gibt man 16.6 mg IrCl[(S,S)-TsDPEN]Cp* und eine Lösung von racemischem 1-Benzyl-N-[2-phenylcyclohexyliden]amin in 10 ml trockenem Dichlormethan. Nach 24 h Rühren bei Raumtemperatur verdünnt man mit 0,5 M aq. Na₂CO₃ bis pH 10 erreicht ist und exrahiert dann mit-Dichlormethan (2 x 30 ml). Die organische Phase wird anschließen über Magnesiumsulfat getrocknet, im Vakuum eingeengt und der Rückstand durch Säulenchromatographie (Kieselgel, Essigsäureethylester/Hexan 1:12) gereinigt. Man erhält (1R,2R)-N-Benzyl-(2-phenylcyclohexyl)-amin in 60% iger Ausbeute. [α]²²_{D}= +33,4° (c 0,7, CHCl₃); ¹H-NMR (300 MHz, CDCl₃) δ =6.9 - 7.4 (m, 10H), 3.68 (d, J = 13.8 Hz, 1H), 3.0 (m, 1H), 2.85 (dt, J = 13.3, 3.2 Hz), 1H), 1.1 - 2.2 (m, 8H);
¹³C-NMR (75 MHz) δ = 143.9, 140.3, 127.7, 127.5, 127.1, 125.9, 125.5, 56.1, 50.7, 46.5, 29.2, 25.9, 24.1, 19.1. Die Bestimmung des Diastereomerenüberschusses (de) erfolgte über ¹H-NMR (de > 98%). Die Ermittlung des Enantiomerenüberschusses erfolgte nach Debenzylierung und nachfolgender Acetylierung der freigesetzten Aminofunktion (ee = 50%).

Entsprechend den Beispielen A1, A2 und A3 und den in der Beschreibung genannten Varianten werden auch die in den folgenden Tabellen 1 und 2 genannten Beispielverbindungen erhalten.

### Anmerkungen zu Tabellen 1 und 2:

Methoden zur Ermittlung der Retentionszeit durch HPLC an chiraler Phase. Alle Messungen mit n-Hexan/Isopropanol als Laufmittel bei 30 °C. Angabe der Referenzen wie folgt: Rₜ (min), Laufmittel und Flow (v_{nHex}/v_{iProH} - ml/min)
(1) Chiralpak AD (Daicel)
(2) Chiralpak OJ (Daicel)
(3) Chiralpak OB (Daicel)
(4) Charakterisiert als N-Benzoylderivat

Einige Daten zu den Verbindungen sind am Ende jeder Tabelle angegeben.

**Tabelle 1: Verbindungen der Formel (Ia-A) und (Ia-b)**

| 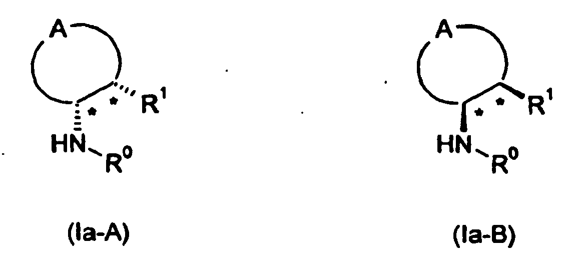 | | | | |
|---|---|---|---|---|
| Die Nummern in der ersten Spalte der Tabelle beziehen sich auf die Verbindungen der Formel (Ia-A) mit den Substituentenbedeutungen in den Spalten unter A, R⁰ und R¹. Entsprechend beziehen sich die Nummern in der zweiten Spalte auf die Verbindungen der Formel (Ia-B) mit den Substituentenbedeutungen in den Spalten unter A, R⁰ und R¹. | | | | |
| Verbind. (Ia-A) Nr. | Verbind. (Ia-B) Nr. | A | R⁰ | R¹ |
| aA1 | aB1 | CH₂ | Me | Me |
| aA2 | aB2 | CH₂CH₂ | Me | Me |
| aA3 | aB3 | CH₂CH₂CH₂ | Me | Me |
| aA4 | aB4 | CH₂CH₂CH₂CH₂ | Me | Me |
| aA5 | aB5 | CH₂CH₂CH₂CH₂CH₂ | Me | Me |
| aA6 | aB6 | CH₂O | Me | Me |
| aA7 | aB7 | CH₂OCH₂ | Me | Me |
| aA8 | aB8 | CH₂ | Me | Et |
| aA9 | aB9 | CH₂ | Me | n-Pr |
| aA10 | aB10 | CH₂CH₂ | Me | Et |
| aA11 | aB11 | CH₂CH₂ | Me | n-Pr |
| aA12 | aB12 | CH₂CH₂CH₂ | Me | Et |
| aA13 | aB13 | CH₂CH₂CH₂ | Me | n-Pr |
| aA14 | aB14 | CH₂CH₂CH₂CH₂ | Me | Et |
| aA15 | aB15 | CH₂CH₂CH₂CH₂ | Me | n-Pr |
| aA16 | aB16 | CH₂CH₂CH₂CH₂CH₂ | Me | Et |
| aA17 | aB17 | CH₂CH₂CH₂CH₂CH₂ | Me | n-Pr |
| aA18 | aB18 | CH₂O | Me | Et |
| aA19 | aB19 | CH₂O | Me | n-Pr |
| aA20 | aB20 | CH₂OCH₂ | Me | Et |
| aA21 | aB21 | CH₂OCH₂ | Me | n-Pr |
| aA22 | aB22 | CH₂ | Et | Me |
| aA23 | aB23 | CH₂CH₂ | Et | Me |
| aA24 | aB24 | CH₂CH₂CH₂ | Et | Me |
| aA25 | aB25 | CH₂CH₂CH₂CH₂ | Et | Me |
| aA26 | aB26 | CH₂CH₂CH=CH=CH= | Et | Me |
| aA27 | aB27 | CH₂O | Et | Me |
| aA28 | aB28 | CH₂OCH₂ | Et | Me |
| aA29 | aB29 | CH₂ | Et | Et |
| aA30 | aB30 | CH₂ | Et | n-Pr |
| aA31 | aB31 | CH₂CH₂ | Et | Et |
| aA32 | aB32 | CH₂CH₂ | Et. | n-Pr |
| aA33 | aB33 | CH₂CH₂CH₂ | Et | Et |
| aA34 | aB34 | CH₂CH₂CH₂ | Et | n-Pr |
| aA35 | aB35 | CH₂CH₂CH₂CH₂ | Et | Et |
| aA36 | aB36 | CH₂CH₂CH₂CH₂ | Et | n-Pr |
| aA37 | aB37 | CH₂CH₂CH₂CH₂CH₂ | Et | Et |
| aA38 | aB38 | CH₂CH₂CH₂CH₂CH₂ | Et | n-Pr |
| aA39 | aB39 | CH₂O | Et | Et |
| aA40 | aB40 | CH₂O | Et | n-Pr |
| aA41 | aB41 | CH₂OCH₂ | Et | Et |
| aA42 | aB42 | CH₂OCH₂ | Et | n-Pr |
| aA43 | aB43 | CH₂ | Allyl | Me |
| aA44 | aB44 | CH₂CH₂ | Allyl | Me |
| aA45 | aB45 | CH₂CH₂CH₂ | Allyl | Me |
| aA46 | aB46 | CH₂CH₂CH₂CH₂ | Allyl | Me |
| aA47 | aB47 | CH₂CH₂CH₂CH₂CH₂ | Allyl | Me |
| aA48 | aB48 | CH₂O | Allyl | Me |
| aA49 | aB49 | CH₂OCH₂ | Allyl | Me |
| aA50 | aB50 | CH₂ | Allyl | Et |
| aA51 | aB51 | CH₂ | Allyl | n-Pr |
| aA52 | aB52 | CH₂CH₂ | Allyl | Et |
| aA53 | aB53 | CH₂CH₂ | Allyl | n-Pr |
| aA54 | aB54 | CH₂CH₂CH₂ | Allyl | Et |
| aA55 | aB55 | CH₂CH₂CH₂ | Allyl | m-Pr |
| aA56 | aB56 | CH₂CH₂CH₂CH₂ | Allyl | Et |
| aA57 | aB57 | CH₂CH₂CH₂CH₂ | Allyl | n-Pr |
| aA58 | aB58 | CH₂CH₂CH₂CH₂CH₂ | Allyl | Et |
| aA59 | aB59 | CH₂CH₂CH₂CH₂CH₂ | Allyl | n-Pr |
| aA60 | aB60 | CH₂O | Allyl | Et |
| aA61 | aB61 | CH₂O | Allyl | n-Pr |
| aA62 | aB62 | CH₂OCH₂ | Allyl | Et |
| aA63 | aB63 | CH₂OCH₂ | Allyl | n-Pr |
| aA64 | aB64 | CH₂ | Bzl | Me |
| aA65 | aB65 | CH₂CH₂ | Bzl | Me |
| aA66 | aB66 | CH₂CH₂CH₂ | Bzl | Me |
| aA67 | aB67 | CH₂CH₂CH₂CH₂ | Bzl | Me |
| aA68 | aB68 | CH₂CH₂CH₂CH₂CH₂ | Bzl | Me |
| aA69 | aB69 | CH₂O | Bzl | Me |
| aA70 | aB70 | CH₂OCH₂ | Bzl | Me |
| aA71 | aB71 | CH₂ | Bzl | Et |
| aA72 | aB72 | CH₂ | Bzl | n-Pr |
| aA73 | aB73 | CH₂ | Bzl | Ph |
| aA74 | aB74 | CH₂CH₂ | Bzl | Et |
| aA75 | aB75 | CH₂CH₂ | Bzl | n-Pr |
| aA76 | aB76 | CH₂CH₂ | Bzl | Ph |
| aA77 | aB77 | CH₂CH₂CH₂ | Bzl | Et |
| aA78 | aB78 | CH₂CH₂CH₂ | Bzl | n-Pr |
| aA79 | a879 | CH₂CH₂CH₂ | Bzl | Ph |
| aA80 | aB80 | CH₂CH₂CH₂CH₂ | Bzl | Et |
| aA81 | aB81 | CH₂CH₂CH₂CH₂ | Bzl | n-Pr |
| aA82 | aB82 | CH₂CH₂CH₂CH₂ | Bzl | Ph |
| aA83 | aB83 | CH₂CH₂CH₂CH₂CH₂ | Bzl | Et |
| aA84 | aB84 | CH₂CH₂CH₂CH₂CH₂ | Bzl | n-Pr |
| aA85 | aB85 | CH₂CH₂CH₂CH₂CH₂ | Bzl | Ph |
| aA86 | aB88 | CH₂O | Bzl | Et |
| aA87 | aB87 | CH₂O | Bzl | n-Pr |
| aA88 | aB88 | CH₂O | Bzl | Ph |
| aA89 | aB89 | CH₂OCH₂ | Bzl | Et |
| aA90 | aB90 | CH₂OCH₂ | Bzl | n-Pr |
| aA91 | aB91 | CH₂OCH₂ | Bzl | Ph |

| | | | | |
|---|---|---|---|---|
| Physikalische Daten zu Verbindungen aus Tabelle 1: Verbindungs-Nr.: aB46: Ref (^{2,4}), Rₜ = 17,2, n-Hexan/Isopropanol = 99,5:0,5 (v/v), Flow = 0,5 ml / min | | | | |

**Tabelle 2: Verbindungen der Formel (Ib-A) und (Ib-B)**

| 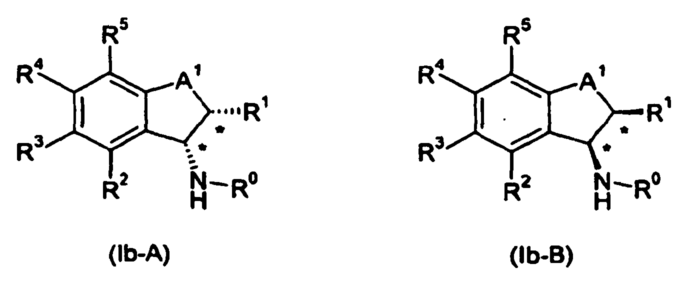 | | | | | | | | |
|---|---|---|---|---|---|---|---|---|
| Die Nummern in der ersten Spalte der Tabelle beziehen sich auf die Verbindungen der Formel (Ib-A) mit den Substituentenbedeutungen in den Spalten unter A, R⁰ bis R⁵. Entsprechend beziehen sich die Nummern in der zweiten Spalte auf die Verbindungen der Formel (1b-B) mit den Substituentenbedeutungen in den Spalten unter A und R⁰ bis R⁵. | | | | | | | | |
| lb-A | Ib-B | A¹ | R⁰ | R¹ | R² | R³ | R⁴ | R⁵ |
| bA1 | bB1 | - | Me | Me | H | H | H | H |
| bA2 | bB2 | - | Me | Me | Me | H | H | H |
| bA3 | bB3 | - | Me | Me | H | Me | H | H |
| bA4 | bB4 | - | Me | Me | H | H | Me | H |
| bA5 | bB5 | - | Me | Me | H | H | H | Me |
| bA6 | bB6 | - | Me | Me | Cl | H | H | H |
| bA7 | bB7 | - | Me | Me | H | Cl | H | H |
| bA8 | bB8 | - | Me | Me | H | H | Cl | H |
| bA9 | bB9 | - | Me | Me | H | H | H | Cl |
| bA10 | bB10 | - | Me | Me | Et | H | H | H |
| bA11 | bB11 | - | Me | Me | H | Et | H | H |
| bA12 | bB12 | - | Me | Me | H | H | Et | H |
| bA13 | bB13 | - | Me | Me | H | H | H | Et |
| bA14 | bB14 | | Me | Me | OMe | H | H | H |
| bA15 | bB15 | - | Me | Me | H | OMe | H | H |
| bA16 | bB16 | - | Me | Me | H | H | OMe | H |
| bA17 | bB17 | - | Me | Me | H | H | H | OMe |
| bA18 | bB18 | - | Me | Me | F | H | H | H |
| bA19 | bB19 | - | Me | Me | H | F | H | H |
| bA20 | bB20 | - | Me | Me | H | H | F | H |
| bA21 | bB21 | - | Me | Me | H | H | H | F |
| bA22 | bB22 | - | Et | Me | H | H | H | H |
| bA23 | bB23 | - | Et | Me | Me | H | H | H |
| bA24 | bB24 | - | Et | Me | H | Me | H | H |
| bA25 | bB25 | - | Et | Me | H | H | Me | H |
| bA26 | bB26 | - | Et | Me | H | H | H | Me |
| bA27 | bB27 | - | Et | Me | Cl | H | H | H |
| bA28 | bB28 | - | Et | Me | H | Cl | H | H |
| bA29 | bB29 | - | Et | Me | H | H | Cl | H |
| bA30 | bB30 | - | Et | Me | H | H | H | Cl |
| bA31 | bB31 | - | Et | Me | Et | H | H | H |
| bA32 | bB32 | - | Et | Me | H | Et | H | H |
| bA33 | bB33 | - | Et | Me | H | H | Et | H |
| bA34 | bB34 | - | Et | Me | H | H | H | Et |
| bA35 | bB35 | - | Et | Me | OMe | H | H | H |
| bA36 | bB36 | - | Et | Me | H | OMe | H | H |
| bA37 | bB37 | - | Et | Me | H | H | OMe | H |
| bA38 | bB38 | - | Et | Me | H | H | H | OMe |
| bA39 | bB39 | - | Et | Me | F | H | H | H |
| bA40 | bB40 | - | Et | Me | H | F | H | H |
| bA41 | bB41 | - | Et | Me | H | H | F | H |
| bA42 | bB42 | - | Et | Me | H | H | H | F |
| bA43 | bB43 | - | Allyl | Me | H | H | H | H |
| bA44 | bB44 | - | Allyl | Me | Me | H | H | H |
| bA45 | bB45 | - | Allyl | Me | H | Me | H | H |
| bA46 | bB46 | - | Allyl | Me | H | H | Me | H |
| bA47 | bB47 | - | Allyl | Me | H | H | H | Me |
| bA48 | bB48 | - | Allyl | Me | Cl | H | H | H |
| bA49 | bB49 | - | Allyl | Me | H | Cl | H | H |
| bA50 | bB50 | - | Allyl | Me | H | H | Cl | H |
| bA51 | bB51 | - | Allyl | Me | H | H | H | Cl |
| bA52 | bB52 | - | Allyl | Me | Et | H | H | H |
| bA53 | bB53 | - | Allyl | Me | H | Et | H | H |
| bA54 | bB54 | - | Allyl | Me | H | H | Et | H |
| bA55 | bB55 | - | Allyl | Me | H | H | H | Et |
| bA56 | bB56 | - | Allyl | Me | OMe | H | H | H |
| bA57 | bB57 | - | Allyl | Me | H | OMe | H | H |
| bA58 | bB58 | - | Allyl | Me | H | H | OMe | H |
| bA59 | bB59 | - | Allyl | Me | H | H | H | OMe |
| bA60 | bB60 | - | Allyl | Me | F | H | H | H |
| bA61 | bB61 | - | Allyl | Me | H | F | H | H |
| bA62 | bB62 | - | Allyl | Me | H | H | F | H |
| bA63 | bB63 | - | Allyl | Me | H | H | H | F |
| bA64 | bB64 | - | Bzl | Me | H | H | H | H |
| bA65 | bB65 | - | Bzl | Me | Me | H | H | H |
| bA66 | bB66 | - | Bzl | Me | H | Me | H | H |
| bA67 | bB67 | - | Bzl | Me | H | H | Me | H |
| bA68 | bB68 | - | Bzl | Me | H | H | H | Me |
| bA69 | bB69 | - | Bzl | Me | Cl | H | H | H |
| bA70 | bB70 | - | Bzl | Me | H | Cl | H | H |
| bA71 | bB71 | - | Bzl | Me | H | H | Cl | H |
| bA72 | bB72 | - | Bzl | Me | H | H | H | Cl |
| bA73 | bB73 | - | Bzl | Me | Et | H | H | H |
| bA74 | bB74 | - | Bzl | Me | H | Et | H | H |
| bA75 | bB75 | - | Bzl | Me | H | H | Et | H |
| bA76 | bB76 | - | Bzl | Me | H | H | H | Et |
| bA77 | bB77 | - | Bzl | Me | OMe | H | H | H |
| bA78 | bB78 | - | Bzl | Me | H | OMe | H | H |
| bA79 | bB79 | - | Bzl | Me | H | H | OMe | H |
| bA80 | bB80 | - | Bzl | Me | H | H | H | OMe |
| bA81 | bB81 | - | Bzl | Me | F | H | H | H |
| bA82 | bB82 | - | Bzl | Me | H | F | H | H |
| bA83 | bB83 | - | Bzl | Me | H | H | F | H |
| bA84 | bB84 | - | Bzl | Me | H | H | H | F |
| bA85 | bB85 | - | p-MeO-Bzl | Me | H | H | H | H |
| bA86 | bB86 | - | p-MeO-Bzl | Me | Me | H | H | H |
| bA87 | bB87 | - | p-MeO-Bzl | Me | H | Me | H | H |
| bA88 | bB88 | - | p-MeO-Bzl | Me | H | H | Me | H |
| bA89 | bB89 | - | p-MeO-Bzl | Me | H | H | H | Me |
| bA90 | bB90 | - | p-MeO-Bzl | Me | Cl | H | H | H |
| bA91 | bB91 | - | p-MeO-Bzl | Me | H | Cl | H | H |
| bA92 | bB92 | - | P-MeO-Bzl | Me | H | H | Cl | H |
| bA93 | bB93 | - | p-MeO-Bzl | Me | H | H | H | Cl |
| bA94 | bB94 | - | p-MeO-Bzl- | Me | Et | H | H | H |
| bA95 | bB95 | - | p-MeO-Bzl | Me | H | Et | H | H |
| bA96 | bB96 | - | p-MeO-Bzl | Me | H | H | Et | H |
| bA97 | bB97 | - | p-MeO-Bzl | Me | H | H | H | Et |
| bA98 | bB98 | - | p-MeO-Bzl | Me | OMe | H | H | H |
| bA99 | bB99 | - | p-MeO-Bzl | Me | H | OMe | H | H |
| bA100 | bB100 | - | p-MeO-Bzl | Me | H | H | OMe | H |
| bA101 | bB101 | - | p-MeO-Bzl | Me | H | H | H | OMe |
| bA102 | bB102 | - | p-MeO-Bzl | Me | F | H | H | H |
| bA103 | bB103 | - | p-MeO-Bzl | Me | H | F | H | H |
| bA104 | bB104 | - | p-MeO-Bzl | Me | H | H | F | H |
| bA105 | bB105 | - | p-MeO-Bzl | Me | H | H | H | F |
| bA106 | bB106 | - | i-Pr | Me | Me | H | H | F |
| bA107 | b8107 | - | i-Pr | Me | H | Me | H | H |
| bA108 | bB108 | - | i-Pr | Me | H | H | Me | H |
| bA109 | bB109 | - | i-Pr | Me | H | H | H | Me |
| bA110 | bB110 | - | i-Pr | Me | Cl | H | H | H |
| bA111 | bB111 | - | i-Pr | Me | H | Cl | H | H |
| bA112 | bB112 | - | i-Pr | Me | H | H | Cl | H |
| bA113 | bB113 | - | i-Pr | Me | H | H | H | Cl |
| bA114 | bB114 | - | i-Pr | Me | Et | H | H | H |
| bA115 | bB115 | - | i-Pr | Me | H | Et | H | H |
| bA116 | bB116 | - | i-Pr | Me | H | H | Et | H |
| bA117 | bB117 | - | i-Pr | Me | H | H | H | Et |
| bA118 | bB118 | - | i-Pr | Me | OMe | H | H | H |
| bA119 | bB119 | - | i-Pr | Me | H | OMe | H | H |
| bA120 | bB120 | - | i-Pr | Me | H | H | OMe | H |
| bA121 | bB121 | - | i-Pr | Me | H | H | H | OMe |
| bA122 | bB122 | - | i-Pr | Me | F | H | H | H |
| bA123 | bB123 | - | i-Pr | Me | H | F | H | H |
| bA124 | bB124 | - | i-Pr | Me | H | H | F | H |
| bA125 | bB125 | - | i-Pr | Me | H | H | H | F |
| bA126 | bB126 | - | t-Bu | Me | H | H | H | H |
| bA127 | bB127 | - | t-Bu | Me | Me | H | H | H |
| bA128 | bB128 | - | t-Bu | Me | Me | Me | H | H |
| bA129 | bB129 | - | t-Bu | Me | H | H | Me | H |
| bA130 | bB130 | - | t-Bu | Me | H | H | H | Me |
| bA131 | bB131 | - | t-Bu | Me | Cl | H | H | H |
| bA132 | bB132 | - | t-Bu | Me | H | Cl | H | H |
| bA133 | bB133 | - | t-Bu | Me | H | H | Cl | H |
| bA134 | bB134 | - | t-Bu | Me | H | H | H | Cl |
| bA135 | bB135 | - | t-Bu | Me | H | H | H | H |
| bA136 | bB136 | - | t-Bu | Me | Et | H | H | H |
| bA137 | bB137 | - | t-Bu | Me | H | Et | H | H |
| bA138 | bB138 | - | t-Bu | Me | H | H | Et | H |
| bA139 | bB139 | - | t-Bu | Me | H | H | H | Et |
| bA140 | bB140 | - | t-Bu | Me | OMe | H | H | H |
| bA141 | bB141 | - | t-Bu | Me | H | OMe | H | H |
| bA142 | bB142 | - | t-Bu | Me | H | H | OMe | H |
| bA143 | bB143 | - | t-Bu | Me | H | H | H | OMe |
| bA144 | bB144 | - | t-Bu | Me | F | H | H | H |
| bA145 | bB145 | - | t-Bu | Me | H | F | H | H |
| bA146 | bB146 | - | t-Bu | Me | H | H | F | H |
| bA147 | bB147 | - | t-Bu | Me | H | H | H | F |
| bA148 | bB148 | - | Ph | Me | H | H | H | H |
| bA149 | bB149 | - | Ph | Me | Me | H | H | H |
| bA150 | bB150 | - | Ph | Me | Me | Me | H | H |
| bA151 | bB151 | - | Ph | Me | H | H | Me | H |
| bA152 | bB152 | - | Ph | Me | H | H | H | Me |
| bA153 | bB153 | - | Ph | Me | Cl | H | H | H |
| bA154 | bB154 | - | Ph | Me | H | Cl | H | H |
| bA155 | bB155 | - | Ph | Me | H | H | Cl | H |
| bA156 | bB156 | - | Ph | Me | H | H | H | Cl |
| bA157 | bB157 | - | Ph | Me | H | H | H | H |
| bA158 | bB158 | - | Ph | Me | Et | H | H | H |
| bA159 | bB159 | - | Ph | Me | H | Et | H | H |
| bA160 | bB160 | - | Ph | Me | H | H | Et | H |
| bA161 | bB161 | - | Ph | Me | H | H | H | Et |
| bA162 | bB162 | - | Ph | Me | OMe | H | H | H |
| bA163 | bB163 | - | Ph | Me | H | OMe | H | H |
| bA164 | bB164 | - | Ph | Me | H | H | OMe | H |
| bA165 | bB165 | - | Ph | Me | H | H | H | OMe |
| bA166 | bB166 | - | Ph | Me | F | H | H | H |
| bA167 | bB167 | - | Ph | Me | H | F | H | H |
| bA168 | bB168 | - | Ph | Me | H | H | F | H |
| bA169 | bB169 | - | Ph | Me | H | H | H | F |
| bA170 | bB170 | CH₂ | Me | Me | H | H | H | H |
| bA171 | bB171 | CH₂ | Me | Me | Me | H | H | H |
| bA172 | bB172 | CH₂ | Me | Me | H | Me | H | H |
| bA173 | bB173 | CH₂ | Me | Me | H | H | Me | H |
| bA174 | bB174 | CH₂ | Me | Me | H | H | H | Me |
| bA175 | bB175 | CH₂ | Me | Me | Cl | H | H | H |
| bA176 | bB176 | CH₂ | Me | Me | H | Cl | H | H |
| bA177 | bB177 | CH₂ | Me | Me | H | H | Cl | H |
| bA178 | bB178 | CH₂ | Me | Me | H | H | H | Cl |
| bA179 | bB179 | CH₂ | Me | Me | Et | H | H | H |
| bA180 | bB180 | CH₂ | Me | Me | H | Et | H | H |
| bA181 | bB181 | CH₂ | Me | Me | H | H | Et | H |
| bA182 | bB182 | CH₂ | Me | Me | H | H | H | Et |
| bA183 | bB183 | CH₂ | Me | Me | OMe | H | H | H |
| bA184 | bB184 | CH₂ | Me | Me | H | OMe | H | H |
| bA185 | bB185 | CH₂ | Me | Me | H | H | OMe | H |
| bA186 | bB186 | CH₂ | Me | Me | H | H | H | OMe |
| bA187 | bB187 | CH₂ | Me | Me | F | H | H | H |
| bA188 | bB188 | CH₂ | Me | Me | H | F | H | H |
| bA189 | bB189 | CH₂ | Me | Me | H | H | F | H |
| bA190 | bB190 | CH₂ | Me | Me | H | H | H | F |
| bA191 | bB191 | CH₂ | Et | Me | H | H | H | H |
| bA192 | bB192 | CH₂ | Et | Me | Me | H | H | H |
| bA193 | bB193 | CH₂ | Et | Me | H | Me | H | H |
| bA194 | bB194 | CH₂ | Et | Me | H | H | Me | H |
| bA195 | bB195 | CH₂ | Et | Me | H | H | H | Me |
| bA196 | bB196 | CH₂ | Et | Me | Cl | H | H | H |
| bA197 | bB197 | CH₂ | Et | Me | H | Cl | H | H |
| bA198 | bB198 | CH₂ | Et | Me | H | H | Cl | H |
| bA199 | bB199 | CH₂ | Et | Me | H | H | H | Cl |
| bA200 | bB200 | CH₂ | Et | Me | Et | H | H | H |
| bA201 | bB201 | CH₂ | Et | Me | H | Et | H | H |
| bA202 | bB202 | CH₂ | Et | Me | H | H | Et | H |
| bA203 | bB203 | CH₂ | Et | Me | H | H | H | Et |
| bA204 | bB204 | CH₂ | Et | Me | OMe | H | H | H |
| bA205 | bB205 | CH₂ | Et | Me | H | OMe | H | H |
| bA206 | bB206 | CH₂ | Et | Me | H | H | OMe | H |
| bA207 | bB207 | CH₂ | Et | Me | H | H | H | OMe |
| bA208 | bB208 | CH₂ | Et | Me | F | H | H | H |
| bA209 | bB209 | CH₂ | Et | Me | H | F | H | H |
| bA210 | bB210 | CH₂ | Et | Me | H | H | F | H |
| bA211 | bB211 | CH₂ | Et | Me | H | H | H | F |
| bA212 | bB212 | CH₂ | Allyl | Me | H | H | H | H |
| bA213 | bB213 | CH₂ | Allyl | Me | Me | H | H | H |
| bA214 | bB214 | CH₂ | Allyl | Me | H | Met | H | H |
| bA215 | bB215 | CH₂ | Allyl | Me | H | H | Me | H |
| bA216 | bB216 | CH₂ | Allyl | Me | H | H | H | Me |
| bA217 | b8217 | CH₂ | Allyl | Allyl | Me | H | H | H |
| bA218 | bB218 | CH₂ | Alkyl | Allyl | H | Me | H | H |
| bA219 | bB219 | CH₂ | Allyl | Allyl | H | H | Me | H |
| bA220 | bB220 | CH₂ | Allyl | Allyl | H | H | H | Me |
| bA221 | bB221 | CH₂ | Allyl | Me | Cl | H | H | H |
| bA222 | bB222 | CH₂ | Allyl | Me | H | Cl | H | H |
| bA223 | bB223 | CH₂ | Allyl | Me | H | H | Cl | H |
| bA224 | bB224 | CH₂ | Allyl | Me | H | H | H | Cl |
| bA225 | bB225 | CH₂ | Allyl | Me | Et | H | H | H |
| bA226 | bB226 | CH₂ | Allyl | Me | H | Et | H | H |
| bA227 | bB227 | CH₂ | Allyl | Me | H | H | Et | H |
| bA228 | bB228 | CH₂ | Allyl | Me | H | H | H | Et |
| bA229 | bB229 | CH₂ | Allyl | Me | OMe | H | H | H |
| bA230 | bB230 | CH₂ | Allyl | Me | H | OMe | H | H |
| bA231 | bB231 | CH₂ | Allyl | Me | H | H | OMe | H |
| bA232 | bB232 | CH₂ | Allyl | Me | H | H | H | OMe |
| bA233 | bB233 | CH₂ | Allyl | Me | F | H | H | H |
| bA234 | bB234 | CH₂ | Allyl | Me | H | F | H | H |
| bA235 | bB235 | CH₂ | Allyl | Me | H | H | F | H |
| bA236 | bB236 | CH₂ | Allyl | Me | H | H | H | F |
| bA237 | bB237 | CH₂ | Bzl | Me | H | H | H | H |
| bA238 | bB238 | CH₂ | Bzl | Me | Me | H | H | H |
| bA239 | bB239 | CH₂ | Bzl | Me | H | Me | H | H |
| bA240 | bB240 | CH₂ | Bzl | Me | H | H | Me | H |
| bA241 | bB241 | CH₂ | Bzl | Me | H | H | H | Me |
| bA242 | bB242 | CH₂ | Bzl | Me | Cl | H | H | H |
| bA243 | bB243 | CH₂ | Bzl | Me | H | Cl | H | H |
| bA244 | bB244 | CH₂ | Bzl | Me | H | H | Cl | H |
| bA245 | bB245 | CH₂ | Bzl | Me | H | H | H | Cl |
| bA246 | bB246 | CH₂ | Bzl | Me | Et | H | H | H |
| bA247 | bB247 | CH₂ | Bzl | Me | H | Et | H | H |
| bA248 | bB248 | CH₂ | Bzl | Me | H | H | Et | H |
| bA249 | bB249 | CH₂ | Bzl | Me | H | H | H | Et |
| bA250 | bB250 | CH₂ | Bzl | Me | OMe | H | H | H |
| bA251 | bB251 | CH₂ | Bzl | Me | H | OMe | H | H |
| bA252 | bB252 | CH₂ | Bzl | Me | H | H | OMe | H |
| bA253 | bB253 | CH₂ | Bzl | Me | H | H | H | OMe |
| bA254 | bB254 | CH₂ | Bzl | Me | F | H | H | H |
| bA255 | bB255 | CH₂ | Bzl | Me | H | F | H | H |
| bA256 | bB256 | CH₂ | Bzl | Me | H | H | F | H |
| bA257 | bB257 | CH₂ | Bzl | Me | H | H | H | F |
| bA258 | bB258 | CH₂ | p-MeO-Bzl | Me | H | H | H | H |
| bA259 | bB259 | CH₂ | p-MeO-Bzl | Me | Me | H | H | H |
| bA260 | bB260 | CH₂ | p-MeO-Bzl | Me | H | Me | H | H |
| bA261 | bB261 | CH₂ | p-MeO-Bzl | Me | H | H | Me | H |
| bA262 | bB262 | CH₂ | p-MeO-Bzl | Me | H | H | H | Me |
| bA263 | bB263 | CH₂ | p-MeO-Bzl | Me | Cl | H | H | H |
| bA264 | bB264 | CH₂ | p-MeO-Bzl | Me | H | Cl | H | H |
| bA265 | bB265 | CH₂ | p-MeO-Bzl | Me | H | H | Cl | H |
| bA266 | bB266 | CH₂ | p-MeO-Bzl | Me | H | H | H | Cl |
| bA267 | bB267 | CH₂ | p-MeO-Bzl | Me | Et | H | H | H |
| bA268 | bB26B | CH₂ | p-MeO-Bzl | Me | H | Et | H | H |
| bA269 | bB269 | CH₂ | p-MeO-Bzi | Me | H | H | Et | H |
| bA270 | bB270 | CH₂ | p-MeO-Bzl | Me | H | H | H | Et |
| bA271 | bB271 | CH₂ | p-MeO-Bzl | Me | OMe | H | H | H |
| bA272 | bB272 | CH₂ | p-MeO-Bzl | Me | H | OMe | H | H |
| bA273 | bB273 | CH₂ | p-MeO-Bzl | Me | H | H | OMe | H |
| bA274 | bB274 | CH₂ | p-MeO-Bzl | Me | H | H | H | OMe |
| bA275 | bB275 | CH₂ | p-MeO-Bzl | Me | F | H | H | H |
| bA276 | bB276 | CH₂ | p-MeO-Bzl | Me | H | F | H | H |
| bA277 | bB277 | CH₂ | p-MeO-Bzl | Me | H | H | F | H |
| bA278 | bB278 | CH₂ | p-MeO-Bzl | Me | H | H | H | F |
| bA279 | bB279 | CH₂ | i-Pr | Me | Me | H | H | F |
| bA280 | bB280 | CH₂ | i-Pr | Me | H | Me | H | H |
| bA281 | bB281 | CH₂ | i-Pr | Me | H | H | Me | H |
| bA282 | bB282 | CH₂ | i-Pr | Me | H | H | H | Me |
| bA283 | bB283 | CH₂ | i-Pr | Me | Cl | H | H | H |
| bA284 | bB284 | CH₂ | i-Pr | Me | H | Cl | H | H |
| bA285 | bB285 | CH₂ | i-Pr | Me | H | H | Cl | H |
| bA286 | bB286 | CH₂ | i-Pr | Me | H | H | H | Cl |
| bA287 | bB287 | CH₂ | i-Pr | Me | Et | H | H | H |
| bA288 | bB288 | CH₂ | i-Pr | Me | H | Et | H | H |
| bA289 | bB289 | CH₂ | i-Pr | Me | H | H | Et | H |
| bA290 | b8290 | CH₂ | i-Pr | Me | H | H | H | Et |
| bA291 | bB291 | CH₂ | i-Pr | Me | OMe | H | H | H |
| bA292 | bB292 | CH₂ | i-Pr | Me | H | OMe | H | H |
| bA293 | bB293 | CH₂ | i-Pr | Me | H | H | OMe | H |
| bA294 | bB294 | CH₂ | i-Pr | Me | H | H | H | OMe |
| bA295 | bB295 | CH₂ | i-Pr | Me | F | H | H | H |
| bA296 | bB296 | CH₂ | i-Pr | Me | H | F | H | H |
| bA297 | bB297 | CH₂ | i-Pr | Me | H | H | F | H |
| bA298 | bB298 | CH₂ | i-Pr | Me | H | H | H | F |
| bA299 | bB299 | CH₂ | t-Bu | Me | H | H | H | H |
| bA300 | bB300 | CH₂ | t-Bu | Me | Me | H | H | H |
| bA301 | bB301 | CH₂ | t-Bu | Me | M | Me | H | H |
| bA302 | bB302 | CH₂ | t-Bu | Me | H | H | Me | H |
| bA303 | bB303 | CH₂ | t-Bu | Me | H | H | H | Me |
| bA304 | bB304 | CH₂ | t-Bu | Me | Cl | H | H | H |
| bA305 | bB305 | CH₂ | t-Bu | Me | H | Cl | H | H |
| bA306 | bB306 | CH₂ | t-Bu | Me | H | H | Cl | H |
| bA307 | bB307 | CH₂ | t-Bu | Me | H | H | H | Cl |
| bA308 | bB308 | CH₂ | t-Bu | Me | H | H | H | H |
| bA309 | bB309 | CH₂ | t-Bu | Me | Et | H | H | H |
| bA310 | bB310 | CH₂ | t-Bu | Me | H | Et | H | H |
| bA311 | bB311 | CH₂ | t-Bu | Me | H | H | Et | H |
| bA312 | bB312 | CH₂ | t-Bu | Me | H | H | H | Et |
| bA313 | bB313 | CH₂ | t-Bu | Me | OMe | H | H | H |
| bA314 | bB314 | CH₂ | t-Bu | Me | H | OMe | H | H |
| bA315 | bB315 | CH₂ | t-Bu | Me | H | H | OMe | H |
| bA316 | bB316 | CH₂ | t-Bu | Me | H | H | H | OMe |
| bA317 | bB317 | CH₂ | t-Bu | Me | F | H | H | H |
| bA318 | bB318 | CH₂ | t-Bu | Me | H | F | H | H |
| bA319 | bB319 | CH₂ | t-Bu | Me | H | H | F | H |
| bA320 | bB320 | CH₂ | t-Bu | Me | H | H | H | F |
| bA321 | bB321 | CH₂ | Ph | Me | H | H | H | H |
| bA322 | bB322 | CH₂ | Ph | Me | Me | H | H | H |
| bA323 | bB323 | CH₂ | Ph | Me | Me | Me | H | H |
| bA324 | bB324 | CH₂ | Ph | Me | H | H | Me | H |
| bA325 | bB325 | CH₂ | Ph | Me | H | H | H | Me |
| bA326 | bB326 | CH₂ | Ph | Me | Cl | H | H | H |
| bA327 | bB327 | CH₂ | Ph | Me | H | Cl | H | H |
| bA328 | bB328 | CH₂ | Ph | Me | H | H | Cl | H |
| bA329 | bB329 | CH₂ | Ph | Me | H | H | H | Cl |
| bA330 | bB330 | CH₂ | Ph | Me | H | H | H | H |
| bA331 | bB331 | CH₂ | Ph | Me | Et | H | H | H |
| bA332 | bB332 | CH₂ | Ph | Me | H | Et | H | H |
| bA333 | bB333 | CH₂ | Ph | Me | H | H | Et | H |
| bA334 | bB334 | CH₂ | Ph | Me | H | H | H | Et |
| bA335 | bB335 | CH₂ | Ph | Me | OMe | H | H | H |
| bA336 | bB336 | CH₂ | Ph | Me | H | OMe | H | H |
| bA337 | bB337 | CH₂ | Ph | Me | H | H | OMe | H |
| bA338 | bB338 | CH₂ | Ph | Me | H | H | H | OMe |
| bA339 | bB339 | CH₂ | Ph | Me | F | H | H | H |
| bA340 | bB340 | CH₂ | Ph | Me | H | F | H | H |
| bA341 | bB341 | CH₂ | Ph | Me | H | H | F | H |
| bA342 | bB342 | CH₂ | Ph | Me | H | H | H | F |
| bA343 | bB343 | CH₂CH₂ | Me | Me | H | H | H | H |
| bA344 | bB344 | CH₂CH₂ | Me | Me | Me | H | H | H |
| bA345 | bB345 | CH₂CH₂ | Me | Me | H | Me | H | H |
| bA346 | bB346 | CH₂CH₂ | Me | Me | H | H | Me | H |
| bA347 | bB347 | CH₂CH₂ | Me | Me | H | H | H | Me |
| bA348 | bB348 | CH₂CH₂ | Me | Me | Cl | H | H | H |
| bA349 | bB349 | CH₂CH₂ | Me | Me | H | Cl | H | H |
| bA350 | bB350 | CH₂CH₂ | Me | Me | H | H | Cl | H |
| bA351 | bB351 | CH₂CH₂ | Me | Me | H | H | H | Cl |
| bA352 | bB352 | CH₂CH₂ | Me | Me | Et | H | H | H |
| bA353 | bB353 | CH₂CH₂ | Me | Me | H | Et | H | H |
| bA354 | bB354 | CH₂CH₂ | Me | Me | H | H | Et | H |
| bA355 | bB355 | CH₂CH₂ | Me | Me | H | H | H | Et |
| bA356 | bB356 | CH₂CH₂ | Me | Me | OMe | H | H | H |
| bA357 | bB357 | CH₂CH₂ | Me | Me | H | OMe | H | H |
| bA358 | bB358 | CH₂CH₂ | Me | Me | H | H | OMe | H |
| bA359 | bB359 | CH₂CH₂ | Me | Me | H | H | H | OMe |
| bA360 | bB360 | CH₂CH₂ | Me | Me | F | H | H | H |
| bA361 | bB361 | CH₂CH₂ | Me | Me | H | F | H | H |
| bA362 | bB362 | CH₂CH₂ | Me | Me | H | H | F | H |
| bA363 | bB363 | CH₂CH₂ | Me | Me | H | H | H | F |
| bA364 | bB364 | CH₂CH₂ | Et | Me | H | H | H | H |
| bA365 | bB365 | CH₂CH₂ | Et | Me | Me | H | H | H |
| bA366 | bB366 | CH₂CH₂ | Et | Me | H | Me | H | H |
| bA367 | bB367 | CH₂CH₂ | Et | Me | H | H | Me | H |
| bA368 | bB368 | CH₂CH₂ | Et | Me | H | H | H | Me |
| bA369 | bB369 | CH₂CH₂ | Et | Me | Cl | H | H | H |
| bA370 | bB370 | CH₂CH₂ | Et | Me | H | Cl | H | H |
| bA371 | bB371 | CH₂CH₂ | Et | Me | H | H | Cl | H |
| bA372 | bB372 | CH₂CH₂ | Et | Me | H | H | H | Cl |
| bA373 | bB373 | CH₂CH₂ | Et | Me | Et | H | H | H |
| bA374 | bB374 | CH₂CH₂ | Et | Me | H | Et | H | H |
| bA375 | bB375 | CH₂CH₂ | Et | Me | H | H | Et | H |
| bA376 | bB376 | CH₂CH₂ | Et | Me | H | H | H | Et |
| bA377 | bB377 | CH₂CH₂ | Et | Me | OMe | H | H | H |
| bA378 | bB378 | CH₂CH₂ | Et | Me | H | OMe | H | H |
| bA379 | bB379 | CH₂CH₂ | Et | Me | H | H | OMe | H |
| bA380 | bB380 | CH₂CH₂ | Et | Me | H | H | H | OMe |
| bA381 | bB381 | CH₂CH₂ | Et | Me | F | H | H | H |
| bA382 | bB382 | CH₂CH₂ | Et | Me | H | F | H | H |
| bA383 | bB383 | CH₂CH₂ | Et | Me | H | H | F | H |
| bA384 | bB384 | CH₂CH₂ | Et | Me | H | H | H | F |
| bA385 | bB385 | CH₂CH₂ | Allyl | Me | H | H | H | H |
| bA386 | bB386 | CH₂CH₂ | Allyl | Me | Me | H | H | H |
| bA387 | bB387 | CH₂CH₂ | Allyl | Me | H | Me | H | H |
| bA388 | bB388 | CH₂CH₂ | Allyl | Me | H | H | Me | H |
| bA389 | bB389 | CH₂CH₂ | Allyl | Me | H | H | H | Me |
| bA390 | bB390 | CH₂CH₂ | Allyl | Me | Cl | H | H | H |
| bA391 | bB391 | CH₂CH₂ | Allyl | Me | H | Cl | H | H |
| bA392 | bB392 | CH₂CH₂ | Allyl | Me | H | H | Cl | H |
| bA393 | bB393 | CH₂CH₂ | Allyl | Me | H | H | H | Cl |
| bA394 | bB394 | CH₂CH₂ | Allyl | Me | Et | H | H | H |
| bA395 | bB395 | CH₂CH₂ | Allyl | Me | H | Et | H | H |
| bA396 | bB396 | CH₂CH₂ | Allyl | Me | H | H | Et | H |
| bA397 | bB397 | CH₂CH₂ | Allyl | Me | H | H | H | Et |
| bA398 | bB398 | CH₂CH₂ | Allyl | Me | OMe | H | H | H |
| bA399 | bB399 | CH₂CH₂ | Allyl | Me | H | OMe | H | H |
| bA400 | bB400 | CH₂CH₂ | Allyl | Me | H | H | OMe | H |
| bA401 | bB401 | CH₂CH₂ | Allyl | Me | H | H | H | OMe |
| bA402 | bB402 | CH₂CH₂ | Allyl | Me | F | H | H | H |
| bA403 | bB403 | CH₂CH₂ | Allyl | Me | H | F | H | H |
| bA404 | bB404 | CH₂CH₂ | Allyl | Me | H | H | F | H |
| bA405 | bB405 | CH₂CH₂ | Allyl | Me | H | H | H | F |
| bA406 | bB406 | CH₂CH₂ | Bzl | Me | H | H | H | H |
| bA407 | bB407 | CH₂CH₂ | Bzl | Me | Me | H | H | H |
| bA408 | bB408 | CH₂CH₂ | Bzl | Me | H | Me | H | H |
| bA409 | bB409 | CH₂CH₂ | Bzl | Me | H | H | Me | H |
| bA410 | bB410 | CH₂CH₂ | Bzl | Me | H | H | H | Me |
| bA411 | bB411 | CH₂CH₂ | Bzl | Me | Cl | H | H | H |
| bA412 | bB412 | CH₂CH₂ | Bzl | Me | H | Cl | H | H |
| bA413 | bB413 | CH₂CH₂ | Bzl | Me | H | H | Cl | H |
| bA414 | bB414 | CH₂CH₂ | Bzl | Me | H | H | H | Cl |
| bA415 | bB415 | CH₂CH₂ | Bzl | Me | Et | H | H | H |
| bA416 | bB416 | CH₂CH₂ | Bzl | Me | H | Et | H | H |
| bA417 | bB417 | CH₂CH₂ | Bzl | Me | H | H | Et | H |
| bA418 | bB418 | CH₂CH₂ | Bzl | Me | H | H | H | Et |
| bA419 | bB419 | CH₂CH₂ | Bzl | Me | OMe | H | H | H |
| bA420 | bB420 | CH₂CH₂ | Bzl | Me | H | OMe | H | H |
| bA421 | bB421 | CH₂CH₂ | Bzl | Me | H | H | OMe | H |
| bA422 | bB422 | CH₂CH₂ | Bzl | Me | H | H | H | OMe |
| bA423 | bB423 | CH₂CH₂ | Bzl | Me | F | H | H | H |
| bA424 | bB424 | CH₂CH₂ | Bzl | Me | H | F | H | H |
| bA425 | bB425 | CH₂CH₂ | Bzl | Me | H | H | F | H |
| bA426 | bB426 | CH₂CH₂ | Bzt | Me | H | H | H | F |
| bA427 | bB427 | CH₂CH₂ | p-MeO-Bzl | Me | H | H | H | H |
| bA428 | bB428 | CH₂CH₂ | p-Meo-Bzl | Me | Me | H | H | H |
| bA429 | bB429 | CH₂CH₂ | p-MeO-Bzl | Me | H | Me | H | H |
| bA430 | bB430 | CH₂CH₂ | p-MeO-Bzl | Me | H | H | Me | H |
| bA431 | bB431 | CH₂CH₂ | p-MeO-Bzl | Me | H | H | H | Me |
| bA432 | bB432 | CH₂CH₂ | p-MeO-Bzl | Me | Cl | H | H | H |
| bA433 | bB433 | CH₂CH₂ | p-MeO-Bzl | Me | H | Cl | H | H |
| bA434 | bB434 | CH₂CH₂ | p-MeO-Bzl | Me | H | H | Cl | H |
| bA435 | bB435 | CH₂CH₂ | p-MeO-Bzl | Me | H | H | H | Cl |
| bA436 | bB436 | CH₂CH₂ | p-MeO-Bzl | Me | Et | H | H | H |
| bA437 | bB437 | CH₂CH₂ | p-MeO-Bzl | Me | H | Et | H | H |
| bA438 | bB438 | CH₂CH₂ | p-MeO-Bzl | Me | H | H | Et | H |
| bA439 | bB439 | CH₂CH₂ | p-MeO-Bzl | Me | H | H | H | Et |
| bA440 | bB440 | CH₂CH₂ | p-MeO-Bzl | Me | OMe | H | H | H |
| bA441 | bB441 | CH₂CH₂ | p-MeO-Bzl | Me | H | OMe | H | H |
| bA442 | bB442 | CH₂CH₂ | p-MeO-Bzl | Me | H | H | OMe | H |
| bA443 | bB443 | CH₂CH₂ | p-MeO-Bzl | Me | H | H | H | OMe |
| bA444 | bB444 | CH₂CH₂ | p-MeO-Bzl | Me | F | H | H | H |
| bA445 | bB445 | CH₂H₂ | p-MeO-Bzl | Me | H | F | H | H |
| bA446 | bB446 | CH₂CH₂ | p-MeO-Bzl | Me | H | H | F | H |
| bA447 | bB447 | CH₂CH₂ | p-MeO-Bzl | Me | H | H | H | F |
| bA448 | bB448 | CH₂CH₂ | i-Pr | Me | Me | H | H | F |
| bA449 | bB449 | CH₂CH₂ | i-Pr | Me | H | Me | H | H |
| bA450 | bB450 | CH₂CH₂ | i-Pr | Me | H | H | Me | H |
| bA451 | bB451 | CH₂CH₂ | i-Pr | Me | H | H | H | Me |
| bA452 | bB452 | CH₂CH₂ | i-Pr | Me | Cl | H | H | H |
| bA453 | bB453 | CH₂CH₂ | i-Pr | Me | H | Cl | H | H |
| bA454 | bB454 | CH₂CH₂ | i-Pr | Me | H | H | Cl | H |
| bA455 | bB455 | CH₂CH₂ | i-Pr | Me | H | H | H | Cl |
| bA456 | bB456 | CH₂CH₂ | i-Pr | Me | Et | H | H | H |
| bA457 | bB457 | CH₂CH₂ | i-Pr | Me | H | Et | H | H |
| bA458 | bB458 | CH₂CH₂ | i-Pr | Me | H | H | Et | H |
| bA459 | bB459 | CH₂CH₂ | i-Pr | Me | H | H | H | Et |
| bA460 | bB460 | CH₂CH₂ | i-Pr | Me | OMe | H | H | H |
| bA461 | bB461 | CH₂CH₂ | i-Pr | Me | H | OMe | H | H |
| bA462 | bB462 | CH₂CH₂ | i-Pr | Me | H | H | OMe | H |
| bA463 | bB463 | CH₂CH₂ | i-Pr | Me | H | H | H | OMe |
| bA464 | bB464 | CH₂CH₂ | i-Pr | Me | F | H | H | H |
| bA465 | bB465 | CH₂CH₂ | i-Pr | Me | H | F | H | H |
| bA466 | bB466 | CH₂CH₂ | i-Pr | Me | H | H | F | H |
| bA467 | bB467 | CH₂CH₂ | i-Pr | Me | H | H | H | F |
| bA468 | bB468 | CH₂CH₂ | t-Bu | Me | H | H | H | H |
| bA469 | bB469 | CH₂CH₂ | t-Bu | Me | Me | H | H | H |
| bA470 | bB470 | CH₂CH₂ | t-Bu | Me | Me | Me | H | H |
| bA471 | bB471 | CH₂CH₂ | t-Bu | Me | H | H | Me | H |
| bA472 | bB472 | CH₂CH₂ | t-Bu | Me | H | H | H | Me |
| bA473 | bB473 | CH₂CH₂ | t-Bu | Me | Cl | H | H | H |
| bA474 | bB474 | CH₂CH₂ | t-Bu | Me | H | Cl | H | H |
| bA475 | bB475 | CH₂CH₂ | t-Bu | Me | H | H | Cl | H |
| bA476 | bB476 | CH₂CH₂ | t-Bu | Me | H | H | H | Cl |
| bA477 | bB477 | CH₂CH₂ | t-Bu | Me | H | H | H | H |
| bA478 | bB478 | CH₂CH₂ | t-Bu | Me | Et | H | H | H |
| bA479 | bB479 | CH₂CH₂ | t-Bu | Me | H | Et | H | H |
| bA480 | bB480 | CH₂CH₂ | t-Bu | Me | H | H | Et | H |
| bA481 | bB481 | CH₂CH₂ | t-Bu | Me | H | H | H | Et |
| bA482 | bB482 | CH₂CH₂ | t-Bu | Me | OMe | H | H | H |
| bA483 | bB483 | CH₂CH₂ | t-Bu | Me | H | OMe | H | H |
| bA484 | bB484 | CH₂CH₂ | t-Bu | Me | H | H | OMe | H |
| bA485 | bB485 | CH₂CH₂ | t-Bu | Me | H | H | H | OMe |
| bA486 | bB486 | CH₂CH₂ | t-Bu | Me | F | H | H | H |
| bA487 | bB487 | CH₂CH₂ | t-Bu | Me | H | F | H | H |
| bA488 | bB488 | CH₂CH₂ | t-Bu | Me | H | H | F | H |
| bA489 | bB489 | CH₂CH₂ | t-Bu | Me | H | H | H | F |
| bA490 | bB490 | CH₂CH₂ | Ph | Me | H | H | H | H |
| bA491 | bB491 | CH₂CH₂ | Ph | Me | Me | H | H | H |
| bA492 | bB492 | CH₂CH₂ | Ph | Me | Me | Me | H | H |
| bA493 | bB493 | CH₂CH₂ | Ph | Me | H | H | Me | H |
| bA494 | bB494 | CH₂CH₂ | Ph | Me | H | H | H | Me |
| bA495 | bB495 | CH₂CH₂ | Ph | Me | Cl | H | H | H |
| bA496 | bB496 | CH₂CH₂ | Ph | Me | H | Cl | H | H |
| bA497 | bB497 | CH₂CH₂ | Ph | Me | H | H | Cl | H |
| bA498 | bB498 | CH₂CH₂ | Ph | Me | H | H | H | Cl |
| bA499 | bB499 | CH₂CH₂ | Ph | Me | H | H | H | H |
| bA500 | bB500 | CH₂CH₂ | Ph | Me | Et | H | H | H |
| bA501 | bB501 | CH₂CH₂ | Ph | Me | H | Et | H | H |
| bA502 | bB502 | CH₂CH₂ | Ph | Me | H | H | Et | H |
| bA503 | bB503 | CH₂CH₂ | Ph | Me | H | H | H | Et |
| bA504 | bB504 | CH₂CH₂ | Ph | Me | OMe | H | H | H |
| bA505 | bB505 | CH₂CH₂ | Ph | Me | H | OMe | H | H |
| bA506 | bB506 | CH₂CH₂ | Ph | Me | H | H | OMe | H |
| bA507 | bB507 | CH₂CH₂ | Ph | Me | H | H | H | OMe |
| bA508 | bB508 | CH₂CH₂ | Ph | Me | F | H | H | H |
| bA509 | bB509 | CH₂CH₂ | Ph | Me | H | F | H | H |
| bA510 | bB510 | CH₂CH₂ | Ph | Me | H | H | F | H |
| bA511 | bB511 | CH₂CH₂ | Ph | Me | H | H | H | F |
| bA512 | bB512 | OCH₂ | Me | Me | H | H | H | H |
| bA513 | bB513 | OCH₂ | Me | Me | Me | H | H | H |
| bA514 | bB514 | OCH₂ | OMe | Me | H | Me | H | H |
| bA515 | bB515 | OCH₂ | Me | Me | H | H | Me | H |
| bA516 | bB516 | OCH₂ | Me | Me | H | H | H | Me |
| bA517 | bB517 | OCH₂ | Me | Me | Cl | H | H | H |
| bA518 | bB518 | OCH₂ | Me | Me | H | Cl | H | H |
| bA519 | bB519 | OCH₂ | Me | Me | H | H | Cl | H |
| bA520 | bB520 | OCH₂ | Me | Me | H | H | H | Cl |
| bA521 | bB521 | OCH₂ | Me | Me | Et | H | H | H |
| bA522 | bB522 | OCH₂ | Me | Me | H | Et | H | H |
| bA523 | bB523 | OCH₂ | Me | Me | H | H | Et | H |
| bA524 | bB524 | OCH₂ | Me | Me | H | H | H | Et |
| bA525 | bB525 | OCH₂ | Me | Me | OMe | H | H | H |
| bA526 | bB526 | OCH₂ | Me | Me | H | OMe | H | H |
| bA527 | bB527 | OCH₂ | Me | Me | H | H | OMe | H |
| bA528 | bB528 | OCH₂ | Me | Me | H | H | H | OMe |
| bA529 | bB529 | OCH₂ | Me | Me | F | H | H | H |
| bA530 | bB530 | OCH₂ | Me | Me | H | F | H | H |
| bA531 | bB531 | OCH₂ | Me | Me | H | H | F | H |
| bA532 | bB532 | OCH₂ | Me | Me | H | H | H | F |
| bA533 | bB533 | OCH₂ | Et | Me | H | H | H | H |
| bA534 | bB534 | OCH₂ | Et | Me | Me | H | H | H |
| bA535 | bB535 | OCH₂ | Et | Me | H | Me | H | H |
| bA536 | bB536 | OCH₂ | Et | Me | H | H | Me | H |
| bA537 | bB537 | OCH₂ | Et | Me | H | H | H | Me |
| bA538 | bB538 | OCH₂ | Et | Me | Cl | H | H | H |
| bA539 | bB539 | OCH₂ | Et | Me | H | Cl | H | H |
| bA540 | bB540 | OCH₂ | Et | Me | H | H | Cl | H |
| bA541 | bB541 | OCH₂ | Et | Me | H | H | H | Cl |
| bA542 | bB542 | OCH₂ | Et | Me | Et | H | H | H |
| bA543 | bB543 | OCH₂ | Et | Me | H | Et | H | H |
| bA544 | bB544 | OCH₂ | Et | Me | H | H | Et | H |
| bA545 | bB545 | OCH₂ | Et | Me | H | H | H | Et |
| bA546 | bB546 | OCH₂ | Et | Me | OMe | H | H | H |
| bA547 | bB547 | OCH₂ | Et | Me | H | OMe | H | H |
| bA548 | bB548 | OCH₂ | Et | Me | H | H | OMe | H |
| bA549 | bB549 | OCH₂ | Et | Me | H | H | H | OMe |
| bA550 | bB550 | OCH₂ | Et | Me | F | H | H | H |
| bA551 | bB551 | OCH₂ | Et | Me | H | F | H | H |
| bA552 | bB552 | OCH₂ | Et | Me | H | H | F | H |
| bA553 | bB553 | OCH₂ | Et | Me | H | H | H | F |
| bA554 | bB554 | OCH₂ | Allyl | Me | H | H | H | H |
| bA555 | bB555 | OCH₂ | Allyl | Me | Me | H | H | H |
| bA556 | bB556 | OCH₂ | Allyl | Me | H | Me | H | H |
| bA557 | bB557 | OCH₂ | Allyl | Me | H | H | Me | H |
| bA558 | bB558 | OCH₂ | Allyl | Me | H | H | H | Me |
| bA559 | bB559 | OCH₂ | Alkyl | Me | Cl | H | H | H |
| bA560 | bB560 | OCH₂ | Allyl | Me | H | Cl | H | H |
| bA561 | bB561 | OCH₂ | Allyl | Me | H | H | Cl | H |
| bA562 | bB562 | OCH₂ | Allyl | Me | H | H | H | Cl |
| bA563 | bB563 | OCH₂ | Allyl | Me | Et | H | H | H |
| bA564 | bB564 | OCH₂ | Allyl | Me | H | Et | H | H |
| bA565 | bB565 | OCH₂ | Allyl | Me | H | H | Et | H |
| bA566 | bB566 | OCH₂ | Allyl | Me | H | H | H | Et |
| bA567 | bB567 | OCH₂ | Allyl | Me | OMe | H | H | H |
| bA568 | bB568 | OCH₂ | Allyl | Me | H | OMe | H | H |
| bA569 | bB569 | OCH₂ | Allyl | Me | H | H | OMe | H |
| - bA570 | bB570 | OCH₂ | Allyl | Me | H | H | H | OMe |
| bA571 | bB571 | OCH₂ | Allyl | Me | F | H | H | H |
| bA572 | bB572 | OCH₂ | Allyl | Me | H | F | H | H |
| bA573 | bB573 | OCH₂ | Allyl | Me | H | H | F | H |
| bA574 | bB574 | OCH₂ | Allyl | Me | H | H | H | F |
| bA575 | bB575 | OCH₂ | Bzl | Me | H | H | H | H |
| bA576 | bB576 | OCH₂ | Bzl | Me | Me | H | H | H |
| bA577 | bB577 | OCH₂ | Bzl | Me | H | Me | H | H |
| bA578 | bB578 | OCH₂ | Bzl | Me | H | H | Me | H |
| bA579 | bB579 | OCH₂ | Bzl | Me | H | H | H | Me |
| bA580 | bB580 | OCH₂ | Bzl | Me | Cl | H | H | H |
| bA581 | bB581 | OCH₂ | Bzl | Me | H | Cl | H | H |
| bA582 | bB582 | OCH₂ | Bzl | Me | H | H | Cl | H |
| bA583 | bBS83 | OCH₂ | Bzl | Me | H | H | H | Cl |
| bA584 | bB584 | OCH₂ | Bzl | Me | Et | H | H | H |
| bA585 | bB585 | OCH₂ | Bzl | Me | H | Et | H | H |
| bA586 | bB586 | OCH₂ | Bzl | Me | H | H | Et | H |
| bA587 | bB587 | OCH₂ | Bzl | Me | H | H | H | Et |
| bA588 | bB588 | OCH₂ | Bzl | Me | OMe | H | H | H |
| bA589 | bB589 | OCH₂ | Bzl | Me | H | OMe | H | H |
| bA590 | bB590 | OCH₂ | Bzl | Me | H | H | OMe | H |
| bA591 | bB591 | OCH₂ | Bzl | Me | H | H | H | OMe |
| bA592 | bB592 | OCH₂ | Bzl | Me | F | H | H | H |
| bA593 | bB593 | OCH₂ | Bzl | Me | H | F | H | H |
| bA594 | bB594 | OCH₂ | Bzl | Me | H | H | F | H |
| bA595 | bB595 | OCH₂ | Bzl | Me | H | H | H | F |
| bA596 | bB596 | OCH₂ | p-MeO-Bzl | Me | H | H | H | H |
| bA597 | bB597 | OCH₂ | p-MeO-Bzl | Me | Me | H | H | H |
| bA598 | bB598 | OCH₂ | p-MeO-Bzl | Me | H | Me | H | H |
| bA599 | bB599 | OCH₂ | p-MeO-Bzl | Me | H | H | Me | H |
| bA600 | bB600 | OCH₂ | p-MeO-Bzl | Me | H | H | H | Me |
| bA601 | bB601 | OCH₂ | p-MeO-Bzl | Me | Cl | H | H | H |
| bA602 | bB602 | OCH₂ | p-Meo-Bzl | Me | H | Cl | H | H |
| bA603 | bB603 | OCH₂ | p-MeO-Bzl | Me | H | H | Cl | H |
| bA604 | bB604 | OCH₂ | p-MeO-Bzl | Me | H | H | H | Cl |
| bA605 | bB605 | OCH₂ | p-MeO-Bzl | Me | Et | H | H | H |
| bA606 | bB606 | OCH₂ | p-MeO-Bzl | Me | H | Et | H | H |
| bA607 | bB607 | OCH₂ | p-MeO-Bzl | Me | H | H | Et | H |
| bA608 | bB608 | OCH₂ | p-MeO-Bzl | Me | H | H | H | Et |
| bA609 | bB609 | OCH₂ | p-MeO-Bzl | Me | OMe | H | H | H |
| bA610 | bB610 | OCH₂ | p-MeO-Bzl | Me | H | OMe | H | H |
| bA611 | bB611 | OCH₂ | p-MeO-Bzl | Me | H | H | OMe | H |
| bA612 | bB612 | OCH₂ | p-MeO-Bzl | Me | H | H | H | OMe |
| bA613 | bB613 | OCH₂ | p-MeO-Bzl | Me | F | H | H | H |
| bA614 | bB614 | OCH₂ | p-MeO-Bzl | Me | H | F | H | H |
| bA615 | bB615 | OCH₂ | p-MeO-Bzl | Me | H | H | F | H |
| bA616 | bB616 | OCH₂ | p-MeO-Bzl | Me | H | H | H | F |
| bA617 | bB617 | OCH₂ | i-Pr | Me | Me | H | H | F |
| bA618 | bB618 | OCH₂ | i-Pr | Me | H | Me | H | H |
| bA619 | bB619 | OCH₂ | i-Pr | Me | H | H | Me | H |
| bA620 | bB620 | OCH₂ | i-Pr | Me | H | H | H | Me |
| bA621 | bB621 | OCH₂ | i-Pr | Me | Cl | H | H | H |
| bA622 | bB622 | OCH₂ | i-Pr | Me | H | Cl | H | H |
| bA623 | bB623 | OCH₂ | i-Pr | Me | H | H | Cl | H |
| bA624 | bB624 | OCH₂ | i-Pr | Me | H | H | H | Cl |
| bA625 | bB625 | OCH₂ | i-Pr | Me | Et | H | H | H |
| bA626 | bB626 | OCH₂ | i-Pr | Me | H | Et | H | H |
| bA627 | bB627 | OCH₂ | i-Pr | Me | H | H | Et | H |
| bA628 | bB628 | OCH₂ | i-Pr | Me | H | H | H | Et |
| bA629 | bB629 | OCH₂ | i-Pr | Me | OMe | H | H | H |
| bA630 | bB630 | OCH₂ | i-Pr | Me | H | OMe | H | H |
| bA631 | bB631 | OCH₂ | i-Pr | Me | H | H | OMe | H |
| bA632 | bB632 | OCH₂ | i-Pr | Me | H | H | H | OMe |
| bA633 | bB633 | OCH₂ | i-Pr | Me | F | H | H | H |
| bA634 | bB634 | OCH₂ | i-Pr | Me | H | F | H | H |
| bA635 | bB635 | OCH₂ | i-Pr | Me | H | H | F | H |
| bA636 | bB636 | OCH₂ | i-Pr | Me | H | H | H | F |
| bA637 | bB637 | OCH₂ | t-Bu | Me | H | H | H | H |
| bA638 | bB638 | OCH₂ | t-Bu | Me | Me | H | H | H |
| bA639 | bB639 | OCH₂ | t-Bu | Me | Me | Me | H | H |
| bA640 | bB640 | OCH₂ | t-Bu | Me | H | H | Me | H |
| bA641 | bB641 | OCH₂ | t-Bu | Me | H | H | H | Me |
| bA642 | bB642 | OCH₂ | t-Bu | Me | Cl | H | H | H |
| bA643 | bB643 | OCH₂ | t-Bu | Me | H | Cl | H | H |
| bA644 | bB644 | OCH₂ | t-Bu | Me | H | H | Cl | H |
| bA645 | bB645 | OCH₂ | t-Bu | Me | H | H | H | Cl |
| bA646 | bB646 | OCH₂ | t-Bu | Me | H | H | H | H |
| bA647 | bB647 | OCH₂ | t-Bu | Me | Et | H | H | H |
| bA648 | bB648 | OCH₂ | t-Bu | Me | H | Et | H | H |
| bA649 | bB649 | OCH₂ | t-Bu | Me | H | H | Et | H |
| bA650 | bB650 | OCH₂ | t-Bu | Me | H | H | H | Et |
| bA651 | bB651 | OCH₂ | t-Bu | Me | OMe | H | H | H |
| bA652 | bB652 | OCH₂ | t-Bu | Me | H | OMe | H | H |
| bA653 | bB653 | OCH₂ | t-Bu | Me | H | H | OMe | H |
| bA654 | bB654 | OCH₂ | t-Bu | Me | H | H | H | OMe |
| bA655 | bB655 | OCH₂ | t-Bu | Me | F | H | H | H |
| bA656 | bB656 | OCH₂ | t-Bu | Me | H | F | H | H |
| bA657 | bB657 | OCH₂ | t-Bu | Me | H | H | F | H |
| bA658 | bB658 | OCH₂ | t-Bu | Me | H | H | H | F |
| bA659 | bB659 | OCH₂ | Ph | Me | H | H | H | H |
| bA660 | bB660 | OCH₂ | Ph | Me | Me | H | H | H |
| bA661 | bB661 | OCH₂ | Ph | Me | Me | Me | H | H |
| bA662 | bB662 | OCH₂ | Ph | Me | H | H | Me | H |
| bA663 | bB663 | OCH₂ | Ph | Me | H | H | H | Me |
| bA664 | bB664 | OCH₂ | Ph | Me | Cl | H | H | H |
| bA665 | bB665 | OCH₂ | Ph | Me | H | Cl | H | H |
| bA666 | bB666 | OCH₂ | Ph | Me | H | H | Cl | H |
| bA667 | bB667 | OCH₂ | Ph | Me | H | H | H | Cl |
| bA668 | bB668 | OCH₂ | Ph | Me | H | H | H | H |
| bA669 | bB669 | OCH₂ | Ph | Me | Et | H | H | H |
| bA670 | bB670 | OCH₂ | Ph | Me | H | Et | H | H |
| bA671 | bB671 | OCH₂ | Ph | Me | H | H | Et | H |
| bA672 | bB672 | OCH₂ | Ph | Me | H | H | H | Et |
| bA673 | bB673 | OCH₂ | Ph | Me | OMe | H | H | H |
| bA674 | bB674 | OCH₂ | Ph | Me | H | OMe | H | H |
| bA675 | bB675 | OCH₂ | Ph | Me | H | H | OMe | H |
| bA676 | bB676 | OCH₂ | Ph | Me | H | H | H | OMe |
| bA677 | bB677 | OCH₂ | Ph | Me | F | H | H | H |
| bA678 | bB678 | OCH₂ | Ph | Me | H | F | H | H |
| bA679 | bB679 | OCH₂ | Ph | Me | H | H | F | H |
| bA680 | bB680 | OCH₂ | Ph | Me | H | H | H | F |

| | | | | | | | | |
|---|---|---|---|---|---|---|---|---|
| Physikalische Daten zu Verbindungen aus Tabelle 2: Verbindungs-Nr.: bA214: Ref (¹), Rₜ = 6,7, n-Hexan/Isopropanol = 92:8 (v/v). Flow = 0,5 ml / min, Verbindungs-Nr.: bA218: Ref (^{1,4}), Rₜ = 13,9, n-Hexan/Isopropanol = 90:10 (v/v). Flow = 1,0 ml / min, Verbindungs-Nr.: bA237: Ref (³), Rₜ = 17,4, n-Hexan/Isopropanol = 99:1 (v/v). Flow = 0,3 ml / min, Verbindungs-Nr.: bB237: Ref (³), Rₜ = 19,9, n-Hexan/Isopropanol = 99:1 (v/v), Flow = 0,3 ml / min, Verbindungs-Nr.: bA239: Ref (^{3,4}), Rₜ = 12,3, n-Hexan/Isopropanol = 90:10 (v/v). Flow = 1,0 ml / min, Verbindungs-Nr.: bA406: Ref (¹), Rₜ m 7,1, n-Hexan/Isopropanol = 96:4 (v/v). Flow = 0,5 ml / min. | | | | | | | | |

## Patentansprüche

1. Verfahren zur Herstellung von optisch aktiven cyclischen Aminen der Formel (I) oder deren Salzen, 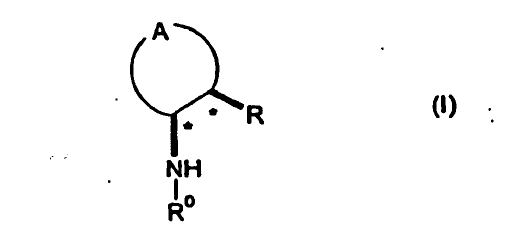 worin
A gemeinsam mit den jeweils mit einem Stern (*) bezeichneten C-Atomen einen carbocyclischen oder heterocyclischen gesättigten oder ungesättigten, nicht- aromatischen Ring mit 3 bis 30 Ringatomen, der zusätzlich zu den Resten R und NH-R⁰ weiter substituiert sein kann, bedeutet,
R⁰ unabhängig von R einen Rest (C₁-C₆)Alkyl, (C₂-C₆)Alkenyl oder (C₃-C₆)Alkinyl, wobei jeder der drei letztgenannten Reste unsubstituiert oder durch einen oder mehrere Reste aus der Gruppe Halogen, Cyano, (C₁- C₄)Alkoxy, (C₁-C₄)Haloalkoxy, (C₁-C₄)Alkylthio, (C₁-C₄)Alkanoyloxy, (C₁-C₄)Haloalkanoyloxy, Aryl, Aryloxy, Aroyl, Aroyloxy und Heterocylyl, wobei jeder der letztgenannten 5 Reste unsubstituiert oder substituiert ist, substituiert ist, oder
(C₃-C₉)Cycloalky), (C₄-C₉)Cycloalkenyl, Aryl oder Heterocyclyl, wobei jeder der letztgenannten 4 Reste unsubstituiert oder substituiert ist, bedeutet,
R unabhängig von R⁰
(C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, Cyano, (C₁-C₆)Alkoxy, (C₁- C₆)Haloalkoxy, (C₁-C₆)Alkylthio, Aryl, das unsubstituiert oder substituiert ist, und Heteroaryl, das unsubstituiert oder substituiert ist, substituiert ist, oder
Aryl, das unsubstituiert oder substituiert ist, oder
Heteroaryl, das unsubstituiert oder substituiert ist,
bedeutet oder
R⁰ und A einen Ring B1 bilden und dabei gemeinsam mit der NH-Gruppe und dem an der NH-Gruppe gebundenen, mit einem Stern (*) bezeichneten C-Atom einen heterocyclischen Ring mit 4 bis 30 Ringatomen, der gegebenenfalls zusätzlich weiter substituiert ist und der gegebenenfalls 1 oder 2 weitere Heteroatome aus der Gruppe N, O und S enthält, bedeuten oder
R und A einen Ring B2 bilden und dabei gemeinsam mit dem an R gebundenen, mit einem Stern (*) bezeichneten C-Atom einen carbocyclischen oder heterocyclischen Ring mit 3 bis 30 Ringatomen, der gegebenenfalls zusätzlich weiter substituiert ist und der im Falle eines heterocyclischen Rings 1 oder 2 oder 3 weitere Heteroatome aus der Gruppe N, O und S enthält, bedeuten oder
R⁰ und R einen Ring B3 bilden und dabei gemeinsam mit der NH-Gruppe und den jeweils mit einem Stern (*) bezeichneten C-Atomen einen heterocyclischen Ring mit 4 bis 30 Ringatomen, der gegebenenfalls zusätzlich weiter substituiert ist und der gegebenenfalls 1 oder 2 weitere Heteroatome aus der Gruppe N, O und S enthält, bedeuten oder
R⁰ und R und gegebenenfalls A zwei oder mehrere der genannten Ringe B1, B2 und 83 zugleich bilden,
wobei R und die Gruppe NH-R⁰ an den beiden jeweils mit einem Stern (*) markierten Ring-C-Atomen in cis-Stellung zueinander ausgerichtet sind und die stereochemische Konfiguration an diesen C-Atomen von der racemischen Konfiguration verschieden ist,
**dadurch gekennzeichnet, dass** man ein Imin der Formel (II) oder ein Salz davon, 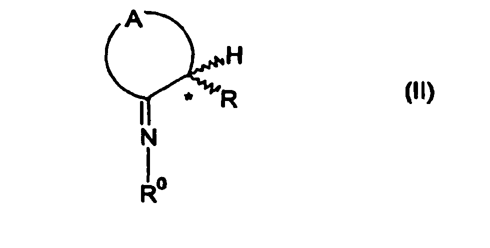 wobei A, R⁰ und R wie in Formel (I) definiert sind und die Verbindung der Formel (II) bezüglich des mit einem Stern (*) markierten Ring-C-Atoms als racemisches Gemisch oder als ein Gemisch mit einem beliebigen anderen Isomerenverhältnis der betreffenden Stereoisomere vorliegt,
in Gegenwart von Wasserstoff oder einem Wasserstoff-Donor und einem nicht enzymatischen Katalysator, der einen katalytisch wirksamen optisch aktiven Komplex eines oder mehrerer Übergangsmetalle aus der Gruppe Ruthenium, Rhodium, Palladium, Iridium, Osmium, Platin, Eisen, Nickel und Samarium mit organischen Liganden enthält, zur Verbindung der Formel (I) umsetzt.

2. Verfahren gemäß Anspruch 1, **dadurch gekennzeichnet, dass** die Verbindungen (I) oder deren Salze mit cis-Konfiguration an den beiden jeweils mit Stern (*) bezeichneten Chiralitätszentren im Vergleich zur trans-Konfiguration mit einer Selektivität von 60 bis 100% erhalten werden, wobei ein Enantiomeres der cis-Verbindung mit einer Enantioselektivität von jeweils mehr als 20% ee bezogen auf den Gesamtgehalt an Enantiomeren der cis-Verbindung erhalten wird.

3. Verfahren gemäß Anspruch 1 oder 2, **dadurch gekennzeichnet, dass** Verbindungen der Formel (Ia) oder deren Salze hergestellt werden, 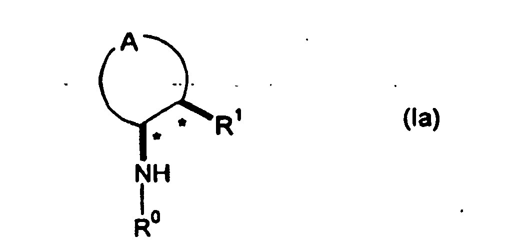 worin
A gemeinsam mit den jeweils mit einem Stern (*) bezeichneten C-Atomen einen carbocyclischen oder heterocyclischen, gesättigten oder ungesättigten, nicht- aromatischen Ring mit 3 bis 30 Ringatomen und im Falle eines heterocyclischen Rings mit 1 bis 3 Heteroringatomen aus der Gruppe N, O und S, der zusätzlich zu den Resten R¹ und NH-R⁰ unsubstituiert oder durch einen oder mehrere Reste aus der Gruppe Halogen, Hydroxy, Amino, Cyano, Nitro, (C₁-C₄)Alkyl, (C₃-C₉)Cycloalkyl, (C₁-C₄)Alkoxy, (C₁-C₄)Alkoxycarbonyl, (C₁-C₄)Alkylthio, (C₁-C₄)Alkylsulfonyl, Aryl und Heterocyclyl, wobei jeder der letztgenannten 8 Reste unsubstituiert oder durch einen oder mehrere Reste aus der Gruppe Halogen, Cyano, (C₃-C₉)Cycloalkyl, (C₁-C₄)Alkoxy, (C₁- C₄)Alkylthio und im Falle cyclischer Reste auch (C₁-C₄)Alkyl und (C₁- C₄)Haloalkyl substituiert ist, weiter substituiert ist, bedeutet.
R⁰ unabhängig von R¹ einen Rest (C₁-C₆)Alkyl, (C₂-C₆)Alkenyl oder (C₃- C₆)Alkinyl, wobei jeder der drei letztgenannten Reste unsubstituiert oder durch einen oder mehrere Reste aus der Gruppe Halogen, Cyano, (C₁-C₄)Alkoxy, (C₁-C₄)Haloalkoxy, (C₁-C₄)Alkylthio, (C₁- C₄)Alkanoyloxy,(C₁-C₄)Haloalkanoyloxy, Aryl, Aryloxy, Aroyl, Aroyloxy und Heterocyclyl, wobei jeder der letztgenannten 5 Reste unsubstituiert oder substituiert ist, substituiert ist,
oder (C₃-C₉)Cycloalkyl, (C₄-C₉)Cycloalkenyl, Aryl oder Heterocyclyl, wobei jeder der letztgenannten 4 Reste unsubstituiert oder substituiert ist, bedeutet,
R¹ unabhängig von R⁰ (C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, Cyano, (C₁-C₄)Alkoxy, (C₁- C₄)Haloalkoxy und (C₁-C₄)Alkylthio substituiert ist,
oder Aryl oder Heterocyclyl, wobei jeder der 2 letztgenannten Reste unsubstituiert oder substituiert ist,
bedeutet,
wobei die Substituenten R¹ und die Aminogruppe an den beiden jeweils mit einem Stern (*) markierten Ring-C-Atomen in cis-Stellung zueinander ausgerichtet sind und die Verbindung (la) als stereochemisch reine Verbindung der Formel (Ia-A) oder (Ia-B), 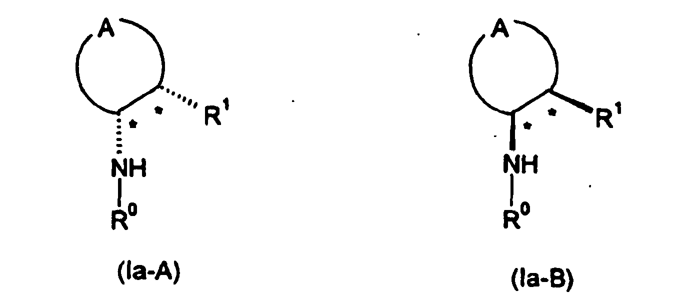 worin die allgemeinen Reste wie in Formel (Ia) definiert sind, oder als Isomerengemisch der Verbindungen der Formeln (Ia-A) und (Ia-B) in einem vom Verhältnis 1:1 verschiedenen Isomerenverhältnis vorliegt.

4. Verfahren gemäß Anspruch 1 oder 2, **dadurch gekennzeichnet, dass** Verbindungen der Formel (Ib) oder deren Salze hergestellt werden, 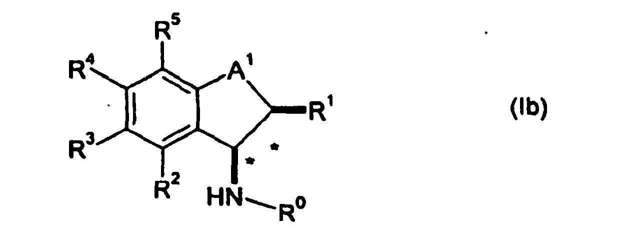 worin
A¹ eine direkte Bindung oder eine Gruppe der Formel (CR⁶R⁷)ₙ, worin n eine Zahl von 1 bis 6 und R⁶ und R⁷ unabhängig voneinander bzw. im Fall, dass n größer 1 ist, die Reste R⁶ bzw. R⁷ jeweils unabhängig voneinander Wasserstoff, (C₁-C₄)Alkyl, (C₁-C₄)Haloalkyl, Halogen, (C₁-C₄)Alkoxy oder (C₁- C₄)Haloalkoxy bedeuten, wobei einzelne Gruppen CR⁶R⁷ durch Heteroatome aus der Gruppe O und S ersetzt sein können, bedeutet,
R⁰ unabhängig von R¹ einen Rest (C₁-C₁₀)Alkyl oder (C₂-C₆)Alkenyl, wobei jeder der beiden letztgenannten Reste unsubstituiert oder durch einen oder mehrere Reste aus der Gruppe Halogen, Cyano, (C₁- C₄)Alkoxy, (C₁-C₄)Haloalkoxy, (C₁-C₄)Alkylthio, (C₁-C₄)Alkanoyloxy, (C₁-C₄)Haloalkanoyloxy, Aryl, Aryloxy, Aroyl, Aroyloxy und Heterocyclyl, wobei jeder der letztgenannten 5 Reste unsubstituiert oder substituiert ist, substituiert ist,
oder (C₃-C₉)Cycloalkyl, (C₄-C₉)Cycloalkenyl, Aryl oder Heterocyclyl, wobei jeder der letztgenannten 4 Reste unsubstituiert oder substituiert ist,
bedeutet,
R¹ unabhängig von R⁰
(C₁-C₆)Alkyl, das unsubstituiert oder durch einen oder mehrere Reste aus der Gruppe Halogen, Cyano, (C₁-C₄)Alkoxy, (C₁-C₄)Haloalkoxy, (C₁- C₄)Alkylthio und Aryl, das gegebenenfalls substituiert ist, und Heterocyclyl, das gegebenenfalls substituiert ist,
substituiert ist, oder
Aryl, das gegebenenfalls substituiert ist, oder
Heterocyclyl, das gegebenenfalls substituiert ist,
bedeutet und
R² H, (C₁-C₄)Alkyl, (C₁-C₃)Haloalkyl, Halogen, (C₁-C₃)Alkoxy, (C₁-C₃)Haloalkoxy oder CN,
R³ H, (C₁-C₄)Alkyl, (C₁-C₃)Haloalkyl, Halogen, (C₁-C₃)Alkoxy, (C₁-C₃)Haloalkoxy oder CN,
R⁴ H, (C₁-C₄)Alkyl, (C₁-C₃)Haloalkyl, Halogen, (C₁-C₃)Alkoxy, (C₁-C₃)Haloalkoxy oder CN und
R⁵ H, (C₁-C₄)Alkyl, (C₁-C₃)Haloalkyl, Halogen, (C₁-C₃)Alkoxy, (C₁-C₃)Haloalkoxy oder CN
bedeuten,
wobei die Substituenten R¹ und die Aminogruppe an den beiden jeweils mit einem Stern (*) markierten Ring-C-Atomen in cis-Stellung zueinander ausgerichtet sind und die Verbindung als stereochemisch reine Verbindung der Formel (Ib-A) oder (Ib-B), 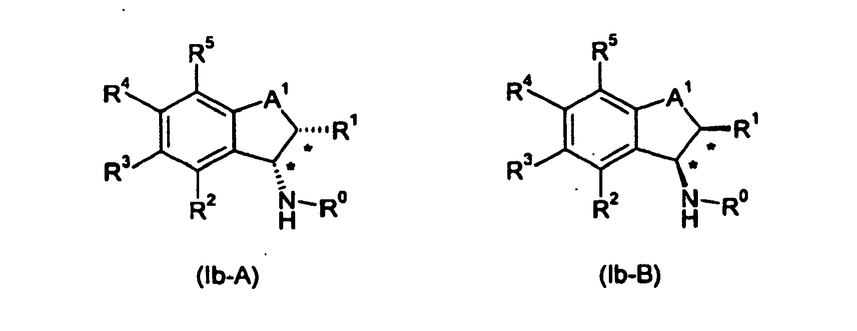 worin die allgemeinen Reste wie in Formel (Ib) definiert sind, oder als Isomerengemisch der Verbindungen der Formeln (Ib-A) und (Ib-B) in einem vom Verhältnis 1:1 verschiedenen Isomerenverhältnis vorliegt.

5. Verfahren gemäß einem der Ansprüche 1 bis 4, **dadurch gekennzeichnet, dass** die Reaktion in Gegenwart eines inerten organischen Lösungsmittels oder in Substanz durchgeführt wird.

6. Verfahren gemäß einem der Ansprüche 1 bis 5, **dadurch gekennzeichnet, dass** die Reaktion als katalytische Hydrierung durchgeführt wird.

7. Verfahren gemäß einem der Ansprüche 1 bis 5, **dadurch gekennzeichnet, dass** die Reaktion als katalytische Transferhydrierung durchgeführt wird.

8. Verfahren gemäß Anspruch 7, **dadurch gekennzeichnet, dass** die Reaktion mit Ameisensäure als Wasserstoffdonor in Gegenwart eines tertiären Amins durchgeführt wird.

9. Verfahren gemäß einem der Ansprüche 1 bis 8, **dadurch gekennzeichnet, dass** das Imin der Formel (II) aus dem entsprechenden Keton und einem Amin der Formel R⁰-NH₂ in-situ hergestellt und reduziert wird.

10. Verfahren gemäß einem der Ansprüche 1 bis 9, **dadurch gekennzeichnet, dass** man aus der Verbindung der Formel (I) oder deren Salzen, die Gruppe R⁰ nach üblichen Methoden abspaltet und Amine der Formel (I') oder deren Salze erhält, 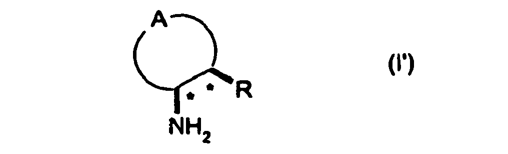 wobei
A und R und die stereochemische Konfiguration wie in Formel (I) definiert sind.

## Claims

1. A process for preparing optically active cyclic amines of the formula (I) or salts thereof 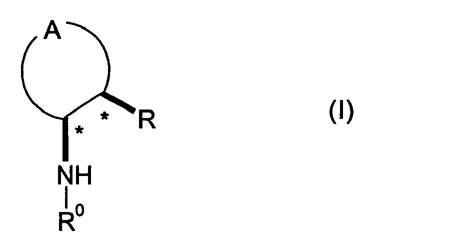 in which
A, together with the carbon atoms designated with an asterisk (*) in each case, is a carbocyclic or heterocyclic, saturated or unsaturated, nonaromatic ring which has from 3 to 30 ring atoms, and may be further substituted in addition to the R and NH-R⁰ radicals
R⁰, independently of R, is a (C₁-C₆)alkyl, (C₂-C₆)alkenyl or (C₃-C₆)alkynyl radical, where each of the three latter radicals may be unsubstituted or substituted by one or more radicals selected from the group consisting of halogen, cyano, (C₁-C₄)alkoxy, (C₁-C₄)haloalkoxy, (C₁-C₄)alkylthio, (C₁-C₄)alkanoyloxy, (C₁-C₄)haloalkanoyloxy, aryl, aryloxy, aroyl, aroyloxy and heterocyclyl, where each of the latter 5 radicals is unsubstituted or substituted, or
(C₃-C₉)cycloalkyl, (C₄-C₉)cycloalkenyl, aryl or heterocyclyl, where each of the latter 4 radicals is unsubstituted or substituted,
R, independently of R⁰, is
(C₁-C₆)alkyl, (C₂-C₆)alkenyl or (C₂-C₆)alkynyl, where each of the three latter radicals is unsubstituted or substituted by one or more radicals selected from the group consisting of halogen, cyano, (C₁-C₆)alkoxy, (C₁- C₆)haloalkoxy, (C₁-C₆)alkylthio, aryl which is unsubstituted or substituted, and heteroaryl which is unsubstituted or substituted, or
aryl which is unsubstituted or substituted, or
heteroaryl which is unsubstituted or substituted,
or
R⁰ and A form a ring B1 and, together with the NH group and the carbon atom which is designated with an asterisk (*) and is bonded to the NH group, are a heterocyclic ring which has from 4 to 30 ring atoms, and is optionally additionally further substituted and which optionally contains 1 or 2 further heteroatoms from the group of N, O and S, or
R and A form a ring B2 and, together with the carbon atom which is designated with an asterisk (*) and is bonded to R, are a carbocyclic or heterocyclic ring which has from 3 to 30 ring atoms, and is optionally additionally further substituted, and which, in the case of a heterocyclic ring, contains 1, 2 or 3 further heteroatoms from the group of N, O and S, or
R⁰ and R form a ring B3 and, together with the NH group and the carbon atoms designated with an asterisk (*) in each case, are a heterocyclic ring which has from 4 to 30 ring atoms, and is optionally additionally further substituted and which optionally contains 1 or 2 further heteroatoms from the group of N, O and S, or
R⁰ and R and, if appropriate, A may simultaneously form two or more of the rings B1, B2 and B3 mentioned,
where R and the NH-R⁰ group on the two ring carbon atoms marked with an asterisk (*) in each case are arranged in cis arrangement to one another and the stereochemical configuration on these carbon atoms is different from the racemic configuration,
which comprises converting an imine of the formula (II) or a salt thereof 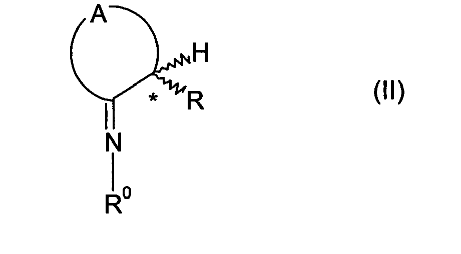 where A, R⁰ and R are each as defined in formula (I) and the compound of the formula (II), with regard to the ring carbon atom marked with an asterisk (*), is present in the form of a racemic mixture or in the form of a mixture with any other isomeric ratio of the stereoisomers in question,
in the presence of hydrogen or a hydrogen donor and a nonenzymatic catalyst which comprises a catalytically active optically active complex of one or more transition metals from the group of ruthenium, rhodium, palladium, iridium, osmium, platinum, iron, nickel and samarium, with organic ligands, to the compound of the formula (I).

2. The process as claimed in claim 1, wherein the compounds (I) or salts thereof with cis configuration on the two chiral centers designated with an asterisk (*) in each case are obtained with a selectivity of from 60 to 100% in comparison to the trans configuration, one enantiomer of the cis compound being obtained with an enantioselectivity of in each case more than 20% ee based on the total content of enantiomers of the cis compound.

3. The process as claimed in claim 1 or 2, wherein compounds of the formula (Ia) or salts thereof are prepared 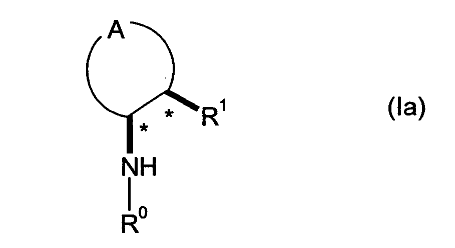 in which
A, together with the carbon atoms designated with an asterisk (*) in each case, is a carbocyclic or heterocyclic, saturated or unsaturated, nonaromatic ring having from 3 to 30 ring atoms and, in the case of a heterocyclic ring which has from 1 to 3 heterocyclic ring atoms from the group of N, O and S and, in addition to the R¹ and NH-R⁰ radicals is unsubstituted or further substituted by one or more radicals selected from the group consisting of halogen, hydroxy, amino, cyano, nitro, (C₁-C₄)alkyl, (C₃-C₉)cycloalkyl, (C₁-C₄)alkoxy, (C₁- C₄)alkoxycarbonyl, (C₁-C₄)alkylthio, (C₁-C₄)alkylsulfonyl, aryl and heterocyclyl, where each of the latter 8 radicals is unsubstituted or substituted by one or more radicals selected from the group consisting of halogen, cyano, (C₃- C₉)cycloalkyl, (C₁-C₄)alkoxy, (C₁-C₄)alkylthio and, in the case of cyclic radicals, also (C₁-C₄)alkyl and (C₁-C₄)haloalkyl,
R⁰ independently of R¹, is a (C₁-C₆)alkyl, (C₂-C₆)alkenyl or (C₃-C₆)alkynyl radical, where each of the three latter radicals is unsubstituted or by one or more radicals selected from the group consisting of halogen, cyano, (C₁-C₄)alkoxy, (C₁-C₄)haloalkoxy, (C₁-C₄)alkylthio, (C₁-C₄)alkanoyloxy, (C₁-C₄)haloalkanoyloxy, aryl, aryloxy, aroyl, aroyloxy and heterocyclyl, where each of the latter 5 radicals is unsubstituted or substituted,
or (C₃-C₉)cycloalkyl, (C₄-C₉)cycloalkenyl, aryl or heterocyclyl, where each of the latter 4 radicals is unsubstituted or substituted,
R¹, independently of R⁰, is
(C₁-C₆)alkyl, (C₂-C₆)alkenyl or (C₂-C₆)alkynyl, where each of the three latter radicals is unsubstituted or substituted by one or more radicals selected from the group consisting of halogen, cyano, (C₁-C₄)alkoxy, (C₁- C₄)haloalkoxy and (C₁-C₄)alkylthio,
or aryl or heterocyclyl, where each of the 2 latter radicals is unsubstituted or substituted,
where the substituents R¹ and the amino group on the two ring carbon atoms marked with an asterisk (*) in each case are arranged in cis arrangement to one another and the compound (Ia) is present in the form of a stereochemically pure compound of the formula (Ia-A) or (Ia-B) 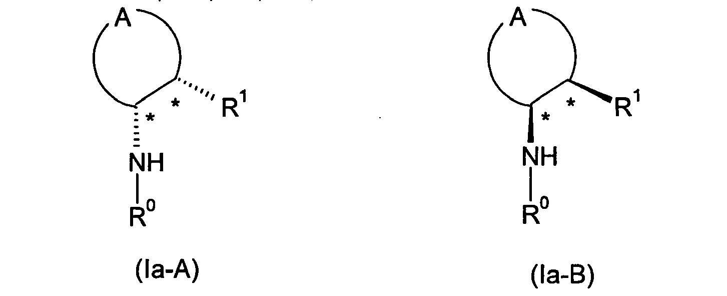 in which the general radicals are each as defined in formula (Ia), or in the form of an isomer mixture of the compounds of the formulae (la-A) and (la-B) in an isomer ratio other than the ratio of 1:1.

4. The process as claimed in claim 1 or 2, wherein compounds of the formula (Ib) or salts thereof are prepared 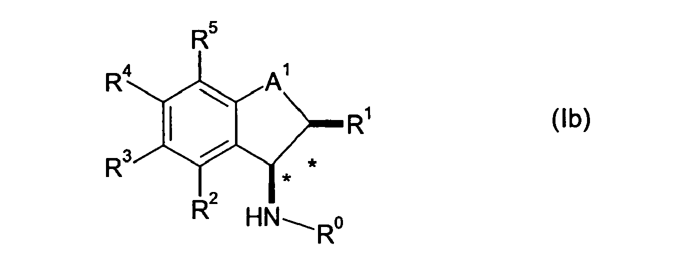 in which
A¹ is a direct bond or a group of the formula (CR⁶R⁷)ₙ in which n is from 1 to 6 and R⁶ and R⁷ are each independently, or, in the case that n is greater than 1, the R⁶ and R⁷ radicals are in each case independently hydrogen, (C₁-C₄)alkyl, (C₁-C₄)haloalkyl, halogen, (C₁-C₄)alkoxy or (C₁-C₄)haloalkoxy, where individual CR⁶R⁷ groups may be replaced by heteroatoms from the group of O and S,
R⁰, independently of R¹, is a (C₁-C₆)alkyl or (C₂-C₆)alkenyl radical, where each of the two latter radicals is unsubstituted or by one or more radicals selected from the group consisting of halogen, cyano, (C₁- C₄)alkoxy, (C₁-C₄)haloalkoxy, (C₁-C₄)alkylthio, (C₁-C₄)alkanoyloxy, (C₁- C₄)haloalkanoyloxy, aryl, aryloxy, aroyl, aroyloxy and heterocyclyl, where each of the latter 5 radicals is unsubstituted or substituted,
or (C₃-C₉)cycloalkyl, (C₄-C₉)cycloalkenyl, aryl or heterocyclyl, where each of the latter 4 radicals is unsubstituted or substituted,
R¹, independently of R⁰, is (C₁-C₆)alkyl which is unsubstituted or substituted by one or more radicals selected from the group consisting of halogen, cyano, (C₁- C₄)alkoxy, (C₁-C₄)haloalkoxy, (C₁-C₄)alkylthio and aryl which is optionally substituted, and heterocyclyl which is optionally substituted, or
aryl which is optionally substituted, or
heterocyclyl which is optionally substituted,
and
R² is H, (C₁-C₄)alkyl, (C₁-C₃)haloalkyl, halogen, (C₁-C₃)alkoxy, (C₁-C₃)haloalkoxy or CN,
R³ is H, (C₁-C₄)alkyl, (C₁-C₃)haloalkyl, halogen, (C₁-C₃)alkoxy, (C₁-C₃)haloalkoxy or CN,
R⁴ is H, (C₁-C₄)alkyl, (C₁-C₃)haloalkyl, halogen, (C₁-C₃)alkoxy, (C₁-C₃)haloalkoxy or CN and
R⁵ is H, (C₁-C₄)alkyl, (C₁-C₃)haloalkyl, halogen, (C₁-C₃)alkoxy, (C₁-C₃)haloalkoxy or CN,
where the substituents R¹ and the amino group on the two ring carbon atoms marked with an asterisk (*) in each case are arranged in cis arrangement to one another and the compound is present in the form of a stereochemically pure compound of the formula (Ib-A) or (Ib-B) 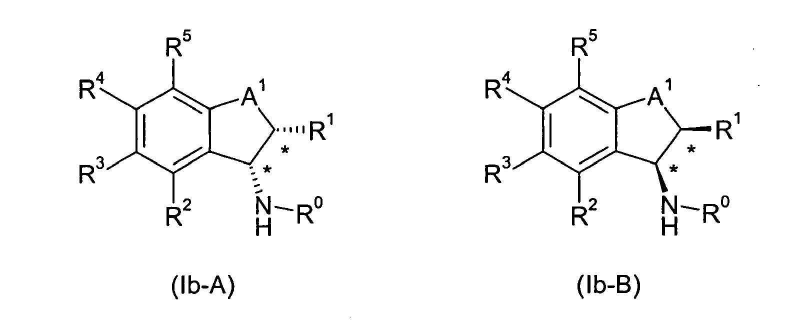 in which the general radicals are each as defined in formula (Ib), or in the form of an isomer mixture of the compounds of the formulae (Ib-A) and (Ib-B) in an isomer ratio other than the ratio of 1:1.

5. The process as claimed in one of claims 1 to 4, wherein the reaction is carried out in the presence of an inert organic solvent or in substance.

6. The process as claimed in one of claims 1 to 5, wherein the reaction is carried out as a catalytic hydrogenation.

7. The process as claimed in one of claims 1 to 5, wherein the reaction is carried out as a catalytic transfer hydrogenation.

8. The process as claimed in claim 7, wherein the reaction is carried out with formic acid as a hydrogen donor in the presence of a tertiary amine.

9. The process as claimed in one of claims 1 to 8, wherein the imine of the formula (II) is prepared in situ from the corresponding ketone and an amine of the formula R⁰-NH₂ and reduced.

10. The process as claimed in one of claims 1 to 9, wherein the R⁰ group is eliminated from the compound of the formula (I) or salts thereof by customary methods to obtain amines of the formula (I') or salts thereof 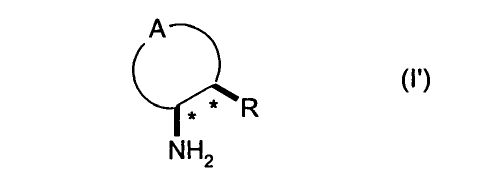 in which
A and R and the stereochemical configuration are each as defined in formula (I).

## Revendications

1. Procédé pour la préparation d'amines cycliques optiquement actives de la formule (I) ou de leurs sels, 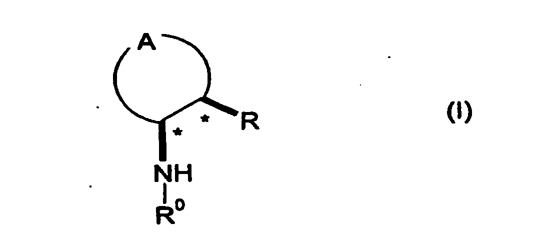 dans laquelle
A représente avec les atomes C marqués à chaque fois d'un astérisque (*) un cycle non aromatique saturé ou insaturé, carbocyclique ou hétérocyclique ayant de 3 à 30 atomes de cycle, lequel peut de plus être encore substitué sur les restes R et NH-R⁰,
R⁰ est indépendamment de R un reste alkyle en C₁-C₆, alcényle en C₂-C₆ ou alcynyle en C₃-C₆, chacun des trois restes cités précédemment étant non substitué ou étant substitué par un ou plusieurs restes choisis dans le groupe constitué d'un atome d'halogène, d'un groupe cyano, alcoxy en C₁-C₄, haloalcoxy en C₁-C₄, alkylthio en C₁-C₄, alcanoyloxy en C₁-C₄, haloalcanoyloxy en C₁-C₄, aryle, aryloxy, aroyle, aroyloxy et hétérocyclyle, chacun des 5 restes cités précédemment étant non substitué ou substitué,
ou cycloalkyle en C₃-C₉, cycloalcényle en C₄-C₉, aryle ou hétérocyclyle, chacun des 4 restes cités précédemment étant non substitué ou substitué,
R représente indépendamment de R⁰
un groupe alkyle en C₁-C₆, alcényle en C₂-C₆ ou alcynyle en C₂-C₆, chacun des trois restes cités précédemment étant non substitué ou substitué par un ou plusieurs restes du groupe constitué d'un atome d'halogène, d'un groupe cyano, alcoxy en C₁-C₆, haloalcoxy en C₁-C₆, alkylthio en C₁-C₆, aryle, lequel est non substitué ou substitué, et hétéroaryle, lequel est non substitué ou substitué, ou
aryle, lequel est non substitué ou substitué, ou
hétéroaryle, lequel est non substitué ou substitué,
ou
R⁰ et A forment un cycle B1 et représentent ce faisant ensemble avec le groupe NH et l'atome C lié au groupe NH marqué d'un astérisque (*) un cycle hétérocyclique de 4 à 30 atomes de cycle, lequel est de plus éventuellement substitué et lequel contient éventuellement un ou deux hétéroatomes supplémentaires choisis dans le groupe constitué de N, O et S,
ou
R et A forment un cycle B2 et représentent ce faisant avec l'atome C lié à R marqué d'un astérisque (*) un cycle hétérocyclique ou carbocyclique de 3 à 30 atomes de cycle, lequel est de plus éventuellement substitué et contient dans le cas d'un cycle hétérocyclique 1 ou 2 ou 3 autres hétéroatomes choisis dans le groupe constitué de N, O et S
ou
R⁰ et R forment un cycle B3 et représentent ce faisant ensemble avec le groupe NH et les atomes C marqués à chaque fois d'un astérisque (*) un cycle hétérocyclique de 4 à 30 atomes de cycle, lequel est éventuellement encore substitué et lequel contient éventuellement 1 ou 2 autres hétéroatomes choisis dans le groupe constitué de N, O et S
ou
R⁰ et R et éventuellement A forment tous à la fois deux ou plusieurs des cycles B1, B2 et B3 cités,
R et le groupe NH-R⁰ étant disposés l'un par rapport à l'autre sur les deux atomes C de cycle marqués à chaque fois d'un astérisque (*) en position cis et la configuration stéréochimique sur ces atomes C étant différente de la configuration racémique,
**caractérisé en ce que** l'on transforme une imine de la formule (II) ou un sel de celle-ci, 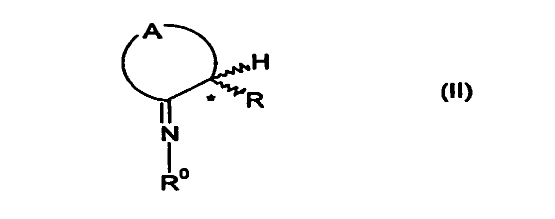 dans laquelle A, R⁰ et R sont comme définis dans la formule (I) et le composé de la formule (II) est présent en ce qui concerne l'atome C de cycle marqué d'un astérisque (*) comme un mélange racémique ou comme un mélange avec un rapport quelconque des autres isomères des stéréoisomères concernés,
en présence d'hydrogène ou d'un donneur d'hydrogène et d'un catalyseur non enzymatique, lequel contient un complexe catalytiquement efficace optiquement actif d'un ou plusieurs métaux de transition choisis dans le groupe constitué du ruthénium, du rhodium, du palladium, de l'iridium, de l'osmium, du platine, du fer, du nickel et du samarium avec des ligands organiques, en un composé de la formule (I).

2. Procédé selon la revendication 1, **caractérisé en ce que** l'on obtient les composés (I) ou leurs sels avec une configuration cis sur les deux centres de chiralité marqués à chaque fois d'un astérisque (*) par rapport à la configuration trans avec une sélectivité de 60 à 100 %, un énantiomère du composé cis avec une énantiosélectivité à chaque fois supérieure à 20 % ee rapportés à la teneur totale en énantiomères du composé cis étant obtenu.

3. Procédé selon la revendication 1 ou 2, **caractérisé en ce que** l'on prépare des composés de la formule (Ia) ou leurs sels, 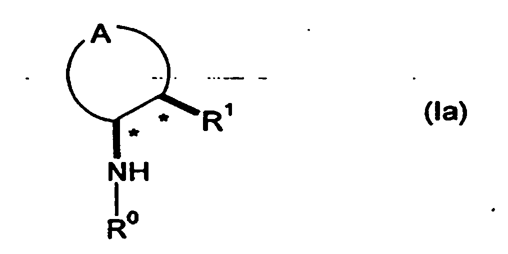 dans laquelle
A représente avec les atomes C marqués à chaque fois d'un astérisque (*) un cycle non aromatique, saturé ou insaturé, carboxylique ou hétérocyclique de 3 à 30 atomes de cycle et dans le cas d'un cycle hétérocyclique avec de 1 à 3 hétéroatomes de cycle choisis dans le groupe constitué de N, O et S, lequel est de plus non substitué sur les restes R¹ et NH-R⁰ ou est de plus substitué par un ou plusieurs restes choisis dans le groupe constitué d'un atome d'halogène, d'un groupe hydroxy, amino, cyano, nitro, alkyle en C₁-C₄, cycloalkyle en C₃-C₉, alcoxy en C₁-C₄, alcoxycarbonyle en C₁-C₄, alkylthio en C₁-C₄, alkylsulfonyle en C₁-C₄, aryle et hétérocyclyle, chacun des 8 restes cités précédemment étant non substitué ou étant substitué par un ou plusieurs restes du groupe constitué d'un atome d'halogène, d'un groupe cyano, cycloakyle en C₃-C₉, alcoxy en C₁-C₄, alkylthio en C₁-C₄ et dans le cas de restes cycliques également alkyle en C₁-C₄ et haloalkyle en C₁-C₄,
R⁰ représente indépendamment de R¹ un reste alkyle en C₁-C₆, alcényle en C₂-C₆ ou alcynyle en C₃-C₆, chacun des trois restes cités précédemment étant non substitué ou étant substitué par un ou plusieurs restes choisis dans le groupe constitué d'un atome d'halogène, d'un groupe cyano, alcoxy en C₁-C₄, haloalcoxy en C₁-C₄, alkylthio en C₁-C₄, alcanoyloxy en C₁-C₄, haloalcanoyloxy en C₁-C₄, aryle, aryloxy, aroyle, aroyloxy et hétérocyclyle, chacun des 5 restes cités précédemment étant non substitué ou substitué,
ou cycloalkyle en C₃-C₉, cycloalcényle en C₄-C₉, aryle ou hétérocyclyle, chacun des 4 restes cités précédemment étant non substitué ou substitué,
R¹ représente indépendamment de R⁰ un groupe alkyle en C₁-C₆, alcényle en C₂-C₆ ou alcynyle en C₂-C₆, chacun des trois derniers restes cités précédemment étant non substitué ou substitué par un ou plusieurs restes choisis dans le groupe constitué d'un atome d'halogène, d'un groupe cyano, alcoxy en C₁-C₄, haloalcoxy en C₁-C₄ et alkylthio en C₁-C₄,
ou aryle ou hétérocyclyle, chacun des deux restes cités précédemment étant non substitué ou substitué,
les substituants R¹ et les groupes amino sur les deux atomes de cycle C marqués à chaque fois d'un astérisque (*) étant disposés les uns par rapport aux autres en position cis et le composé (Ia) étant présent comme un composé stéréochimiquement pur de la formule (Ia-A) ou (Ia-B), 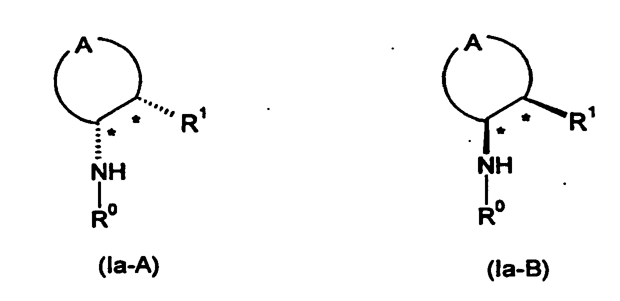 dans lesquelles les restes généraux sont comme définis dans la formule (Ia), ou comme un mélange d'isomères des composés des formules (Ia-A) et (Ia-B) dans un rapport d'isomères différent du rapport 1:1.

4. Procédé selon la revendication 1 ou 2, **caractérisé en ce que** l'on prépare des composés de la formule (Ib) ou leurs sels, 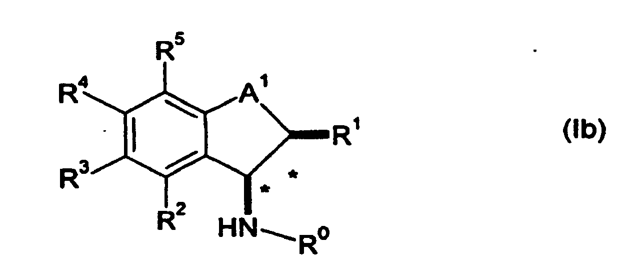 dans laquelle
A¹ représente une liaison directe ou un groupe de la formule (CR⁶R⁷)ₙ, dans laquelle n est un nombre de 1 à 6 et R⁶ et R⁷ représentent indépendamment les uns des autres respectivement dans le cas où n est supérieur à 1, à chaque fois l'hydrogène, un groupe alkyle en C₁-C₄, haloalkyle en C₁-C₄, un atome d'halogène, un groupe alcoxy en C₁-C₄ ou haloalcoxy en C₁-C₄, les groupes individuels CR⁶R⁷ pouvant être remplacés par des hétéroatomes du groupe constitué de O et de S,
R⁰ représente indépendamment de R¹ un reste alkyle en C₁-C₆ ou alcényle en C₂-C₆,
chacun des deux restes cités précédemment étant non substitué ou substitué par un ou plusieurs restes du groupe constitué d'un atome d'halogène, d'un groupe cyano, alcoxy en Cₗ-C₄, haloalcoxy en C₁-C₄, alkylthio en C₁-C₄, alcanoyloxy en C₁-C₄, haloalcanoyloxy en C₁-C₄, aryle, aryloxy, aroyle, aroyloxy et hétérocyclyle, chacun des 5 restes cités précédemment étant non substitué ou substitué,
ou cycloalkyle en C₃-C₉, cycloalcényle en C₄-C₉, aryle ou hétérocyclyle, chacun des 4 restes cités précédemment étant non substitué ou substitué,
R¹ représente indépendamment de R⁰,
un groupe alkyle en C₁-C₆, lequel est non substitué ou est substitué par un ou plusieurs restes choisis dans le groupe constitué d'un atome d'halogène, d'un groupe cyano, alcoxy en C₁-C₄, haloalcoxy en C₁-C₄, alkylthio en C₁-C₄ et aryle, lequel est éventuellement substitué, et hétérocyclyle, lequel est éventuellement substitué, ou
aryle, lequel est éventuellement substitué, ou
hétérocyclyle, lequel est éventuellement substitué,
et
R² représente H, un groupe alkyle en C₁-C₄, haloalkyle en C₁-C₃, un atome d'halogène, un groupe alcoxy en C₁-C₃, haloalcoxy en C₁-C₃ ou CN,
R³ représente H, un groupe alkyle en C₁-C₄, haloalkyle en C₁-C₃, un atome d'halogène, un groupe alcoxy en C₁-C₃, haloalcoxy en C₁-C₃ ou CN,
R⁴ représente H, un groupe alkyle en C₁-C₄, haloalkyle en C₁-C₃, un atome d'halogène, un groupe alcoxy en C₁-C₃, haloalcoxy en C₁-C₃ ou CN et
R⁵ représente H, un groupe alkyle en C₁-C₄, haloalkyle en C₁-C₃, un atome d'halogène, un groupe alcoxy en C₁-C₃, haloalcoxy en C₁-C₃ ou CN,
les substituants R¹ et les groupes amino sur les deux atomes de cycle C marqués à chaque fois d'un astérisque (*) étant disposés les uns par rapport aux autres en positon cis et le composé étant présent comme un composé stéréochimiquement pur de la formule (Ib-A) ou (Ib-B), 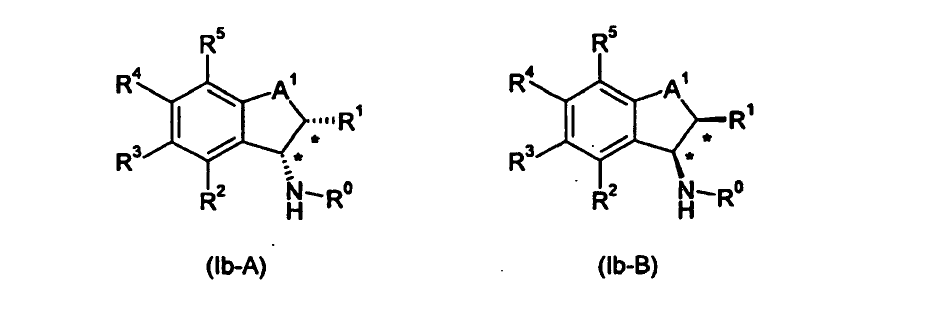 dans lesquelles les restes généraux sont comme définis dans la formule (Ib), ou comme un mélange d'isomères des composés des formules (Ib-A) et (Ib-B) dans un rapport d'isomères différent du rapport 1:1.

5. Procédé selon l'une quelconque des revendications 1 à 4, **caractérisé en ce que** l'on réalise la réaction en présence d'un solvant organique inerte ou en substance.

6. Procédé selon l'une quelconque des revendications 1 à 5, **caractérisé en ce que** l'on réalise la réaction comme une hydrogénation catalytique.

7. Procédé selon l'une quelconque des revendications 1 à 5, **caractérisé en ce que** l'on réalise la réaction comme une hydrogénation de transfert catalytique.

8. Procédé selon la revendication 7, **caractérisé en ce que** l'on réalise la réaction avec de l'acide formique comme donneur d'hydrogène en présence d'une amine tertiaire.

9. Procédé selon l'une quelconque des revendications 1 à 8, **caractérisé en ce que** l'on prépare l'imine de la formule (II) à partir de la cétone correspondante et d'une amine de la formule R⁰-NH₂ *in situ* et on la réduit.

10. Procédé selon l'une quelconque des revendications 1 à 9, **caractérisé en ce que** l'on obtient le composé de la formule (I) ou ses sels, on sépare le groupe R⁰ selon des procédés classiques et on obtient l'amine de la formule (I') ou ses sels, 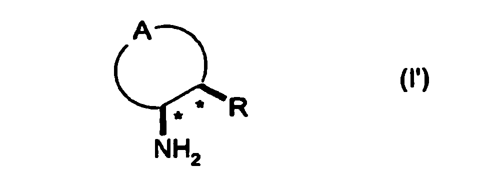 dans laquelle A et R ont la configuration stéréochimique comme définie dans la formule (I).
